(19) Europäisches Patentamt
European Patent Office
Office européen des brevets

(11) Publication number: **0 117 767**
**A1**

(12) # EUROPEAN PATENT APPLICATION

(21) Application number: **84400024.0**

(22) Date of filing: **06.01.84**

(51) Int. Cl.³: **C 12 N 15/00**, C 12 P 21/00, C 12 P 21/04, C 12 N 7/00, A 61 K 39/23 // C12R1/19

(30) Priority: **07.01.83 US 456423**
**22.12.83 US 564567**

(43) Date of publication of application: **05.09.84**
**Bulletin 84/36**

(84) Designated Contracting States: **BE DE FR GB NL**

(71) Applicant: **MOLECULAR GENETICS, INC., 10320 Bren Road East, Minnetonka Minnesota 55434 (US)**

(72) Inventor: **Halling, Shirley Mae, 2919 Vernon Avenue So., St. Louis Park Minnesota (US)**
Inventor: **Smith, Susan Lori, 2608 Huntington Avenue So., St. Louis Park Minnesota (US)**
Inventor: **Muscoplat, Charles Craig, 7800 Winsdale Avenue, Golden Valley Minnesota (US)**
Inventor: **Collett, Marc Stephen, 5697 Green Circle Drive, Minnetonka Minnesota (US)**

(74) Representative: **Corre, Jacques Denis Paul et al, Cabinet Regimbeau 26, Avenue Kléber, F-75116 Paris (FR)**

(54) **Production of parvovirus subunit vaccines.**

(57) Methods and compositions are provided for the cloning and expression of a parvovirus (PV) structural protein genetic sequence in single-cell host organisms. Also described are methods for culturing these novel single-cell organisms to produce the PV-gene product and methods for purification of the gene product. The PV-related polypeptide produced by the recombinant DNA techniques described herein may be formulated for use as an immunogen in vaccines to protect against parvovirus infection.

EP 0 117 767 A1

PRODUCTION OF PARVOVIRUS SUBUNIT VACCINES

The present invention utilizes recombinant DNA techniques to insert a DNA sequence coding for a parvovirus structural protein, or a portion thereof, into a DNA vector, such as viral DNA, plasmid DNA or bacteriophage DNA, such that the vector is capable of replicating and directing expression of the parvovirus gene in a bacterial host or other single cell system. The resulting recombinant DNA molecule is introduced into host cells and thereby enables the production of a parvovirus-related protein, or a portion or molecular variant thereof, by the host cells. The protein produced is then isolated, purified and modified for use as an immunogen in a vaccine against infection by parvovirus.

RECOMBINANT DNA TECHNOLOGY AND GENE EXPRESSION

Recombinant DNA technology involves insertion of specific DNA sequences into a DNA vehicle (vector) to form a recombinant DNA molecule which is capable of replication in a host cell. Generally, the inserted DNA sequence is foreign to the recipient DNA vehicle, i.e., the inserted DNA sequence and the DNA vector are derived from organisms which do not exchange genetic information in nature, or the inserted DNA sequence may be wholly or partially synthetically made. In recent years several general methods have been developed which enable construction of recombinant DNA molecules. For example, U.S. Pat. No. 4,237,224 to Cohen and Boyer describes production of such recombinant plasmids using restriction enzymes and methods known as ligation. These recombinant plasmids are then introduced and replicated in unicellular organisms by means of transformation. Because of the general applicability of the techniques described therein, U.S.

Pat. No. 4,237,224 is hereby incorporated by reference into the present specification.

Another method for introducing recombinant DNA molecules into unicellular organisms is described by Collins and Hohn in U.S. Pat. No. 4,304,863 which is also incorporated herein by reference. This method utilizes a packaging/transduction system with bacteriophage vectors (cosmids).

Regardless of the method used for construction, the recombinant DNA molecule must be compatible with the host cell, i.e., capable of autonomous replication in the host cell. The recombinant DNA molecule should also have a marker function which allows the selection of host cells so transformed by the recombinant DNA molecule. In addition, if all of the proper replication, transcription and translation signals are correctly arranged on the DNA molecule, the foreign gene will be properly expressed in the transformed cells and their progeny.

As is characteristic of all viruses which infect eucaryotic cells, parvovirus requires a eucaryotic host cell system in which to replicate its genome, express its viral genes and generate its progeny. The signals and control elements for DNA replication, gene expression and virus assembly in eucaryotes differ from those of procaryotes. This is of critical importance when attempts are made to express in procaryotic host cells a gene which is naturally expressed only in eucaryotic cells.

These different genetic signals and processing events control many levels of gene expression; for instance, DNA transcription and messenger RNA translation. Transcription of DNA is dependent upon the presence of a promoter which is a DNA sequence that directs the binding of RNA polymerase and thereby promotes transcription. The DNA sequences of eucaryotic promoters differ from those of procaryotic promoters. Furthermore, eucaryotic promoters and accompanying genetic signals may not be recognized in or may not function in a procaryotic system.

Similarly, translation of messenger RNA (mRNA) in procaryotes depends upon the presence of the proper procaryotic signals which differ from those of eucaryotes. Efficient translation of mRNA in procaryotes requires a ribosome binding site called the Shine-Dalgarno (SD) sequence on the mRNA. This sequence is a short nucleotide sequence of mRNA that is located before the start codon (AUG) which encodes the amino-terminal methionine of the protein. The SD sequences are complementary to the 3'-end of the 16S rRNA (ribosomal RNA) and probably promote binding of mRNA to ribosomes by duplexing with the rRNA to allow correct positioning of the ribosome. For a review on maximizing gene expression, see Roberts and Lauer, 1979, Methods in Enzymology 68:473.

In addition to these different control elements, many eucaryotic genes contain intervening sequences (introns) which are not present in the mature mRNA or the corresponding double stranded complementary DNA (cDNA) coding for the gene (for review see Chambon, 1981, Sci. Amer. 244:60-71). During mRNA processing in eucaryotes, these introns are spliced out of the mRNA prior to translation (for reviews see Crick, 1979, Science 204:264; Sharp, 1981, Cell 23:643).

Expression of eucaryotic genes cloned in procaryotic hosts requires that the coding sequence of the gene of interest be available in a form uninterrupted by intervening sequences. Therefore, when eucaryotic genetic sequences which contain intervening sequences are to be cloned in a procaryotic host, isolation of mRNA as the source of the genetic information offers an advantage over the isolation of the relevant DNA sequence. Accordingly, the isolated mRNA is transcribed into single stranded complementary DNA (cDNA) copies which are then enzymatically processed into double-stranded DNA molecules which can be inserted into cloning vectors.

Such recombinant DNA techniques have been recently used for cloning cDNA copies of RNA viral genomes such as poliovirus (Kitamura et al., 1981, Nature (London) 291:547; Racaniello and Baltimore, 1981, Proc. Natl. Acad. Sci. USA 78(8):4887; Racaniello and Baltimore, 1981, Science 214:916); vesicular stomatitis virus (Rose, 1980, Cell 19:415); and influenza (Sleigh, et al., 1979, Nucleic Acids Res. 7:879). These techniques also have application in the development of antiviral vaccines (Heiland and Gething, 1981, Nature (London) 292:851).

One difficulty encountered in cloning the cDNA copy is the enzymatic processing of the single-stranded cDNA molecule into a full length (or nearly full length) double-stranded form. Even if reaction conditions are adjusted to obtain complete cDNA copies of the mRNA, parts of the sequence may be lost during synthesis of the second DNA strand. Second strand synthesis employs a DNA polymerase enzyme (e.g., E. coli Pol I, AMV reverse transcriptase) to utilize the single-stranded cDNA as both primer and template. This results in the formation of a double-stranded DNA molecule which has a single-stranded loop located at the terminus corresponding to the 5'-terminus of the original mRNA molecule (Efstradiadis, 1976, Cell 7:279). This single-stranded loop must be digested with the single-strand specific nuclease, S1 nuclease, before the double stranded molecule can be inserted into a cloning vector. The S1 nuclease digestion often results in the removal of portions of the cDNA corresponding to the 5'-terminus of the mRNA molecule and reduces the yield of full length cDNA clones. Several modifications of this procedure which eliminate the need for S1 nuclease digestion have been reported (Land, et al., 1981, Nucleic Acids Res. 9:2251; Okayama and Berg, 1982, Molecular and Celluar Biology 2(2):161).

Many other factors complicate the expression of eucaryotic genes in procaryotes even after the proper signals are inserted and appropriately positioned. A clear understanding of the nature of these factors and the mechanisms by which they operate is presently lacking. However, one such factor is the presence of an active proteolytic system in E. coli and other bacteria. This protein-degrading system appears to selectively destroy "abnormal" or foreign proteins such as eucaryotic proteins. A tremendous utility, therefore, would be afforded by the development of a means to protect eucaryotic proteins expressed in bacteria from proteolytic degradation. One strategy is to construct hybrid genes in which the eucaryotic sequence is ligated in phase (i.e., in the correct reading frame) with a procaryotic gene. Expression of this hybrid gene results in a fusion protein product (a protein that is a hybrid of procaryotic and foreign or eucaryotic amino acid sequences).

Construction of hybrid genes was the approach used in the molecular cloning of genes encoding a number of eucaryotic proteins, such as somatostatin (Itakura et al., 1977, Science 198:1056), rat pre-proinsulin (Villa-Komaroff et al., 1978, Proc. Natl. Acad. Sci., U.S.A. 75:3727), growth hormone (Seeburg et al., Nature 276:795), and ovalbumin-like protein (Mercereau-Puijalon et al., 1978, Nature 275:505). Additionally, procaryotic promoters have been ligated to such fusion gene sequences in the case of ovalbumin (Fraser et al., 1978, Proc. Natl. Acad. Sci., U.S.A. 75:5936) and ß-globin (Guarente et al., 1980, Cell 20:543). The Guarente et al. system involves inserting the lac promoter, including the SD sequence, at varying distances in front of the ATG of the fusion gene. Although the molecular cloning and expression of several eucaryotic genes has been accomplished, this has not heretofore been done for any parvovirus genes encoding structural proteins. Nor is the state of the art such that expression of foreign or eucaryotic genes in procaryotic host cells may be routinely performed.

Successful expression of a cloned gene requires efficient transcription of DNA, translation of the mRNA and in some instances post-translational modification of the protein. Expression vectors have been used to express genes in a suitable host and to increase protein production. The cloned gene should be placed next to a strong promoter which is controllable so that transcription can be turned on when necessary. Cells can be grown to a high density and then the promoter can be induced to increase the number of transcripts. These, if efficiently translated will result in high yields of protein production. This is an especially efficient system if the foreign protein is deleterious to the host cell.

VACCINES

There are four basic approaches for the control and therapy of viral infections: (1) vaccines that elicit an active immune response; (2) chemotherapeutic agents that inhibit viral replication; (3) agents that induce the synthesis of interferon; and (4) administration of antibodies for passive immunity. All four can be used to prevent infection, but are more effective when applied early in infection. Vaccination, or active immunization is usually ineffective after infection has begun.

Viruses are small and relatively simple in structure. Viruses contain a central core of nucleic acid (either DNA or RNA) surrounded by a protein coat called a capsid. In many viruses, the capsid is surrounded by a loose membranous envelope which is similar to a cellular membrane in composition. Nonenveloped viruses are referred to as naked capsids. The nucleic acid core of viruses contains, through a complex coding scheme, the information needed for infecting cells of host tissues, multiplying themselves and producing the protective capsid.

When a host is infected by a virus, it reacts to the capsid as a foreign protein. The host develops immunity which is manifested by the formation of antibodies, serum globulin proteins, and lymphocytes which are capable of sticking to the viral coat proteins and inactivating the virus. Thus, conventional vaccines providing active immunity are prepared by producing large amounts of virus in cell tissue culture or in laboratory animals and then rendering the virus harmless without destroying its immunological properties. Traditionally this is accomplished either by inactivating the infectivity of the virus (<u>i.e.</u>, "killing" the virus,

usually by treatment with various chemical agents such as formaldehyde) for use in inactivated vaccines, or by selecting an avirulent or attenuated (modified) virus for use in live vaccines (attenuated vaccines). Attenuation is obtained by adapting the virus to unusual conditions (to different animal hosts and cell cultures) and by frequent passages of large viral inocula to select mutants which have lost virulence. A few vaccines still used in veterinary practice consist of fully virulent infectious virus injected in a site where virus multiplication is of little consequence. This procedure is considered too dangerous for use in man.

Attenuated virus used in live vaccines are generally excellent immunogens because the attenuated virus multiplies in the host thus eliciting long-lasting immunity. Attenuated vaccines induce humoral antibodies directed against all viral antigens, both surface and internal antigens of the virion. However, a number of problems are encountered with the use of live vaccines, such as insufficient attenuation of the virus, genetic instability of the virus, contamination by adventitious viruses in cell cultures used to grow the vaccine virus, and, finally, the vaccine may be unstable (e.g., heat-labile).

While the use of inactivated vaccines (employing "killed" or inactivated virus that does not multiply) avoids the difficulties encountered with the use of live vaccines, killed viruses do not multiply in the immunized animal and usually produce antibodies directed against only virion structural components. There are two major difficulties with inactivated vaccines. Firstly, it is frequently not possible to inactivate all the virus particles. Secondly there is the difficulty of producing enough virus to provide the necessary quantity of the relevant antigen. Other difficulties encountered with the use of inactivated vaccines are that the immunity achieved is brief and often requires additional immunizations, the antigenic sites may be altered by the inactivating chemical treatment, and thus be less immunogenic or may induce antibodies that are less effective at neutralizing viral infections, and that these vaccines do not induce satisfactory levels of IgA.

Subunit vaccines contain only the necessary and relevant immunogenic material, such as the capsid proteins of nonenveloped icosahedral viruses or the peplomers (glycoprotein spikes) of enveloped viruses. Subunit vaccines can be made by isolating the relevant subunit from highly purified viral fractions, or by synthesizing the relevant polypeptide. A major advantage of subunit vaccines is the exclusion of genetic material of viral origin and of toxic, contaminating or otherwise undesirable substances of the host or donor. However, at present, production of subunit vaccines using these methods is too expensive for widespread commercial veterinary use. Recombinant DNA technology has much to offer in the production of subunit vaccines; the molecular cloning and host cell expression of viral genes which encode the relevant immunogenic portions of the virus or its proteins can produce sufficient quantities of the relevant polypeptide for use as an immunogen in a subunit vaccine.

Vaccines are often administered in conjunction with various adjuvants. The adjuvants aid in attaining a more durable and higher level of immunity using smaller amounts of antigen in fewer doses than if the immunogen were administered alone. The mechanism of adjuvant action is complex and not completely understood. However, it may involve the stimulation of phagocytosis and other activities of the reticuloendothelial system as well as a delayed release and degradation of the antigen. Examples of adjuvants include Freund's adjuvant (complete or incomplete), Adjuvant 65 (containing peanut oil, mannide monooleate and aluminum monostearate), Adjuvant L-121 and mineral gels such as aluminum hydroxide, aluminum phosphate, or alum. Freund's adjuvant is no longer used in vaccine formulations for humans or for food animals because it contains nonmetabolizable mineral oil and is a potential carcinogen; however, the mineral gels are widely used in commercial veterinary vaccines.

## PARVOVIRUS

Porcine parvovirus (PPV) infection is widespread in the swine population throughout the world. The virus is primarily involved in reproductive diseases of swine. Transplacental infection of porcine fetuses has been demonstrated by infecting pregnant sows with PPV and using antibodies directed against PPV to detect the virus in the fetus. A viremia (virus in the blood) and a slight leukopenia (a low white blood cell count) have been observed in pregnant sows infected experimentally. Infection of susceptible swine during gestation most often results in the death of the fetus or embryo and its resorption or mummification; however, infertility, abortion, stillbirth, neonatal death and reduced vitality may also be consequences of in utero PPV infection. In contrast to infections in utero, most infections in post natal swine result in either subclinical or mild clinical signs. Humoral immunity, either as a consequence of natural exposure or from vaccination will often prevent viremia in the pregnant sow, and thus prevent transplacental virus transfer (from both dam to fetus and from fetus to fetus) and subsequent fetal death. Since reproductive disease is the major clinical manifestation of PPV infection, immunity in pregnant sows is important. Currently conventional vaccines are made from inactivated or killed PPV ("Parvo-Vac", Norden Industries, Inc., Lincoln, Nebraska) or live virus vaccines are made from modified PPV (Paul and Mengeling, 1980, Am. J. Vet. Res. 41(12):2007.

Porcine parvovirus agglutinates red blood cells of guinea pig, rat, chicken, rhesus monkey and human type O. PPV is readily propagated in actively dividing cell cultures of porcine origin, and can be isolated from vaginal mucus, semen, normal and aborted fetuses, intestinal tract, brain, lungs and turbinates. Thus, it may also be desirable to vaccinate boars as well as sows to prevent the spread of parvoviral disease resulting from contact. PPV induces high hemagglutination-inhibition (HAI) and serum-neutralizing (SN) antibody titers in infected pigs.

Canine parvovirus infection in dogs results in two sets of clinical symptoms. The most common effect is a contagious enteric disease that is manifested by diarrhea (often bloody), vomiting, loss of appetite, depression, fever, and sometimes, death caused by severe dehydration. A dog of any age may be affected, but the disease is more severe in puppies. Widespread outbreaks of severe contagious enteritis were first reported in 1978 when canine parvovirus (CPV), a previously unrecognized pathogen of dogs, was found to be associated with these outbreaks. Recent serological surveys demonstrate that CPV infection is now quite common. A second syndrome that results from CPV infection is the cardiac form which occurs in very young pups (less than 8 weeks of age). The cardiac form is manifested by acute inflammation of the heart muscle and may result in sudden death or other clinical symptoms such as difficulty in breathing, extreme depression, weakness, unwillingness to nurse and irregular heartbeat.

Direct transmission of CPV occurs when a dog with parvovirus infection contacts a healthy dog; infection results from ingestion of parvovirus from the feces of an infected dog. The virus then invades the lymph gland of the throat where it multiplies and later enters the bloodstream causing viremia. During the viremia phase massive amounts of virus spread from the bloodstream to all parts of the body such as the intestine, bone marrow, spleen, other lymph nodes and the heart. As the infection spreads the symptoms of illness become apparent. The disease is spread when the victim sheds parvovirus in its stools. The highest concentration of virus in the stool is observed when the infected dog is showing signs of illness, however, a dog can be a source of infection to other dogs without manifesting signs of illness.

The control of CPV infection by sanitation measures alone is extremely difficult because the virus is a hardy organism that is easily spread. Vaccination is the most effective control measure for canine parvovirus disease. Currently vaccines are formulated using inactivated and modified living CPV as well as inactivated and modified living viruses which are antigenically closely related to CPV, e.g., feline panleukopenia virus, (FPV) and mink enteritis virus (MEV) (Pollack and Carmichael, 1982, Cornell Vet. 72:16). In dogs, a sufficiently high titer of circulating antibodies will destroy parvovirus following exposure, however, active immunization may be blocked in puppies that have temporary or passive immunity derived from maternal antibodies directed against CPV which are passed from mother to puppy via colostrom ("first milk").

The parvoviruses (*e.g.*, Kilham rat virus, minute virus of mice, mink enteritis virus, canine parvovirus and porcine parvovirus) and the adeno-associated viruses (AAV) are members of the family Parvoviridae. While the parvoviruses and AAVs have certain similar physical and chemical characteristics, the parvoviruses are nondefective or autonomously replicating viruses, whereas the AAVs are defective and depend totally upon the multiplication of the unrelated adenoviruses for their own replication. A striking difference between autonomous parvoviruses and defective parvoviruses (AAV's) is that the genome of the autonomous viruses is a unique single-stranded DNA molecule whereas the genome of the defective AAV's is comprised of equimolar quantities of complementary single-stranded DNA.

Parvoviruses are small icosahedral non-enveloped DNA containing viruses. Electron microscopy shows the virions to be about 20 nm in diameter. Infectious particles of porcine parvovirus (virions containing DNA) have a buoyant density in cesium chloride of 1.39 g/mℓ, whereas empty particles (virions lacking DNA) have a buoyant density of 1.30 g/mℓ; immature and defective particles may have intermediate densities. The viral genome of porcine parvovirus consists of a linear molecule of single-stranded DNA approximately 5100 nucleotides in length, and the single-stranded viral genome of canine parvovirus is 4900 $\pm$ 100 nucleotides in length. The polarity of the parvoviral genome is negative, *i.e.*, the single-stranded DNA of the genome of nondefective parvoviruses is complementary to the cellular viral mRNA; thus the viral genomic DNA is considered negative in polarity.

Replication of autonomous parvovirus DNA is dependent upon the unusual structure of its ends, both of which have inverted terminal repeats (terminal palindromes) that form hairpin structures. During the replication process, the virion single-stranded DNA is converted to a linear duplex replicative form (RF) DNA. The RF DNA is amplified via a semiconservative mode of DNA replication. The RF DNA serves as a template for viral RNA synthesis and for the synthesis of single-stranded DNA of the negative polarity (minus strand) that ultimately becomes incorporated into progeny virions.

The major structural proteins of parvoviruses are presumably encoded by the 5'-termini of the viral genomes (e.g., minute virus of mouse). The capsids of parvoviruses contain three polypeptides, each having a different molecular weight, the largest of which is approximately 83,000 daltons. The tryptic peptides of the smaller structural proteins are a subset of the tryptic peptides of the large polypeptide. For example, porcine parvovirus contains three major structural proteins designated A, B, and C (molecular weights of 83,000, 64,000, and 60,000 daltons, respectively), which appear to be structurally and immunologically related (Molitor et al., 1983, J. Virol., 45:842-854).

The three species of structural polypeptides have closely related sequences, i.e., specific peptides derived proteolytically from C appear to be present in B; and peptides of B are a subset of those in A. The A protein (the largest) is a minor species which seems to be metabolically stable during maturation. On the other hand, the polypeptide B may be a precursor for C. The C polypeptide is derived by proteolytic cleavage of the B polypeptide in the assembled DNA-containing virion. Current evidence suggests that one gene encodes coat protein, i.e., polypeptides A and B are presumably synthesized from a common sequence in the genome. Finally, antisera directed against any individual polypeptide reacts with the other two proteins. Furthermore, antisera generated against each individual virion structural protein are capable of neutralizing virus infectivity. This suggests that all three viral structural proteins contain neutralizing antigenic determinants (Molitor et al., J. Virol., 1983, 45:842-854).

## SUMMARY OF THE INVENTION

Methods and compositions are provided for the cloning and expression of parvovirus genes or portions thereof encoding viral structural proteins in single-cell host organisms. Also described are methods for culturing these novel single-cell organisms to produce the parvovirus gene product and methods for purifying the gene product. The parvovirus-related proteins produced by the recombinant DNA techniques described herein may be formulated for use as immunogens in vaccines to protect against viral infection. Alternatively, these parvovirus-related proteins may be used to generate antisera which can be employed in the detection of virus for diagnostic purposes.

The isolated parvovirus genes or gene fragments were inserted into plasmid vectors to form recombinant plasmids which serve as biologically functional replication units. The recombinant plasmids were constructed so as to facilitate both the replication and expression of the parvovirus genetic sequences upon transformation of compatible host cells. In addition the plasmids were constructed so that transformed microorganisms actively expressing the parvovirus genetic sequences could be identified in one step. Methods are described for the isolation, stabilization, and purification of the expressed gene products. These methods take advantage of the fact that fusion proteins are resistant to degradation in the host cell. Methods are provided for the co-production of both the fusion and the unfused product. Methods are also provided for the isolation and purification of the co-aggregate forming proteins. Finally, methods are described for isolating the expressed product and for its use in formulating a vaccine. The methods and compositions of the present application are demonstrated for porcine parvovirus (PPV) and for canine parvovirus (CPV).

## BRIEF DESCRIPTION OF THE FIGURES

The present invention may be more fully understood by reference to the following detailed descriptions of the invention, examples of specific embodiments of the invention, and the appended figures.

In all figures within this text, restriction endonuclease enzyme recognition sites are indicated by the following abbreviations: AccI (Ac); AvaI (Av); BamHI (Bm); BglII (Bg); BstEII (Bs); EcoRI (RI); EcoRII (R2); EcoRV (RV); HaeIII (Ha); HincII (Hi2); HindIII (H3); HinfI (Hf); NcoI (Nc); PstI (Ps); PvuII (Pv); RsaI (Rs); SacI (Sc); SmaI (Sm); XbaI(Xb); and XmnI (Xm). Restriction endonuclease enzymes themselves are indicated by their typical abbreviations.

Unless otherwise indicated, the figures which follow are not drawn to scale.

FIG. 1 is a diagrammatic representation of the construction of pPPV-6 from virion genomic DNA.

FIG. 2A is a diagrammatic representation of the restriction map of pPPV-6. Only relevant restriction sites are indicated. Fragment sizes are denoted in base pairs (b.p.). The approximated values were determined from restriction digests analyzed by gel electrophoresis. All other b.p. values were determined from sequence data shown in FIG. 3 (base pair numbers are determined from midpoints of the respective restriction enzyme recognition sites).

FIG. 2B is a diagrammatic representation of the restriction analyses of both the PPV viral genome and the recombinant plasmid pPPV-6. The numbers below each fragment represent the approximate fragment size in b.p. as determined by gel electrophoresis using double-stranded linear DNA as marker molecular weight standards. These results indicate that the PPV DNA of pPPV-6 is derived from one end of the viral genome.

FIGS. 3 and 3A represent the nucleotide sequence of the PvuII/BglII fragment (i.e., the PPV DNA insert) of the positive strand of pPPV-6. Only relevant restriction sites are indicated.

FIG. 4A is a diagrammatic representation of the process used for subcloning a portion of PPV DNA (derived from pPPV-6) into M13 cloning vectors.

FIG. 4B is a diagrammatic representation of the process for separation and identification (by hybridization) of the complementary strands of the PPV DNA subcloned in M13.

FIG. 4C is a diagrammatic representation of the relation and orientation of the PPV DNA insert of pPPV-6 with respect to the 3' and 5' ends of the viral genome.

FIG. 5 is a diagrammatic representation of the construction of pPP405 and pPP406 from pPPV-6 and the expression vector pJS413.

FIG. 6 is a diagrammatic representation of the construction of pPP510 from pPPV-6 and the expression vector pHK413.

FIGS. 7 and 7A represent the constructions of various recombinant plasmids which direct the production of fusion proteins. The bars (broadened areas) are drawn to scale and represent the location and position of the PPV DNA coding sequence within each recombinant plasmid.

FIG. 8A is a diagrammatic representation of the construction of pPP405t and pPP406t.

FIG. 8B is a diagrammatic representation of the construction of pPP1000t.

FIG. 9A is a diagrammatic representation of the preparation of the three DNA fragments which are ligated to form pPP605.

FIG. 9B is a diagrammatic representation of the construction of pPP605.

FIG. 10 is a diagrammatic representation of the construction of pPP530.

FIG. 11 is a diagrammatic representation of the construction of pPP550.

FIG. 12 is a diagrammatic representation of the construction of pPP450.

FIG. 13 is a diagrammatic representation of the construction of pHK414-7X from expression vector pHK414.

FIG. 14 is a diagrammatic representation of the construction of pPP450A which allows for the production of PPV as a fused and as an unfused protein in E. coli transformants containing amber suppressor tRNAs.

FIG. 15 is a diagrammatic representation of the construction of p830.

FIG. 16 is a diagrammatic representation of the construction p803/405 and p989.

FIG. 17 is a diagrammatic representation of pPP450tc which contains ptac/PPV sequences.

FIG. 18 represents the construction of pPP450Atc.

FIG. 19 represents the constructions of various recombinant plasmids which contain CPV DNA coding sequences. The bars (broadened areas) are drawn to scale (based on approximated base pair values) and represent the location and position of the CPV DNA coding sequences within each plasmid.

FIG. 20 represents a 722 b.p. nucleotide sequence of the positive CPV DNA strand as determined from the PvuII site towards the 3'-end of the CPV genome of the HincII/PvuII$^{(-)}$ DNA fragment of pCP200. This sequence is based on data obtained from sequencing portions of each DNA strand.

FIG. 21 is a diagrammatic representation of the construction of pCP322.

FIG. 22 is a diagrammatic representation of the construction of pCP422.

FIG. 23 is a diagrammatic representation of the construction of pCP450.

FIG. 24 is a diagrammatic representation of the construction of pCP450A which allows for the production of CP as a fused and as an unfused protein in E. coli transformants containing an amber suppressor.

FIG. 25 represents the construction of p881.

FIG. 26 is a diagrammatic representation of the construction of pCP450tc which contains ptac/CPV sequences.

FIG. 27 is a diagrammatic representation of the construction of pCP450Atc.

FIG. 28 is a diagrammatic representation of the construction of pCP450AtcF3.

## DESCRIPTION OF THE INVENTION

This invention relates to the use of recombinant DNA techniques to produce parvovirus (PV) polypeptides which can be used as immunogens in vaccine formulations. More specifically, the production of polypeptides related to structural proteins of parvoviruses is described. These polypeptides, when used in a subunit vaccine formulation, efficiently protect the recipient animal against parvovirus infection. For instance, the PPV structural proteins or any antigenically significant portion thereof may be used in a subunit vaccine which would efficiently protect a recipient animal against infection; if the recipient animal is a pregnant sow, the fetus or embryo will also be protected from PPV infections _in utero_. The CPV structural proteins or any antigenically significant portion thereof may be used as the immunogen in a vaccine formulation to protect a recipient dog or puppy from CPV infection. Finally, a subunit vaccine prepared using a polypeptide corresponding to a structural protein of one parvovirus may be used to protect recipient animals from infection by another closely related parvovirus provided the vaccine and the related parovirus carry the same epitope (antigenic determinant). Thus, a CPV subunit vaccine prepared according to methods described herein, might be used in the protection against feline panleukopenia virus, mink enteritis, or PPV provided common epitopes exist.

The polypeptides and antisera produced according to the method of the present invention may also be used for diagnostic purposes. For instance, the polypeptides may be used as antigens for in vitro immunoassays to determine antisera titers in animals; such in vitro assays are currently known and used by those skilled in the art. Alternatively, antisera, antibodies, or monoclonal antibodies directed against the polypeptides produced herein may be used in immunoassays for the detection of parvovirus which may be present in the body fluids or feces of infected animals.

The recombinant plasmids, constructed as described herein, provide for host cell production of a protein which is a PV-related polypeptide, and which is stable and resistant to host cell degradation; such plasmids enable the generation of large quantities of a PV-related protein or fusion protein containing immunological and antigenic determinants of a PV structural protein. Since the techniques described herein may be used to produce polypeptides related to any of the PV structural proteins, it can readily be seen that various immunogens and vaccine formulations can be prepared.

The method of this invention may be divided into the following stages for the purpose of description: (1) identification and isolation of the relevant PV gene or gene fragment, (2) insertion of the gene or gene fragment into a cloning expression vector to form a recombinant DNA molecule which is used to transform single-cell organisms, (3) identification and growth of the transformed single-cell organisms which are capable of replicating and expressing the gene, (4) identification and purification of the gene product, (5) determination of the immunopotency of the gene product by assessment of its ability to elicit the production of neutralizing antibodies, and (6) formulation and use of subunit vaccines.

## ISOLATION OF PV GENETIC SEQUENCES

The PV structural protein genetic sequences may be obtained from any relevant PV strain. Isolation of the PV DNA sequence may be accomplished in a number of ways. One method which utilizes the unique hairpin structure of the ends of the PV genome involves propagating PV and isolating the viral single-stranded DNA from purified virus. The single-stranded genome can be prepared for insertion into an appropriate cloning vector by converting the genomic DNA to a double-stranded molecule by treatment with a DNA polymerase such as E. coli DNA polymerase or the Klenow fragment of DNA polymerase I; the 3'-hairpin structure of the viral genome serves as the primer for this reaction.

Once the DNA polymerase reaction is complete, the hairpin ends of the double-stranded DNA molecule must be modified for insertion into a DNA cloning vector. Any appropriate cloning vector may be used, including but not limited to plasmid vectors, phage vectors, cosmids etc. A number of approaches for cloning are possible, including but not limited to the following: (1) the double-stranded DNA molecule may be first ligated and then cleaved at the hairpin termini using a single-strand specific endonuclease such as S1 nuclease. This results in a blunt ended double-stranded DNA molecule, which, due to the ligation step, retains the terminal portion of the DNA molecule. In an alternative embodiment, (2) the double-stranded DNA molecule may be treated with a single-strand specific endonuclease such as S1 nuclease so that the hairpin termini are cleaved resulting in a blunt-ended double-stranded DNA molecule. Since, according to this embodiment, the DNA molecule was not ligated before S1 nuclease treatment, the position on the molecule where polymerization ceased is a nick which is susceptible to cleavage by a single-strand specific endonuclease such as S1 nuclease. Thus, the use of this method may result in a loss of the terminus of the molecule up to the position where polymerization ceased. Finally, (3) the double-stranded DNA molecule may be cleaved at restriction enzyme recognition sites that are located at or near either terminus. Depending on the restriction enzymes chosen, the resulting molecule will have blunt ends, staggered (single-stranded) cohesive termini, or a combination of both.

The strategies used for the subsequent insertion of the prepared DNA molecule into a cloning vector depend upon the nature of the ends of the molecule and cloning vector. For instance, if the double-stranded DNA molecule prepared by any method described above has staggered cohesive termini (at either one end or both ends) then it can be inserted into any appropriate cloning vector which possesses or is modified to contain complementary termini. On the other hand, if the double-stranded DNA molecule prepared above has blunt ends, it may be blunt-end ligated into any appropriate cloning vector. Finally, the termini of a double-stranded, blunt-ended DNA molecule prepared above may be modified by the ligation of DNA linkers (which encode restriction enzyme recognition sequences) or by the addition of homopolymeric tails (e.g. using the enzyme terminal deoxynucleotidyl transferase, TdT, to catalyze the polymerization of nucleotides at the 3'-termini of double-stranded or single-stranded DNA molecules) that are complementary to the termini of any appropriate cloning vector.

0117767

An alternative method for isolating and cloning PV structural protein genetic sequences involves isolating from virus-infected cells, the replicative form (RF) of the PV genome which exists as a double-stranded DNA molecule. This RF molecule may be cleaved at specific restriction sites and then ligated into a cloning vector possessing complementary termini. Alternatively, after removal of any protein bound to the termini, the RF molecule may be directly ligated into a cloning vector, or the ends of the RF molecule may be modified by homopolymeric tailing or by the ligation of nucleotide linkers that are complementary to the termini of any appropriate cloning vector (as described above).

To efficiently clone RF DNA molecules it is first desirable to purify the RF DNA after it is isolated from infected cells. The DNA preparation obtained from infected cells often contains a mixture of DNA molecules comprising single-stranded viral DNA, RF duplex DNA (monomers, dimers, etc.), and replicative intermediates containing extensive single-stranded tails; in addition, the RF DNA may contain a number of nicks or small gaps.

The RF duplex monomer may be isolated or purified by removing all single-stranded DNA from the DNA preparation (thus removing viral DNA, replicative intermediates with single-stranded tails, and RF DNA that is extensively nicked). In order to remove or bind DNA containing any single-stranded regions, the DNA preparation may be fractionated by chromatography using a benzylolated DEAE (diethylaminoethyl) resin (Hirt, 1967, J. Mol. Biol. 26:365-369). After such chromatography of the DNA preparation the single-stranded DNA molecules remain bound to the resin and the double-stranded DNA molecules (RF form) which do not bind to the resin are collected. Subsequently, the RF monomers may be isolated from the mixture of monomers, dimers, etc., by gel electrophoresis; however, the majority of RF DNA (e.g., 90-95%) is usually monomeric. Finally, it may be desirable to repair any remaining nicks or small gaps in the RF monomeric DNA by treatment with a DNA polymerase and DNA ligase before performing tailing reactions or restriction endonuclease cleavage reactions necessary to prepare the RF DNA for cloning.

In yet another embodiment of the present invention, the sequences encoding the structural proteins of PV may be isolated in the mRNA form which then may be processed to make cDNA which can be cloned by methods previously described in this application (see Section 2.1) or by methods disclosed in patent application Ser. No. 407,696, filed August 12, 1982. Any other methods used to clone cDNA or mRNA may be used in this embodiment of the present invention. Thus, mRNA may be isolated from infected cell lysates by procedures such as precipitation using lithium chloride, and/or column chromatography using oligo dT (or Poly U) immobilized on a solid support such as dextran, cellulose, polyacrylamide, agarose, etc. The isolated mRNA may be then converted to cDNA using RNA directed DNA polymerase (e.g., reverse transcriptase) and the cDNA molecules may be either directly ligated into cloning vectors (i.e., blunt end ligation) or the cDNA molecules may be modified for insertion into a cloning vector by tailing using TdT or by ligating appropriate DNA linkers onto the termini of the cDNA.

Regardless of the method used to obtain the PV genetic sequences, after annealing and/or ligating the tailed PV genetic sequence, and cloning vector, the resultant recombinant molecules may be used to transform appropriate host cells. The recombinant molecules which may then be isolated from transformants, may be analyzed by restriction endonuclease digestion and gel electrophoresis in order to map or characterize the inserted PV DNA sequence.

## INSERTION OF THE PV DNA FRAGMENTS INTO CLONING EXPRESSION VECTORS

Selected fragments of the PV DNA (e.g., by analogy to other PV genomes which have been characterized, such as minute virus of mice, fragments from the 5'-region of the PV genome presumably encode the PV structural proteins) are inserted into an appropriate expression vector, i.e., a vector which contains the necessary elements for transcription and translation of the inserted gene (see Roberts and Lauer, 1979, Methods in Enzymology, 68:473). The PV DNA fragments which are inserted or ligated into cloning expression vectors may be derived from the viral genome, from the RF DNA of the virus, from recombinant DNA molecules containing PV DNA sequences, or may be synthesized. Thus, any PV gene (or any portion thereof) which is to be inserted into an expression vector may be isolated from any appropriate source, including but not limited to the viral genomic DNA, RF DNA, recombinant vectors containing PV sequences, cDNA, mRNA, and replicative intermediates which have been filled in using a DNA polymerase and DNA ligase.

In order to obtain efficient expression of the gene (or a portion of the gene), a promoter must be present in the expression vector. RNA polymerase normally binds to the promoter and initiates transcription of a gene or a group of linked genes and regulatory elements (called an operon). Promoters vary in their "strength",

*i.e.*, their ability to promote transcription. For the purpose of molecular cloning it is desirable to use strong promoters in order to obtain a high level of transcription and, hence, expression of the gene. Depending upon the host cell system utilized, any one of a number of suitable promoters may be used. For instance, when cloning in an *E. coli*, its bacteriophages or plasmids, promoters such as the *lac* promoter, *trp* promoter, *rec*A promoter, ribosomal RNA promoter, the $P_R$ and $P_L$ promoters of coliphage lambda and others including but not limited to *lac*uv5, *trp*-*lac*uv5 (*tac*) hybrid promoter, *omp*F, *bla*, *lpp* and the like, may be used to direct high levels of transcription of adjacent DNA segments. Additionally, *E. coli* promoters produced by recombinant DNA or other synthetic DNA techniques may be used to provide for transcription of the inserted gene.

When cloning in a eucaryotic host cell, enhancer sequences (*e.g.*, the 72 b.p. tandem repeat of SV40 DNA, retroviral long terminal repeats or LTRs, etc.) may be inserted to increase transcriptional effeciency. Enhancer sequences are a set of eucaryotic promoter elements that appear to increase transcriptional efficiency in a manner relatively independent of their position and orientation with respect to a nearby gene. Unlike the classic promoter elements (*e.g.*, the polymerase binding site and the Goldberg-Hogness "TATA" box) which must be located immediately 5' to the gene, enhancer sequences have a remarkable ability to function upstream from, within, or downstream from eucaryotic genes; therefore, the position of the enhancer sequence with respect to the inserted PV gene is less critical.

Specific initiation signals are also required for efficient gene transcription and translation in procaryotic and eucaryotic cells. These transcription and translation initiation signals may vary in "strength" as measured by the quantity of gene specific messenger RNA and protein synthesized, respectively. The DNA expression vector, which contains a promoter, may also contain any combination of various "strong" transcription and/or translation initiation signals. Thus, any SD-ATG combination that can be utilized by host cell ribosomes may be employed; such combinations include but are not limited to the SD-ATG combination from the cro gene or the N gene of coliphage lambda, or from the E. coli tryptophan E, D, C, B or A genes. Additionally, any SD-ATG combination produced by recombinant DNA or other synthetic techniques may be used.

Any of the methods previously described for the insertion of DNA fragments into a vector may be used to ligate a promoter and other control elements into specific sites within the vector.

Accordingly, PV genetic sequences containing those regions coding for the structural proteins can be ligated into an expression vector at a specific site in relation to the vector promoter and control elements so that when the recombinant DNA molecule is introduced into a host cell the PV genetic sequence can be expressed (i.e., transcribed and translated) by the host cell. The recombinant DNA molecule may be introduced into appropriate host cells by transformation, transduction or transfection (depending upon the vector/host cell system). Transformants are selected based upon the expression of one or more appropriate gene markers normally present in the vector, such as ampicillin resistance or tetracycline resistance in pBR322, or thymidine kinase activity in eucaryotic host systems.

Expression of such marker genes indicates that the recombinant DNA molecule is intact and is replicating. Expression vectors may be derived from cloning vectors, which usually contain a marker function; such cloning vectors may include, but are not limited to the following: SV40 and adenovirus vectors, yeast vectors, bacteriophage vectors such as lambda gt-WES-lambda B, Charon 28, Charon 4A, lambda gt-1-lambda BC, lambda gt-1-lambda B, M13mp7, M13mp8, M13mp9, or plasmid DNA vectors such as pBR322, pAC105, pVA51, pACYC177, pKH47, pACYC184, pUB110, pMB9, pBR325, Col E1, pSC101, pBR313, pML21, RSF2124, pCR1 or RP4.

Cloning in a eucaryotic host system may result in the production of a polypeptide that serves as a better immunogen than those produced by procaryotic transformants due to secondary protein modifications which occur during eucaryotic metabolism. Such secondary protein modifications include but are not limited to acylation, glycosylation, methylation, phosphorylation, sulfation, etc. The processing reactions of a eucaryotic host may mimic the processing patterns of the naturally occurring PV structural proteins, e.g., the eucaryotic host cell might phosphorylate or glycosylate the clonally derived polypeptide product at sites similar to those of the natural protein.

In addition, host cell strains may be chosen which inhibit the action of the promoter unless specifically induced. In this way greater than 95% of the promoter-directed transcription may be inhibited in uninduced cells. In certain operons the addition of specific inducers is necessary for efficient transcription of the inserted DNA; for example, the lac operon is induced by the addition of lactose or IPTG (isopropylthio-

β-D-galactoside, an analog of lactose). A variety of other operons, such as *trp*, *pro*, *tac*, etc., are under different controls. The *trp* operon is induced when tryptophan is absent in the growth media; and the $P_L$ promoter of lambda can be induced by an increase in temperature in host cells containing a temperature sensitive lambda represor, *e.g.*, cI857. Thus, expression of the genetically engineered PV protein may be controlled. This is important if the protein product of the cloned gene is lethal or detrimental to host cells. In such cases, transformants may be cultured under conditions such that the promoter is not induced, and when the cells reach a suitable density in the growth medium, the promoter can be induced for production of the protein.

One such promoter/operator system is the so-called "*tac*" or *trp*-*lac* promoter/operator system (referred to as "*tac*"). This hybrid promoter is constructed by combining the -35 b.p. (-35 region) of the *trp* promoter and the -10 b.p. (-10 region or Pribnow box) of the *lac* promoter (the sequences of DNA which are the RNA polymerase binding site). In addition to maintaining the strong promoter characteristics of the tryptophan promoter, *tac* is also easily controlled by the *lac* repressor (from *lac* $I^q$). This construction may explain the higher efficiency of expression of this hybrid promoter with respect to either one of the parental promoters.

Gene expression can also be controlled at the level of translation. In host cells transformed with amber, ochre, or opal modified plasmids (See Section 5.6.3.), translation of the mRNA produced from the plasmid DNA stops at the nonsense codon and only the desired protein (unfused) is expressed. In an appropriate host (one that is able to "suppress" the nonsense codon), some of the ribosomes, "read through" the nonsense codon and express fusion protein. Since the level of suppression varies in different host cells, it is possible to regulate the ratio of protein to fusion protein by choosing the appropriate permissive host cell.

The expression of proteins maybe regulated in several other ways. For example, in order to improve the initiation of translation, an A/T DNA segment maybe inserted between the $SD^{cro}$ and the ATG initial codon for the start of translation. An oligomer which contain an A/T rich sequence may be synthesized using state-of-the-art nucleic acid chemistry. A particularly useful method is described in U.S. Patent Application Serial No. 491,099 by Kempe, et al., filed May 5, 1983 which is incorporated herein by reference. This A/T rich segment may be needed to destabilize the secondary structure of the mRNA within this region and thereby facilitate ribosome binding and subsequent translation along the message.

In many recombinant clones studied only one translational stop codon (e.g., TAG, TAA or TGA) is present for termination of translation, and that no transcriptional-stop like sequences are found in the 3' untranslated region of the clones. Therefore a triple translational stop sequence, with all 3 codons may be inserted in phase with the reading frame of the protein. Again, this linker is synthesized using methods referred to above. This construction should then provide for efficient termination of translation by assuring proper translational termination with little change of read-through into the untranslated region of the mRNA.

Recent studies have shown that the tryptophan attenuator region of the tryptophan operon provides for the efficient termination of the mRNA transcript from the DNA. This extremely stable stem and loop structure is characteristically GC-rich and posseses dyad symmetry, followed by an oligo-(T) stretch at the site of termination similar to the well known features of rho-independent transcription termination sites in procaryotes. By taking advantage of this system, a sequence coding for the attenuator portion of the tryptophan operon may be placed 3' of the translational stop codons of the genome. This construction may now allow for the effective termination of transcription of the mRNA.

The regulation of expression of any protein includes, but is not limited to the construction and use of promoter/operator systems, A/T rich sequences, transcriptional and translational termination sequences, and any other modified protein construct (either naturally occurring or synthetically made) which provides an "optimal" system for expression of a protein in a compatible host cell.

## PREPARATION OF FUSION PROTEINS

To maximize the level of expression of the cloned PV sequences it may be desirable to ligate these sequences to a gene encoding another protein, such as a host cell protein. If the host cell protein is large and inherently contains an assayable function additional advantages are obtained because expression of the ligated genes may result in a fusion protein product that can be identified on the basis of its large molecular weight and assayable function. For example, production of a PV-related

polypeptide fused to ß-galactosidase, hereinafter referred to as PV/ß-galactosidase fusion protein offers several advantages for cloning and expression of a PV genetic sequence in an E. coli host. First, fusion protein production simplifies the identification and isolation of the protein product. The unfused PV-related protein (i.e., unfused to ß-galactosidase) produced by E. coli transformants is smaller than the fusion protein and as such might co-migrate with several other host proteins when analyzed by SDS-polyacrylamide gel electrophoresis. However, a fusion protein can often be easily detected and identified by SDS-polyacrylamide electrophoresis (SDS-PAGE) due to its unique high molecular weight. Second, retention of enzymatic function of the host protein can be used to identify transformants and may also allow for an approximation of the level of protein production (hence expression) directed by the vector using a colorimetric assay specific for ß-galactosidase activity according to the method of Miller (pages 47-55, and 352-355 in Experiments in Molecular Genetics, Cold Spring Harbor Press, New York, N.Y., 1972). Third, as discussed in Section 2.1., hybrid gene sequences, e.g., procaryotic nucleotide sequences ligated to eucaryotic nucleotide sequences, when expressed in procaryotes result in production of fusion proteins which may be protected from host cell proteolysis.

The present invention is not limited to the production of a PV/ß-galactosidase fusion protein; any gene of either eucaryotic or procaryotic origin may be ligated in phase with a PV genetic sequence to provide advantages similar to the PV/ß-galactosidase fusion protein product. Examples include, but are not limited to: galactokinase; trp D, E or leader; pilus genes; and eucaryotic genes, such as thymidine kinase, ß-globin,

SV-40 T-antigen, bovine growth hormone or Rous Sarcoma Virus transforming gene.

In order to construct a hybrid gene which encodes a fusion protein, the two genes must be joined within open coding sequences such that the reading frame of each is maintained. Also, as previously explained, if the host cell is a strain which represses or inhibits the action of the promoter, the fusion protein will be produced only in response to induction.

## IDENTIFICATION OF THE PROTEINS PRODUCED

Once transformants which contain the correct DNA construction are identified, analyses of the immunoreactivity and antigenicity of the products expressed by the transformants (e.g., fusion proteins or PV-related polypeptides) are required. Immunological analyses of these expression products of the PV genetic sequences are especially important because the ultimate goal is to use the PV-related products (e.g., the fusion proteins, PV-related polypeptides or proteins) so produced in vaccine formulations. The analysis of immunoreactivity is most easily carried out using antisera directed against the PV structural protein, whereas antigenicity may be evaluated by determining test animal antisera titers which develop in response to immunization with the PV-related product and the ability of the antisera so produced to neutralize PV infection.

Identification of the PV-related products described in this invention is, therefore, based upon two requirements. First, the PV-related product should be produced only in response to induction of the promoter. Second, the PV-related product should be immunoreactive with antibodies directed against the relevant parvovirus or with monoclonal antibodies directed against the PV structural protein itself; the PV-related product should be immunoreactive whether it results from the expression of an entire PV gene sequence, a portion of a PV gene sequence or from two or more genetic sequences which are ligated to direct the production of a fusion protein. This reactivity may be demonstrated by standard immunological techniques, such as immunoprecipitations, immunodiffusion, radio-immune competition, immunoelectrophoresis or the immunological detection of proteins which were separated by polyacrylamide gel electrophoresis and then transferred to nitrocellulose.

## PURIFICATION OF THE PROTEINS PRODUCED

Cells containing the cloned gene are grown up in a large volume, and the protein produced after induction of the promoter is isolated from such cells or from the medium if the protein is excreted. The protein may be isolated and purified either by standard chromatography including ion exchange, affinity or sizing resins, by centrifugation, or by any other standard technique for the purification of proteins.

Since certain fusion proteins form aggregates when overproduced in cells and when in solution, a method for isolating aggregate-forming proteins is particularly useful for isolating the fusion proteins produced in the present invention. These fusion proteins can then be used in vaccine formulations. Purification of fusion proteins (hereinafter referred to as aggregate purification) involves the extraction, separation and/or purification of aggregate-forming proteins from protein mixtures such as lysates of cell cultures by disruption of cells followed by washing the aggregated material. Additionally, the aggregated material may be solubilized by the addition of a solvent that is both a strong hydrogen acceptor and a strong hydrogen donor (or a combination of solvents each having one of these properties); the aggregate-forming proteins may then be precipitated by dilution with a compatible buffer. Suitable protein solvents include, but are not limited to urea (from about 4M to about 8M), formamide (at least about 80%, volume/volume basis), and guanidine hydrochloride (from about 4M to about 8M). Some solvents which are capable of solubilizing aggregate-forming proteins, for example SDS (sodium dodecyl sulfate), 70% formic acid, are inappropriate for use in this procedure because subsequent precipitation of the solubilized aggregate-forming proteins has proved to be inefficient. Although guanidine hydrochloride and other similar agents are denaturants, studies indicate that this denaturation is not irreversible and that renaturation may occur upon removal (by dialysis, for example) or dilution of the denaturant, allowing re-formation of immunologically and/or biologically active protein.

One embodiment of the fusion protein isolation technique is outlined as follows (hereinafter referred to as the non-denaturing aggregate purification procedure): the cell pellet is quick frozen using dry ice/methanol, weighed, and 3-4 g of cells are resuspended in at least 25 mℓ of a buffer solution [e.g., 50 mM Tris-HCl (tris hydroxymethylaminomethane-hydrochloride), pH 8.0, 2mM EDTA (ethylenediaminetetraacetic acid) and 200 mM NaCl]. That is, the cells are suspended in a buffer solution at an approximate concentration of from about 100 to 200 grams of cells/liter. Concentrations less than about 160 grams/liter are preferred. To this suspension lysozyme is added to a final concentration of about 130 μg/mℓ and the resulting mixture is allowed to stand at 4°C for 20 minutes with occasional shaking. Nonidet P40 (NP-40, Shell trademark, polyoxyethylene (9) p-*tert*-octylphenol), a non-ionic detergent used to solubilize membranes, is added to a final concentration of about 0.1% and the solution mixed. Then, the suspension is homogenized for approximately 1 minute using a Polytron grinder (Brinkman Instruments, Westbury, N.Y.) or equivalent.

The suspension is centrifuged at 8,000 x g for 30 minutes, and the pellet resuspended in a wash buffer, *e.g.*, 20mM Tris-HCl (pH 7.2), 1mM EDTA, 150mM NaCl and 2% Triton-X 100 [polyoxyethylene (9-10) p-*tert*-octylphenol, a non-ionic detergent], and homogenized with the Polytron grinder. This step of centrifugation, washing, and grinding may be repeated to further wash the pellet and remove as much cellular debris as possible.

Alternatively, the pellet of aggregates may be further treated by the addition of a denaturant (hereinafter referred to as the denaturing aggregate purification procedure) as follows: The suspension is centrifuged, the supernatant removed completely and the pellet resuspended in about one-fifth volume of 6M guanidine hydrochloride (in distilled water). For instance, 3 g of cells washed with 25 mℓ of buffer should be resuspended at this step in 5 mℓ of 6M guanidine hydrochloride solution. It may be difficult to resuspend the pellet at this stage and sonication or homogenization may be required in order to obtain a homogenous solution. The solution is allowed to stand at 22°C for 20 minutes and is then centrifuged at 8,000 x g for 30 minutes to remove debris, saving the supernatant which at this point contains the fusion protein.

The fusion protein is precipitated from the guanidine hydrochloride supernatant by the addition of about four volumes of aqueous buffer. Almost any aqueous buffer may be used at this stage; however, the addition of a small amount of non-ionic detergent, such as 0.5% NP-40 or Triton X-100 may be helpful. This suspension is allowed to stand at 4°C for 30 minutes and is then centrifuged at 8,000 x g for 10 minutes. The supernatant is discarded, the pellet (containing the fusion protein precipitate) is resuspended in an appropriate buffer, e.g., Phosphate Buffered Saline (PBS) in any appropriate volume. Brief sonication or homogenization may aid in obtaining a homogenous suspension or slurry (since aggregate-forming proteins are insoluble in water).

It may be desirable to remove any remaining DNA from the suspension of aggregates or the fusion protein precipitate before using either as an immunogen. To this end the suspension of aggregates or fusion protein precipitate is pelleted and resuspended in wash buffer containing no detergent. To either suspension a final concentration of about 10mM $MgCl_2$ and 2 µg/mℓ Deoxyribonuclease I (P.L. Biochemicals, Milwaukee, Wis.) is added. After incubation at 37°C for 30 minutes, the suspension of aggregates or fusion protein precipitate is pelleted by centrifugation, washed with buffer, repelleted, and resuspended in fresh buffer, thus removing most of the deoxyribonuclease.

Another embodiment of the fusion protein isolation technique involves purification of aggregate proteins from larger volumes of culture. This method is a modification of the aggregation purification procedure described <u>supra</u>. The cell pellet is quick frozen in a dry ice/ ethanol bath, weighed, and approximately 30-60 g of cells are resuspended in 125 mℓ of BIP buffer (50mM Tris pH 8.0, 10mM EDTA) at 37°C with shaking for 10 minutes. The partially resuspended cells are homogenized using a Polytron grinder (Brinkman Instruments, Westbury, N.Y.). To this suspension 50 mg of lysozyme (10 mg/mℓ in BIP) is added while the cells are further homogenized and the cells are placed at room temperature for 20-30 minutes. Nonidet P40 (described <u>supra</u>) is added to a final concentration of 0.1% and the mixture is homogenized using a Polytron grinder for 5 minutes.

The cell lysate is centrifuged at 26,890 x g for 20 minutes using a SS-34 Sorvall rotor and the supernatant is disgarded. The pellet is resuspended in 150 mℓ B2P buffer (10mM Tris pH 7.5, 1MM EDTA, 1% Triton X-100), homogenized for 2-5 minutes, and centrifuged for 20 minutes at 26,890 x g. This step is repeated, and the pellet (containing the aggregate protein) is then washed two times with water. Finally, the aggregate protein is resuspended in water using the Polytron grinder.

In an alternate embodiment of the present invention, aggregate protein is solubilized and soluble unfused viral protein is isolated. Aggregate protein (4 mg/mℓ) is suspended in 20 mℓ of buffer (75mM Tris-HCl, 10M urea, and 0.5M ß-mercaptoethanol), sonicated for 2 minutes, and heated at 65°C for 30 minutes. the suspension is centrifuged at 20,000 x g for 20 minutes, and the supernatant is diluted by the addition of an equal volume of water.

The sample is applied to a 30 mℓ DE52 (Diethylaminoethyl cellulose, Whatman Chemical Separation Ltd., Kent, England) column. The column is washed with several volumes of buffer (30mM Tris-HCl, 4M urea, 50mM ß-mercaptoethanol, pH 9.0). The protein is eluted from the column using a gradient from 0 to 150mM NaCl (400 mℓ of buffer containing 30mM Tris-HCl, 4M urea, 50mM ß-mercaptoethanol, pH 9.0).

Fractions are collected and an aliquot of each fraction is analyzed by SDS-PAGE for the presence of unfused protein. Fractions containing unfused protein are pooled and dialyzed against 30mM Tris-HCl, pH 9.0. Finally, the unfused protein is neutralized and concentrated at least 10 fold by ultrafiltration using an Amicon Model #8050 Ultrafiltration Cell (Amicon Corp., Danvers, MA).

## PREPARATION OF UNFUSED PROTEIN

As previously explained, transformants which produce unfused proteins, generally do so in smaller quantities than transformants which produce fusion proteins; this is true even when the gene sequence for the unfused protein is under the control of an inducible promoter. In addition, the unfused proteins produced by bacterial transformants may be less stable than fusion proteins. In an alternate embodiment of the present invention, a host cell transformant can be engineered to produce, for example, large quantities of both fused PPV/ß-galactosidase fusion protein and unfused BGH (bovine growth hormone) related proteins which will coaggregate and can be purified easily. (See U.S. Patent Application Temporary Serial No. 548,917 filed November 7, 1983 by George *et al.*) Three embodiments of this mode of the invention are described in the subsections below.

CO-TRANSFORMATION OF HOST CELLS

Any appropriate host cell can be co-transformed with two or more plasmids. One plasmid carries the desired unfused protein gene and one plasmid carries a fusion protein gene. Each gene should be under the control of a promoter and translational control elements. The same or different promoter systems may be used to control expression of each gene. If a different promoter is used for each gene sequence then the ratio of the expression of the sequences may be controlled by varying the degree of induction of each promoter.

In addition, the two plasmids may carry different selectable drug markers for antibiotic resistance (e.g., tetracycline and ampicillin resistance) and may be maintained within the same cell if grown in the presence of the two antibiotics. With both plasmids in the cell, expression of the fusion protein and the labile unfused protein can occur.

These transformed cells produce insoluble aggregates which contain both the unfused proteins and the large fusion protein. There is no requirement that the fusion protein be a PV fusion protein. In fact, any combination of fusion protein with unfused protein will have the aggregation and stabilization properties.

For example, a significant stabilization of the unfused Bovine Growth Hormone (hereinafter referred to as BGH) protein was observed when two plasmids were used to transform one host cell, each plasmid directing the expression of its respective protein: unfused BGH and porcine parvovirus/ß-galactosidase fusion protein (PPV/ß-galactosidase). Pulse-chase experiments indicated that the half-life of the unfused modified-BGH proteins had increased from a 1-2 minute level to between 30 minutes and 1 hour. Additionally, when these proteins were highly expressed within the cell, they form insoluble aggregates which contain both the unfused BGH protein and the large PPV/ß-galactosidase fusion protein. Subsequent purification of the aggregate proteins indicated that the protein could be readily obtained in gram quantities. The cloning and expression of nucleotide sequences encoding BGH is described in U.S. Pat. Application Temporary Serial No. 548,917.

## TRANSFORMATION OF HOST CELLS WITH ONE PLASMID CARRYING TWO GENES

In an alternate embodiment of the present invention, plasmids which carry both the unfused protein gene and the larger fusion protein gene on a single vector are designed and constructed. As described supra for the co-infection scheme, this embodiment of the invention is suitable for any combination of fusion protein and any unfused protein.

The plasmids are constructed such that each gene on the plasmid has its own promoter and expression control elements. The same or different promoter systems may be used to control expression of each gene, therefore this embodiment of the present invention also allows for the regulation of the ratio of the fusion protein and the desired gene product.

## MODIFICATION OF THE FUSION PROTEIN GENE

In one embodiment of the present invention, a recombinant plasmid which encodes a PV fusion protein is altered at the junction of the two gene sequences which comprise the fusion protein gene. A chain termination sequence such as amber (TAG), ochre (TAA), or opal (TGA) is located between the two gene sequences; the chain terminator must be in phase with the translational reading frames of both gene sequences. Such an alteration may be accomplished by cleaving the plasmid at a restriction site (or sites) located between the two gene sequences and then ligating a nucleotide linker sequence encoding a chain terminator such as amber, ochre, or opal into the cleaved site on the plasmid so that the chain terminator is in phase with the translational reading frames of both gene sequences. (See for example U.S. Pat. Application Serial No. 510,551, by Watson _et al._, filed July 6, 1983 which is herein incorporated by reference.)

Introduction of these amber, ochre, or opal modified plasmids into a host cell containing the appropriate tRNA suppressors results in the synthesis of both an unfused protein as well as a fusion protein (since suppression is significantly less than 100%). A tRNA suppressor is a tRNA that has undergone a mutation that allows the tRNA to recognize the termination codon and results in the occasional but not regular insertion of an amino acid at the site of the termination codon. Therefore, host cells carrying the suppressor tRNA characteristically produce both the unfused protein and a fusion protein of normal length. Every nonsense or termination suppressor has a characteristic efficiency, indicated by the fraction of protein chains that are completed.

There are at least two ways to introduce the amber, ochre or opal modified plasmids into a suppressor cell background: (1) the transformant (_i.e._, a host cell transformed with amber, ochre or opal modified plasmid) can be infected with a lysogenic transducing phage that carries the appropriate suppressor tRNA gene (e.g., Ø80 pSU3 carries the SupF suppressor of amber mutations); or (2) the amber, ochre or opal modified plasmids can be used to transform cell lines which contain suppressor tRNAs of amber, ochre, or opal respectively. Examples of such strains include but are not limited to LE392 (containing supE and supF suppressors of amber mutations), YMC (containing supF), and C600 (supE). The various amber suppressor tRNA genes in E. coli include but are not limited to: supB, supC, supD, supE, supF, supG, supL, supM, supN, supO, supP, supU, supV; the various ochre suppressor tRNA genes in E. coli include but are not limited to: supB, supC, supG, supL, supM, supN, supO, supV; and the various opal suppressor tRNA genes in E. coli incude but are not limited to: supK (see Bachmann and Low, 1980, Microbiological Reviews 44(1): 1-56).

The host cells containing the appropriate suppressor tRNA gene are transformed with the amber, ochre, or opal modified plasmids and can produce the protein as both a fusion protein and as an unfused protein (the proportions of fused to unfused protein produced depends upon the extent of suppression in the host cell).

The amber, ochre, or opal modified plasmids may be further modified to ensure that translation of the corresponding mRNA transcript will terminate at the 3'- terminus of the fusion protein gene. Proper chain termination is important because it contributes to the overall efficiency of translation. A number of approaches which may be used to effect chain termination are described below. In one embodiment, all three chain termination sequences may be inserted in tandem at the 3'- region of the fusion protein gene so that they are in phase with the translational reading frame of the gene sequence. The presence of the chain terminator sequences in tandem will reduce the chance of read-through, consequently, translation will terminate at the chain termination codons on the mRNA transcript.

Alternatively, one or more chain termination sequences of the appropriate type may be inserted into the 3'- region of the fusion protein gene. For example, an amber modified plasmid may be further modified by the insertion (in phase) of one or more opal or ochre sequences at the 3'- end of the fusion protein gene. When this plasmid is inserted into a host cell containing an amber tRNA suppressor (such as supD, supE, supF, supP, supU, supV in E. coli) that does not suppress ochre or opal, the host cell will produce both the fusion protein and the unfused protein and translation of the fusion protein will terminate at the location of the opal and/or ochre codons located at the 3'- end of the mRNA transcript.

In a similar fashion, an opal modified plasmid may be further modified by the insertion (in phase) of one or more amber or ochre sequences at the 3'- end of the fusion protein gene. When this plasmid is inserted into a host cell containing an opal tRNA suppressor (such as supK in E. coli) that does not suppress amber or ochre, the host cell will produce both the fusion protein and the unfused protein and translation of the fusion protein will terminate at the location of the amber and/or ochre codons located at the 3'- end of the mRNA transcript.

Similarly, an ochre modified plasmid may be further modified by the insertion (in phase) of one or more opal sequences at the 3'- end of the fusion protein gene. When this plasmid is inserted into a host cell containing an ochre tRNA suppressor (such as supB, supC, supG, supL, supM, supN, supO, supV in E. coli) that does not suppress opal, the host cell will produce both the fusion protein and the unfused protein and translation of the fusion protein will terminate at the location of the opal codon (or codons) located at the 3'- end of the mRNA transcript.

## FORMULATION OF A VACCINE

The purpose of this invention is to produce, by recombinant DNA techniques, a polypeptide related to a PV structural protein which may be used as an immunogen in a subunit vaccine to protect against PV infections. If the PV-related protein produced is immunoreactive with specific PV neutralizing antibodies, it would be expected that the PV-related protein would elicit an immune response capable of neutralizing the relevant virus *in vivo*. Vaccines made from genetically engineered immunogens should be safer than conventional vaccines made from attenuated or killed virus because there is no risk of infection of the recipient. Alternatively, the genetically engineered PV product may be used to produce antibodies for use in passive immunotherapy or as a diagnostic tool. For instance the PV specific antibody may be labeled with a dye, an enzyme such as peroxidase, ferritin, a fluorescent compound, or a radioactive compound. Such a labeled antibody may be used to detect PV antigens (and, therefore, parvovirus) in various body fluids obtained from animals suspected to be infected with PV. Such techniques for immunoassay *in vitro* diagnostic assays are well known in the art and may be applied to body fluids obtained from mucous membranes, semen, blood and excrement. Finally, the genetically engineered product may be used as antigen in an *in vitro* immunoassay to determine and monitor the titers of antisera in vaccinated animals.

Although the PV/ß-galactosidase fusion protein product itself may be useful in a vaccine formulation, it may be advantageous to first remove and/or modify all or part of the ß-galactosidase moiety in order to produce a more effective PV-related immunogen.

While whole cells or crude extracts of induced transformants may be used to vaccinate animals, the genetically engineered PV-related protein may also be isolated and purified from the host cells using standard protein isolation techniques or techniques previously described (see Section 5.5). The final purified product may then be adjusted to an appropriate concentration and formulated with any suitable vaccine adjuvant and packaged for use. Suitable adjuvants include but are not limited to: surface active substances, *e.g.*, hexadecylamine, octadecylamine, lysolecithin, dimethyl-dioctadecylammonium bromide, N,N-dioctadecyl-N'-N-bis(2-hydroxyethyl-propane diamine), methoxyhexadecylglycerol, and pluronic polyols; polyanions, *e.g.*, pyran, dextran sulfate, poly IC, polyacrylic acid, carbopol; peptides, *e.g.*, muramyl dipeptide, dimethylglycine, tuftsin; oil emulsions; and alum. Finally, the protein product may be incorporated into liposomes for use in a vaccine formulation, or may be conjugated to polysaccharides or other polymers.

The genetically engineered DNA molecules described herein allow great flexibility for vaccine production. For example, a vaccine could be formulated using a PV-related protein produced by transformants containing any portion of the genetic sequences of PV encoding for a structural protein or multiple copies of such sequences in tandem. The PV genetic sequences (or portion thereof) may be ligated to genes that encode other immunogens so that the fused protein product could be used in the preparation of multivalent vaccines. Additionally, the PV genetic sequences (or portion thereof) could be ligated to other PV gene sequences (or portions thereof) in any combination. Finally, the genetic sequences of PV may be reorganized to increase the immunogenicity of the vaccine. For example, the PV genetic sequences may be altered so that the protein product presents specific epitopes to the immune system (e.g., an antigenic site of the protein that is normally only partially exposed may be presented to the immune system); or the regions of the genetic sequences that encode immunosuppressive portions of the protein can be deleted; or the regions that separate epitopes can be deleted.

## EXAMPLE: PORCINE PARVOVIRUS

The PPV genomic DNA was isolated from purified PPV virions which were propagated in fetal pigs *in utero*. The single-stranded genomic DNA was converted to double-stranded DNA, ligated into pBR322, and used to transform *E. coli*. The recombinant plasmids isolated from *E. coli* transformants were analyzed by restriction endonuclease digestion and the one containing the largest PPV DNA segment (pPPV-6) was further analyzed in order to determine the orientation of the PPV DNA sequence within the recombinant plasmid.

*E. coli* was transformed with selected PPV DNA sequences inserted into expression vectors. These PPV sequences were ultimately derived from the recombinant plasmid pPPV-6. The PPV gene product which was isolated as a fusion protein was then formulated for use as an immunogen. The procedure is described in detail below.

## GENERAL PROCEDURES USED FOR PREPARATION OF THE PLASMIDS

The following subsections describe the general procedures and materials used for host cell transformation, DNA isolation, enzyme reactions and ligation reactions. Unless otherwise indicated in the text, these procedures were utilized in the practice of this example of the present invention.

## PLASMID DNA ISOLATION

Host cell bacteria *Escherichia coli*, (*E. coli*) were transformed (Gautier and Bonewald, 1980, Molec. Gen. Genet. 178:375) and large (microgram) quantities of plasmid DNA were isolated from cultures of *E. coli* transformants grown in LB medium (Miller, Experiments in Molecular Genetics, Cold Spring Harbor Press, New York, N.Y., 1972) to which casamino acids have been added (about 2 g per liter) and approximately 0.016M $MgSO_4$. Plasmids were isolated from cells in late logarithmic stage of growth using a modification of the method of Guerry *et al.* (1973, J. Bacteriol. 116:1063).

## CONDITIONS FOR RESTRICTION ENZYME DIGESTIONS

Restriction enzymes used in the present application were obtained from New England Biolabs, Inc., Beverly, Ma., unless otherwise indicated. An enzyme unit is defined as the amount required to digest 1.0 µg of

lambda DNA in 15 minutes at 37°C in a total reaction mixture of 50 µl.

All restriction enzyme total digestions were accomplished under the following conditions: each 1 µg DNA was incubated with 0.5 to 5.0 units of enzyme at 37°C for 60 minutes in 20 µl buffer. Partial digestions were accomplished by modifying the conditions used for total digestion as follows: each 1 µg DNA was incubated with 0.1 units of enzyme at 37°C for 15 minutes. Thus, the reaction conditions for partial digestions were adjusted to obtain an average of one cleavage per DNA molecule. When restriction enzyme reactions were terminated before electrophoresis they were terminated by the addition of 0.1% sodium dodecyl sulfate (SDS) or by heating the reaction mixture at 65° for 10 minutes.

## RESTRICTION ENZYME BUFFERS

The buffer used for BamHI digestions consisted of: 150mM NaCl, 6.6mM Tris-HCl (pH 7.4), 6.6mM $MgCl_2$ and 6.6mM ß-mercaptoethanol (ß-ME).

The buffer used for AvaI, BglII, BstEII, EcoRV, HaeIII, HincII, HindIII, HinfI, NcoI, PvuII, RsaI, or XmnI digestions consisted of: 60mM NaCl, 6.6mM Tris-HCl (pH 7.4), 6.6mM $MgCl_2$ and 6.6mM ß-ME.

The buffer used for EcoRI digestions consisted of 50mM NaCl, 100mM Tris-HCl (pH 7.5), and 5mM $MgCl_2$.

The buffer used for PstI digestions consisted of 50mM $(NH_4)_2SO_4$, 20mM Tris-HCl (pH 7.5), and 10mM $MgCl_2$.

The buffer used for SmaI digestions consisted of 20mM KCl, 6mM Tris-HCl (pH 8.0), 6mM $MgCl_2$, and 6mM ß-ME.

The buffer used for SacI digestions consisted of: 6.6mM Tris-HCl (pH 7.4), 6.6mM $MgCl_2$ and 6.6mM ß-ME.

The buffer used for AccI digestions consisted of: 6mM NaCl, 6mM Tris-HCl (pH7.5), 6mM $MgCl_2$, and 6mM ß-ME.

## MODIFICATION OF DNA

DNA polymerases catalyze the stepwise elongation of a DNA chain. Chain growth is in the 5' to 3' direction (i.e., addition of nucleotides occurs at the 3'-terminus) and the sequence of the polymer is determined by that of the template because the incoming nucleotide must be complementary to the template, i.e., form the correct base pair with the template strand. The known DNA polymerases cannot initiate chain synthesis, thus DNA synthesis requires a primer strand with a free 3'-hydroxyl terminus annealed to a template strand. Chain elongation proceeds by the nucleophilic attack of the 3'-hydroxyl terminus of the primer on the 5'-phosphate of the incoming nucleotide. The new chain is base paired and antiparallel to the template strand. As a result of the DNA polymerase reaction the single-stranded template strand is "filled-in" or converted to a double-stranded DNA molecule.

A second important feature of the DNA polymerases is the "proof-reading" function. A 3' to 5' exonuclease activity associated with DNA polymerase I acts on nonbase-paired termini and can degrade both single- and double-stranded DNA in the 3' to 5' direction yielding mononucleotides. Thus an incorrectly added nucleotide is removed before polymerization continues, and the fidelity of the enzyme is further enhanced. DNA polymerase I also has a 5' to 3' exonuclease activity specific for double-stranded DNA (yielding 5'-mononucleotides and oligonucleotides) which can also excise mismatched regions in DNA.

Proteolytic treatment of DNA polymerase I by the method of Klenow (Klenow et al., 1971, Eur. J. Biochem. 22:371) yields two fragments, large and small. The large fragment or Klenow fragment (76,000 daltons) retains the polymerase and 3'- 5' exonuclease activity whereas the small fragment retains the 5'- 3' exonuclease activity. One unit of DNA polymerase I or DNA polymerase I-large fragment (New England Biolabs, Beverly, Ma.) is defined as the amount converting 10 nmoles of deoxyribonucleotides to an acid insoluble form in 30 minutes at 37°C. The assay conditions for both enzymes are the same except that the reaction mixture for DNA polymerase I contains 67mM $KPO_4$ (pH 7.5) while the reaction mixture for the Klenow fragment contains 40mM $KPO_4$ (pH 7.5) in the following buffer: 6.6mM $MgCl_2$, 1.0mM ß-ME, 2mM dAT copolymer, 33µM dATP, 33µM $^3$H-dTTP and enzyme.

In the present application, when DNA polymerase Klenow fragment was used to fill in DNA single-stranded or cohesive ends that result from restriction enzyme cleavage, the following procedure was employed: restriction enzyme reactions were terminated by heating at 68°C for 10 minutes. To the same reaction mixture a final concentration of 50µM of each deoxynucleoside triphosphate was added and for each microgram of DNA in the reaction mixture 10-100 units of DNA polymerase Klenow fragment was added. In other words an excess of DNA polymerase Klenow fragment was used to ensure that single-stranded ends were completely filled in.

Terminal deoxynucleotidyl transferase (TdT) catalyzes the polymerization of nucleotides at the 3'-termini of single- or double-stranded DNA molecules. Unlike DNA polymerase, no template is necessary. In addition to its deoxynucleotide polymerizing activity, TdT catalyzes the limited addition of ribonucleotides. In recombinant DNA technology, TdT is used to attach homopolymeric tails to the 3'-termini of DNA molecules. The TdT enzyme is activated by divalent cations, such as $Mg^{++}$. In the presence of $Mg^{++}$, TdT catalyzes the polymerization of deoxynucleotides to the 3'-terminus of single-stranded DNA; thus either single-stranded DNA or 3'-staggered ends may be tailed. Interestingly, if the TdT reaction mixture contains $Co^{++}$ instead of $Mg^{++}$, homopolymeric tails can be successfully attached to the 3'-terminus of double-stranded DNA whether or not the end of the molecule is staggered (either 3'- or 5'-staggered ends) or blunt. One unit of TdT enzyme (P.L. Biochemicals, Milwaukee, Wis.) incorporates 1 nmol d(A) into oligonucleotides per hour at 37°C when using $d(pT)_6$ as primer.

S1 nuclease degrades RNA or denatured DNA (<u>i.e.</u>, single-stranded DNA) into mononucleotides, but will not (under proper conditions) degrade double-stranded DNA or DNA/RNA hybrids. One unit of S1 nuclease (Boehringer Mannheim, Indianapolis, Ind.) is defined as the amount of enzyme required to acid solubilize 1 µg of denatured calf thymus DNA in 30 minutes at 37°C. Unless otherwise indicated, the reaction buffer used for S1 nuclease consisted of 30mM sodium acetate (pH 4.6), 250mM NaCl, 1mM $ZnSO_4$ and 5% glycerol. S1 digestions were

accomplished by incubating 2000 units of enzyme with 0.1 µg DNA and 20 µg RNA at 45°C for 30 minutes.

Mung-bean nuclease is a single-strand specific enzyme which hydrolyzes the phosphodiester bonds in DNA and RNA yielding 5'-phosphoryl terminated products. The enzyme has been demonstrated to exhibit a pronounced preference for single-stranded DNA. One unit of mung-bean nuclease (P.L. Biochemicals, Inc., Milwaukee, Wis.) produces one microgram acid soluble product from 0.5 mg/mℓ heat denatured DNA per minute at 37°C in a reaction mixture containing 0.03M sodium acetate (pH 4.6), 0.05M NaCl, 1mM zinc chloride, and 5% glycerol. Unless otherwise indicated, mung-bean nuclease digestions were carried out in this buffer.

BAL 31 nuclease is a multifunctional enzyme that contains a highly specific single-stranded endodeoxyribonuclease activity and a processive exonuclease activity that simultaneously degrades both the 3'- and 5'-termini of double-stranded DNA (dsDNA). One unit of nuclease BAL 31 (New England Biolabs, Inc. Beverly, Ma.) is defined as the amount required to release 1.0 µg of acid soluble nucleotides from denatured calf thymus DNA (650 µg/mℓ) in one minute at 30°C. The reaction buffer used for BAL 31 consisted of: 20mM Tris-HCl (pH 8.0), 600mM NaCl, 12mM $CaCl_2$, 12mM $MgCl_2$, 1.0mM EDTA and DNA. Incubations were at 30°C; BAL 31 digests were accomplished by incubating 30 µg of DNA with 0.5 units BAL 31 for 1, 2, 4, 6, 8 and 10 minutes. Reactions were terminated by the addition of EDTA to a final concentration of 50mM or by the addition of an equal volume of phenol equilibrated with 10mM Tris-HCl, pH 7.5, 5mM NaCl and 1mM EDTA (or phenol equilibrated with 20mM Tris-HCl, pH 7.5, 20mM NaCl, and 20mM EDTA).

GEL PURIFICATION OF DNA FRAGMENTS

After restriction enzyme or nuclease treatment, DNA fragments of varying sizes were separated by gel electrophoresis in either agarose or polyacrylamide slab gels at low voltage (approximately 2-20 volts/cm for agarose gels and 10 volts/cm for polyacrylamide gels). Gels were treated with ethidium bromide for visualization of DNA using ultraviolet light (Southern, 1979, Methods in Enzymology 68:152). Prior to such electrophoresis, some (but not all) enzyme reactions were terminated.

In order to recover particular DNA fragments from gels, the appropriate bands were sliced out of the gel and the DNA was eluted either by diffusion, at 37°C with agitation, into a buffer containing 0.5M NaCl, 20mM Tris-HCl (pH 7.5), and 1mM EDTA, or by electroelution into dialysis tubing. In some cases particular DNA fragments were recovered from low melting point (LMP) agarose gels. To this end, the appropriate bands were sliced out of the gel, the slices were melted at 65°-68°C and then diluted with an appropriate buffer (e.g., 10mM Tris-HCl, pH 8, 1mM EDTA). After recovery from gels, if necessary the DNA (gel purified) was concentrated and/or purified by ethanol precipitation and DEAE-cellulose chromatography (Smith, 1980, Methods in Enzymology 65:371).

.1.6. DNA LIGATION

All ligations were accomplished using T4 DNA ligase (New England Biolabs, Inc., Beverly, Ma.). One unit of T4 DNA ligase is defined as the amount required to yield 50% ligation of HindIII fragments of bacteriophage lambda DNA in 30 minutes at 16°C in 20 µℓ of ligase buffer and a 5'-DNA terminus concentration of 300 µg/mℓ.

Unless otherwise indicated, DNA ligations were carried out in ligase buffer consisting of: 20mM Tris-HCl (pH 7.8), 10mM $MgCl_2$, 60 mM ß-ME, 1.0mM ATP and a DNA concentration ranging from 15-20 µg/mℓ. Ligation reactions were incubated 4 to 24 hours at room temperature or 15°C using approximately 300 units of T4 DNA ligase per 10 µℓ reaction volume.

## PROPAGATION AND PURIFICATION OF PPV

Pregnant sows, approximately 35-40 days of gestation, were obtained from a local swine producer. At 40-50 days of gestation, laparotomies were performed and each fetus was identified. The amniotic sac of alternating fetuses was injected with PPV in order to propagate the virus yet prevent complete abortion. Each injection consisted of 0.2 mℓ of PPV, NADL-8 strain (Mengeling, 1972, Am. J. Vet. Res. 33:2239) containing $10^{5.5}$ $TCID_{50}$ (the Tissue Culture Infective Dose which produced a cytopathogenic effect in 50% of the cells in a confluent monolayer) or 1024 HA/50 µℓ (hemagglutination units/50 µℓ); the virus was originally obtained from NADC, Ames, Iowa). The uterine horns were returned to the abdominal cavity and the abdomen was closed. Ten to 15 days later, the sows were taken to slaughter and the entire uterus was collected and transported back to the laboratory. Internal tissues from each fetus (lung, kidneys, liver, heart, spleen and intestines) were collected and minced in 50mM Tris-HCl, 25mM EDTA, pH 8.7 (TE buffer). Following overnight freezing, minced tissue fluids from each individual fetus were tested by hemagglutination of guinea pig red blood cells for presence of virus (see below). Tissues from fetuses that were hemagglutination positive were pooled, diluted to a 20% (wt/vol) suspension in additional TE buffer, and homogenized in a Waring blender. The suspension was frozen and thawed twice and sonicated at 4°C for three, one minute intervals, prior to virus purification.

PPV was purified by a modification of a procedure used for the purification of minute virus of mice (Tattersall et al., 1976, J. Virol. 20:273) as described elsewhere (Molitor et al., 1983, J. Virol. 45:842-854). All manipulations were carried out at 4°C. Sonicated homogenates of HA positive fetal viscera were cleared of cellular debris by centrifugation at 12,000 x g for 30 minutes. To the resulting supernatant, $CaCl_2$ was added to a final concentration of 25mM; the virus was allowed to precipitate for 30 minutes. The precipitate was collected by centrifugation at 12,000 x g for 20 minutes and was resuspended by gentle sonication in TE buffer. Any insoluble material was removed by brief centrifugation, and the cleared supernatant was adjusted to a final CsCl density of 1.32 g/mℓ in TE buffer. This solution was layered on top of an equal volume of TE buffer containing CsCl having a density of 1.40 g/mℓ. Centrifugation was performed at 120,000 x g for 18 hours at 4°C (Beckman SW41 rotor). Gradients were fractionated (approximately 24-30 fractions), the refractive index of each fraction was determined, and the presence of viral antigen was assayed by hemagglutination. The HA assay involving the PPV-specific hemagglutination of guinea pig red blood cells has been previously described (Joo et al., 1976, Arch. Virol. 51:123; Mengeling, 1972, Am. J. Vet. Res. 33:2239). Fractions having a density of 1.39 g/mℓ and containing HA activity were pooled and dialyzed against TE buffer. The resultant material was assessed for purity by electron microscopy and SDS-polyacrylamide gel electrophoresis. Such preparations routinely contained homogenous full 20 nm parvovirus particles, with no apparent contaminating materials and was composed of only three protein species with molecular weights of 83,000, 64,000, and 61,000 daltons.

## EXTRACTION OF PPV VIRION GENOMIC DNA

Purified virus (500 µg) in 400 µℓ of 10mM Tris-HCl, pH 7.2, 1mM EDTA was incubated with 10 µg of RNase A at 37°C for 20 minutes. The solution was then adjusted to a final concentration of 1% SDS, 150mM NaCl, and extracted with phenol two times. Following two ether extractions, the aqueous phase was precipitated with ethanol. Analysis of the resultant DNA preparation by neutral agarose gel electrophoresis revealed a single, sharp band of DNA migrating at a molecular weight corresponding to 2.6 X $10^6$ daltons relative to double stranded DNA markers. Given that the parvovirus genomes are single-stranded and have hairpin termini (Ward and Tattersall, eds., 1978, Replication of Mammalian Parvoviruses, Cold Spring Harbor Press, N.Y.) and that the molecular weight markers are double-stranded DNA, the molecular weight as determined by gel electrophoresis does not necessarily represent the actual size of the viral genome.

## MOLECULAR CLONING OF PPV GENOMIC DNA

The cloning strategy for the PPV DNA was devised to utilize the unique hairpin structure of the termini of the genomic DNA. Briefly, the genomic DNA was converted to a double-stranded molecule using DNA polymerase; the

3'-hairpin structure served as a primer for this reaction. After filling in the entire length of the genome, the molecule was treated with DNA ligase. This resulted in double-stranded DNA molecule with single-stranded hairpin loops at each end. The single-stranded terminal loops were digested with S1 nuclease and the resultant double-stranded DNA molecule was tailed with dC using TdT. The dC-tailed double-stranded PPV DNA molecule was then annealed to pBR322 which had been previously cleaved at its PstI site and dG-tailed. The resultant recombinant molecules were then used to transform E. coli hosts. Details of the procedure are explained below and outlined in FIG. 1.

The single-stranded PPV genomic DNA isolated in section 6.3 was converted to double-stranded DNA by using the 3' base-paired hairpin structure as a primer for the synthesis of the second strand of DNA catalyzed by DNA polymerase. To this end 0.4 µg of PPV viral DNA was incubated at 14°C for 3-8 hours in 40 µℓ of the following reaction mixture: 75mM HEPES (N-2-hydroxyethylpiperazine-N-2-ethane sulfonic acid), pH 6.9, 100mM KCl, 10mM $MgCl_2$, 400µM of each deoxynucleoside triphosphate (i.e., dATP, dTTP, dCTP, dGTP), and 50-75 units/mℓ E. coli DNA polymerase I (or the Klenow fragment of DNA polymerase I). Neutral agarose gel electrophoresis of the resultant product showed a single, sharp band at an apparent double-stranded molecular weight of 3.3 X $10^6$ daltons, or approximately 5100 base pairs of DNA. After phenol extraction and several ethanol precipitations, this material was dissolved in 40 µℓ of a solution consisting of: 66mM Tris-HCl (pH 7.5), 6mM $MgCl_2$, 10mM DTT (dithiothreitol), and 0.5mM ATP. One unit of T4 DNA ligase was added and the mixture was incubated at 4°C for 6-8 hours. In order to cleave the single-stranded DNA hairpin termini, one half of this ligation mixture was diluted 10-fold into S1 buffer (300mM NaCl, 50mM sodium acetate, pH 4.5, 1mM $ZnCl_2$) and incubated at 22°C for 15 minutes with 0.5 units of S1 nuclease. This mixture was then phenol extracted and ethanol precipitated several times. The S1 nuclease-treated double-stranded PPV DNA (80 ng) was dC tailed by incubating at 37° for 10 minutes in a 20 μℓ reaction mixture containing 100mM sodium-cacodylate (pH 7.2), 2mM $CoCl_2$, 1mM EDTA, 40μM dCTP, 500 units/mℓ TdT (P-L Biochemicals, Milwaukee, Wis.). The reaction mixture was then incubated at 65°C for 10 minutes to terminate the tailing reaction. This entire reaction mixture was then mixed with 100 ng of linearized dG-tailed pBR322 in a final volume of 150 μℓ of buffer containing 150mM NaCl, 10mM Tris-HCl, pH 7.2, 1mM EDTA in order to allow annealing of the single-stranded complementary ends. (The pBR322 vector had been previously cleaved with PstI and dG-tailed using TdT according to the procedure described above except that 80μM dGTP was substituted for dCTP and the incubation was for 15 minutes instead of 10 minutes). The annealing reaction mixture was incubated at 65°C for 5 minutes, then at 42°C for three hours. Aliquots of this annealed mixture were then used to transform E. coli K12 strain HB101 [$F^-$, hsdS20 ($r_B^-$, $m_B^-$), recA13, ara-14, proA2, lacY1, galK2, rpsL20 ($Sm^r$), xyl-5, mtl-1, supE44, $lambda^-$]. Bacteria were plated out on nutrient agar dishes containing 20 μg/mℓ tetracycline. Tetracycline-resistant bacterial colonies were then tested for their sensitivity to ampicillin (100 μg/mℓ). Ampicillin-sensitive, tetracycline-resistant bacterial clones were then used for subsequent analyses.

## RESTRICTION ANALYSES OF SELECT RECOMBINANT PLASMIDS

Ampicillin-sensitive, tetracycline-resistant bacterial clones were cultured in liquid nutrient broth containing 20 μg/mℓ tetracycline. Plasmid DNA was isolated from several clones and initially screened for DNA insert size by *Pst*I restriction followed by agarose gel electrophoresis (restriction mapping). These results demonstrated that all ampicillin-sensitive, tetracycline-resistant clones contained additional, non-pBR322 DNA, however, the size of the inserted DNA in the recombinant plasmids of the various clones ranged from 500 b.p. to 3200 b.p. One recombinant plasmid, pPPV-6, containing an insert DNA of approximately 3200 b.p., was chosen for further analyses. The pPPV-6 plasmid DNA was shown to contain PPV-specific DNA by filter hybridization to purified virion genomic DNA (see section 6.4.2.)

The pPPV-6 plasmid was mapped (see FIG. 2A) using the following restriction enzymes: *Eco*RI, *Hin*dIII, *Bgl*II, *Pvu*II, and *Sac*I (for mapping techniques see Boseley *et al*., 1980, Methods in Enzymology 65: 478). The PPV DNA sequences found in pPPV-6 were found to contain a unique *Hin*dIII, *Eco*RI, *Pvu*II, and *Sac*I site. There are two *Bgl*II sites in the PPV sequence which map 566 b.p. apart. Sequence data (see section 6.4.3. and FIG. 3A) places a *Sac*I site and a *Hae*III site in the 566 b.p. *Bgl*II fragment. Sequence data also places a *Nco*I and a *Bst*EII site between the *Hin*dIII and *Eco*RI site in the PPV sequence of pPPV-6.

RELATION OF pPPV-6 TO VIRAL GENOME

To determine the segment of PPV DNA sequences that were cloned thus generating pPPV-6, several restriction endonuclease sites of the viral genome were mapped. An analysis of the restriction maps of the viral genome and pPPV-6 revealed that pPPV-6 contains 3200 b.p. of the 5100 b.p. viral genome and that the PPV DNA of pPPV-6 was derived from one end of the viral genome (see FIG. 2A and 2B).

In order to map the viral genome, virion single-stranded genomic DNA was isolated and filled-in with DNA polymerase as previously described using radioactive nucleotides. The resultant double-stranded viral DNA was then subjected to restriction enzyme digestions followed by agarose gel electrophoresis and autoradiography. Sizes of fragments were determined by comparison of the relative mobilities of viral fragments to those of molecular weight standards such as radiolabeled bacteriophage lambda restriction fragments. The restriction map of the viral genome was then compared to the restriction map of pPPV-6 (see FIG. 2B). When the filled in viral genome was restricted with EcoRI, two fragments were generated. These fragments migrated through agarose at the same rate as predicted for linear double-stranded DNA fragments of 3650 and 1450 b.p. Note that when the PPV insert in pPPV-6 is restricted with EcoRI endonuclease a fragment 1450 b.p. long is also generated. When the filled-in viral genome was restricted with both EcoRI and BglII only the smaller, 1450 b.p., EcoRI fragment was cleaved by BglII generating three fragments of about 70, 560, and 750 b.p. in length. As the BglII restriction of the 1450 b.p. EcoRI/PstI pPPV-6 fragment generated fragments that were the same size as those generated by the BglII restriction of the 1,450 b.p. EcoRI viral genomic fragment, the recombinant plasmid, pPPV-6, includes most of the PPV sequences of the 1,450 b.p. EcoRI genomic fragment, and extends to one end (or almost to one end) of the viral genome. The remaining PPV sequences present in pPPV-6, extend in the opposite direction from the EcoRI site for approximately 1750 b.p. Since the large EcoRI fragment generated from restriction of the viral genome was 3650 b.p., pPPV-6 lacks approximately 1900 b.p. of genomic sequences at this end of the viral DNA.

Restriction mapping analysis revealed that pPPV-6 contained approximately 3200 b.p. of PPV DNA derived from one end of the 5100 b.p. linear (negative strand) viral genome; however, this analysis did not demonstrate which terminus (3' or 5') of the viral genome was included in pPPV-6. Since the viral structural proteins are presumably encoded by the 5'-end of the negative stranded viral genome, it was important to determine that pPPV-6 contained sequences from the 5'-end of the viral genome, i.e., the coding sequence for PPV structural proteins. Accordingly, pPPV-6 was found to contain the 5'-end of the viral genome using the following strategy: complementary strands of a portion of the cloned PPV DNA were separated by subcloning into the single-stranded DNA cloning vector, M13 (described below). The polarity of each subcloned PPV DNA strand was determined by hybridization with single-stranded viral genomic DNA; i.e., the strand that hybridized to the negative stand genome was the complementary or positive strand, and the strand that did not hybridize was the same polarity or negative strand. Knowledge of the location and relative order of the restriction sites on a particular PPV DNA strand, for instance, the negative strand, allowed orientation of these sites within the viral genomic strand (also the negative strand) with respect to the 5' and 3' ends of the viral genome. The procedure is outlined in FIG. 4 and described more fully below.

M13 is a single-stranded filamentous coliphage; the viral DNA is circular, positive in polarity, and 6407 bases in length. The intracellular replicative form (RF) of the phage is double-stranded and has been modified to generate M13mp8 and M13mp9. The RF of M13mp8 and M13mp9 each contains a 789 fragment which encodes the amino terminus of lacZ from the E. coli lac operon. The amino terminal sequence of lacZ in this fragment was modified by the insertion of a 33 b.p. synthetic DNA fragment encoding a unique restriction enzyme recognition sites. The order of the cloning sites in these two vectors are as follows: EcoRI, SmaI (XmaI), BamHI, SalI (AccI, HincII), PstI, and HindIII; they occur only once within the M13 vectors. Double-stranded DNA fragments may be ligated into the double-stranded DNA form of M13 (RF, or replicative form) using any of these cloning sites. Loss of ß-galactosidase activity in E. coli JM103 [ (lac, pro) thi, strA, supE, endA, sbcB15, hsdR4, F'traD36, proAB, $lacI^{q}Z$ M15] transformants carrying M13 is an indication that the foreign DNA was actually inserted into M13, i.e., cloning was successful. The cloning sites in M13mp8 are aligned in the opposite orientation from those in M13mp9, i.e., in M13mp8 EcoRI is 5' to the HindIII site, whereas in M13mp9 EcoRI is 3' to the HindIII site. Thus, identical DNA fragments cloned into M13mp8 and M13mp9 using a combination of any two of these cloning sites will result in opposite orientations of the cloned DNA (i.e., the inserted or foreign DNA) in each vector (Messing, J., 1981, Third Cleveland Symposium on Macromolecules: Recombinant DNA, Ed. A. Walton, Elsevier, Amsterdam p.

143). If the chimeric RF molecules of M13mp8 and M13mp9 contain an identical DNA insert the resultant M13mp8 virions (single-stranded M13 phages) will contain one strand of the inserted DNA sequence while M13mp9 will contain the other. Therefore, the complementary strands of the inserted DNA fragment may be separated by isolating the single-stranded virion M13 DNA from E. coli clones containing the recombinant M13mp8 and M13mp9.

Several primers may be used with the M13 vector in determining the DNA sequence of the cloned DNA according to the dideoxynucleotide method (Sanger et al., 1977, Proc. Natl. Acad. Sci. U.S.A. 74:5463) or in generating a radioactive hybridization probe for a region of DNA or RNA that is complementary to a strand of the DNA fragment which has been cloned into the M13 vector. Note that the hybridization primer does not prime through the cloned fragments. If you limit the concentration of one of the four nucleotides in a DNA polymerase reaction mixture containing the M13 vector, the hybridization primer, and radioactive nucleotides, only a portion of the vector DNA is double-stranded and that portion is radioactively labeled by the incorporation of radionucleotides as a result of the primed synthesis of a complementary M13 vector DNA strand. Thus, while a portion of the M13 vector is converted to a radiolabeled double-stranded DNA, the cloned DNA fragment remains single-stranded and is free to anneal to its complementary strand; thus the recombinant plasmid may be used as a probe (Hu and Messing, 1982, Gene, 17:271). The use of M13mp8 and M13mp9 to orient the pPPV-6 clone to the viral genome is explained below. Details for each step in the procedure follow.

A portion of the pPPV-6 insert DNA (the HindIII/EcoRI fragment, 268 b.p.) was subcloned into the RF DNA of M13mp9 and M13mp8 (see FIG. 4A). As explained above, this allowed for the cloning of this fragment in M13 in opposite orientations. The chimeric M13 RF molecules were used to infect E. coli, and the resultant M13 single-stranded phages were harvested. The M13 phages, containing single-stranded M13 DNA carrying opposite (complementary) strands of the pPPV-6 HindIII/EcoRI fragment were designated M13mp9/6-6 and M13mp8/6-3 (see Fig 4B). A portion of each recombinant single-stranded phage DNA was labeled with radioactive nucleotides; the labeled recombinant DNA molecules were then used as hybridization probes to purified single-stranded genomic PPV DNA (isolated as described above) which was immobilized on nitrocellulose. It was found that only the radioactive PPV-specific probe M13mp9/6-6, hybridized to PPV viral DNA, indicating that M13mp9/6-6 contained the PPV positive strand and that the orientation of the HindIII/EcoRI PPV DNA fragment in the M13mp8/6-3 vector containing the opposite strand, was the same as that in the PPV genome. As described above, the EcoRI site is 5' to the HindIII site in M13mp8. Thus the EcoRI site is located the 5'-end of the HindIII site in the viral genome which is negative in polarity. The relative orientation of other restriction sites of the PPV genome was deduced relative to the EcoRI and HindIII sites. The orientation of the restriction sites within the PPV genome and the orientation of the cloned pPPV-6 DNA to the viral genome and the direction of transcription (arrow) of the PPV DNA insert of pPPV-6 is depicted in FIG. 4C.

Therefore, the clone pPPV-6 maps to the 5'-end of the genome (see FIG. 4C). Since the genetic sequences that encode the major structural protein or proteins of nondefective or autonomously replicating parvoviruses are presumably located in the 5' half of the genome, pPPV-6 should contain the gene (or a major portion thereof) that encodes the structural polypeptides of PPV. Details of the M13 procedure are explained below.

The procedures used for subcloning the pPPV-6 fragment into M13 and for hybridizations were a modification of those described by Messing (Hu and Messing, 1982, Gene 17:27): 1 µg each of pPPV-6, M13mp8 (RF), and M13mp9 (RF) were restricted with HindIII and EcoRI in separate reaction mixtures. The reaction products were extracted with an equal volume of phenol equilibrated with 10mM Tris-HCl, pH 7.5, 5mM NaCl, and 1mM EDTA (or phenol equilibrated with 20mM Tris-HCl, pH 7.5, 20mM NaCl, and 1mM EDTA). After centrifugation in a microcentrifuge (12,000 x g) for 1-5 minutes the supernatants were separated and extracted with equilibrated phenol:chloroform:isoamyl alcohol (25:24:1). Sodium acetate (3M) was added to the mixtures to achieve a final concentration of 0.3M. The DNA was then precipitated from the mixtures by the addition of 3 volumes of ethanol and incubation at -70°C for 10 minutes. The precipitates were pelleted by centrifugation in a microcentrifuge (12,000 x g) for 5-10 minutes and the pellets were washed with 70% ethanol and dried under vacuum. Each of the DNA pellets (consisting of the cleaved plasmids) were resuspended in buffer containing 20mM Tris-HCl, pH 7.5, 20mM NaCl, and 1mM EDTA resulting in a final DNA concentration of 5 µg/mℓ.

The cleaved pPPV-6 DNA was ligated into each of the cleaved vectors, M13mp8 and M13mp9 as follows: 4 µℓ of restricted pPPV-6 was added to 4 µℓ of restricted M13

DNA (ligations of the pPPV-6 fragments to M13mp9 and M13mp8 were accomplished in separate vessels), to which was added 2 µℓ 10XC (0.5M Tris-HCl, pH 7.5, 100mM $MgCl_2$, 10mM DTT), 1 µℓ 1mM ATP, 7 µℓ distilled water ($dH_2O$) and 300 units of T4 DNA ligase. This ligation reaction mixture was incubated at 12.5°C overnight.

The ligated DNA was then mixed with competent E. coli host cells and the cells were plated on media with IPTG and x-gal (5-bromo-4-chloro-3-indolyl-ß-F-galactoside). Thus, E. coli JM103 was transformed with the ligation mix according to the method of Messing et al., 1981, Nucl. Acids Res. 9:309 as follows: 2.0 mℓ of JM103 at log growth phase ($OD_{660}$=0.3-0.4) in YT medium (8 g/ℓ Bacto tryptone, 5 g/ℓ Bacto yeast extract, 5g/ℓ NaCl) were centrifuged at 7000 x g for 5 minutes in order to collect the cells. The cell pellet was resuspended in 1.0 mℓ 50mM $CaCl_2$ and maintained on ice for 20 minutes. The cells were recentrifuged and resuspended in 0.2 mℓ 50mM $CaCl_2$, and transformed with 10 µℓ of the ligation mix. The mixture was maintained on ice for 40 minutes. The mixture was then heat shocked at 42°C for 2 minutes and the following was added directly and mixed: 10 µℓ 10mM IPTG, 50 µℓ 2% x-gal in dimethylformamide, 0.2 mℓ fresh JM103 cells in log growth phase, 3 mℓ soft agar (6 gm Bacto-agar in 1 liter YT medium). This mixture was plated directly onto YT agar plates (15 gm bacto-agar in 1 liter YT medium). After solidification of the agar, the plates were incubated overnight at 37°C. Plaques, which in this instance denote areas of slow growth due to phage infection of cells in a bacterial lawn in a soft agar overlay, were usually detected after about four hours. The color reaction occured a few hours later.

Phage which infect JM103 giving rise to colorless plaques (indicating that M13 contained a DNA insert) were analyzed for DNA inserts by agarose gel electrophoresis as follows: 2 mℓ aliquots of JM103 culture ($OD_{660}$=0.01-0.05) were inoculated with phage and infected cells from white plaques (by transfer with a sterile toothpick); control cultures were inoculated with blue plaques. The cultures were vigorously shaken at 37°C for 6 hours. After decanting 1.5 mℓ and removing cells by centrifugation in a microcentrifuge (12,000 x g), 1 µℓ 2% SDS was added to a 20 µℓ aliquot of the supernatant which was then prepared for agarose gel electrophoresis (1% agarose) by the addition of 5-10 µℓ loading dye (50% glycerol, 0.025% bromophenol blue (BPB) 0.025% xylene cyanol, and 1mM EDTA). After electrophoresis at 100 volts for 5 hours, the gel was stained with ethidium bromide and the DNA bands were visualized by illumination with ultraviolet light. The mobility of single-stranded DNA decreases proportionally to the length of the inserted DNA. Those phages which contained the pPPV-6 HindIII/EcoRI fragment (approximately 270 b.p.) migrated more slowly than those with no insert.

Those M13 phages which were found to contain inserted DNA were analyzed for their ability to hybridize to the PPV genome. The chimeric M13 phages M13mp9/6-6, M13mp8/6-3 (which presumably contained PPV DNA) and a control, M13mp8/15 (M13mp8 containing a 4 b.p. insert) were labeled using the hybridization probe primer as previously explained (Hu and Messing, 1982, Gene 17:271).

The labeled M13 vectors were hybridized to the PPV genome which was immobilized on nitrocellulose. To this end 40ng purified PPV genome was spotted on 3 pieces of nitrocellulose. Unlabeled M13mp8/6-3, M13mp9/6-6, and M13mp8/15 were spotted on each nitrocellulose filter as controls. The drying, prehybridization, hybridization, and film exposure procedure of Hu and Messing (1979, Gene 17:271) was used to hybridize each of the labeled M13mp8/6-3, M13mp9/6-6, and M13mp8/15 to a nitrocellulose filter containing the PPV genomic DNA. As previously explained, only the labeled M13mp9/6-6 hybridized to the immobilized PPV genome. Thus the DNA insert in M13mp9/6-6 was complementary to the PPV genome. Since the phage M13mp9/6-6 also hybridized to M13mp8/6-3, the phage M13mp8/6-3 contains the viral genomic or negative strand. Thus the PPV insert in the plasmid pPPV-6 can be oriented to the PPV genome (FIG. 4C).

## NUCLEOTIDE SEQUENCING

Two methods were used to determine portions of the PPV DNA sequence in pPPV-6. The dideoxy chain termination method (Sanger et al., 1977, Proc. Natl. Acad. Sci. U.S.A. 74: 5463) was employed to determine the nucleotide sequence of portions of pPPV-6 subcloned into M13mp8 and M13mp9 (Messing, 1981, Third Cleveland Symposium on Macromolecules: Recombinant DNA, ed., A. Walton, Elsevier, Amsterdam, p. 143). The chemical cleavage method of sequencing DNA (Maxam and Gilbert, 1977, Proc. Natl. Acad. Sci. USA 74: 5463) was employed to determine the DNA sequence of portions of pPPV-6 either directly (i.e., using fragments derived from pPPV-6) or indirectly (i.e., using portions of pPPV-6 subcloned into expression vectors as described in Section 6.5. and its subsections). The sequences of both DNA strands of the PvuII/BglII 1687 b.p. fragment were determined and the sequence of the positive strand is given in FIG. 3 and FIG. 3A.

## CLONING AND EXPRESSION OF PPV DNA

A number of fragments derived from the PPV DNA insert of pPPV-6 were ligated into expression vectors (e.g., pJS413 and pHK413) to form recombinant plasmids (designated as the pPP plasmid series). Many of these recombinant plasmids, when used to transform appropriate host cells, were found to be capable of directing the production of a PPV/ß-galactosidase fusion proteins.

These expression plasmids, containing PPV DNA fragments, were used to transform an E. coli host strain in which the transcription of DNA from the lac operon is inhibited unless the promoter is specifically induced. Primary transformants were selected for drug resistance (an ampicillin resistance gene, $amp^r$, is carried on a pPP400's, 500's, 700's, 800's, 900's, and 1000's; a tetracycline resistance gene, $tet^r$, is carried on the "t" series of pPP plasmids e.g., pPP406t; and both $amp^r$ and $tet^r$ are carried on the pPP600's) and resistant colonies were screened for inducible ß-galactosidase activity. Induction of many transformants resulted in the production of fusion proteins (approximately 120,000 daltons or greater) that were immunoreactive to antibodies directed against specific structural proteins of PPV or whole virus.

## THE EXPRESSION VECTORS pJS413, pHK413, AND pHK414

The expression vectors pJS413(6483 b.p. or about 6.5 kilobases, kb) and pHK413 (6485 b.p. or about 6.5 kb), and pHK414 (6485 b.p. or about 6.5 kb) are pBR322 derivatives which contain the $amp^r$ (ß-lactamase) gene, a lac promoter (Plac), lac and cro ribosome binding sites ($SD^{lacZ}$ and $SD^{cro}$ are represented in all figures as

$SD^z$ and $SD^{cro}$, respectively), a chain initiation ATG derived from cro followed by 65 nucleotides of the cro gene, and a modified β-galactosidase gene (the i-z gene, hereinafter referred to as the z-gene). These expression vectors are described in detail in a U.S. Patent Application Serial No. 449,187 by J. Salstrom et al., filed December 13, 1982 which is incorporated herein by reference. Each plasmid has unique cloning sites which span the cro-z junction: the pJS413 cloning sites are BglII, SmaI, and BamHI; the pHK413 cloning sites are BglII, HindIII, SmaI, and BamHI; the pHK414 cloning site are BglII, HindIII, SmaI, BamHI. In each plasmid the z-gene is not in phase with the cro ATG, thus, the intact plasmids do not direct the production of β-galactosidase. However when a DNA fragment of the appropriate length is inserted into any of these cloning sites on the plasmids, the reading frame of the z-gene may be readjusted with respect to the cro ATG and, provided that the inserted DNA sequence contains no termination signals (e.g., TGA, TAA, or TAG) that are in phase with the cro ATG or z gene, a β-galactosidase fusion protein will be produced by host cell transformants. Accordingly, insertion of a 3n+1 b.p. DNA fragment between the cro ATG and z-gene of pJS413, or insertion of a 3n+2 b.p. DNA fragment between the cro ATG and z-gene of pHK413 or pHK414, results in a readjustment of the reading frames and production of a fusion protein in the host cell transformant, provided the inserted sequences do not contain termination signals in the same reading frame. Of course, the inserted DNA sequence and/or the cloning sites of pJS413, pHK413, and pHK414 may be altered in any fashion in order to readjust the reading frame across the cro-z junction.

INSERTION OF PPV DNA INTO pJS413 AND pHK413

Various portions of the PPV DNA sequences from pPPV-6 were inserted into the cloning expression vectors pJS413 and pHK413 (FIG. 5 and FIG. 6 depict an example using each vector). To aid in understanding the following subsections, each recombinant molecule listed and described below is represented in FIG. 7. Within the text that follows, DNA fragments are identified according to their length in base pairs (b.p.) and according to their termini generated by restriction enzymes. All DNA fragment sizes which are represented in terms of base pairs are determined from DNA sequence date (FIG. 3). If a DNA fragment has a single-stranded cohesive end, the b.p. number indicated for that fragment includes the nucleotides comprising the single-stranded cohesive end. The termini of each DNA fragment containing PPV sequences are indicated on either side of a slash in the order of the direction of transcription of the PPV DNA sequence. For example, a 907 b.p. HindIII/BglII DNA fragment signifies a DNA fragment which has a HindIII cohesive end at its amino-coding terminus and a BglII cohesive end at its carboxy-coding terminus and is 907 nucleotides in length.

pPP405 and pPP406

The construction of these plasmids is depicted in FIG. 5. A 730 b.p. DNA fragment containing PPV sequences (referred to as PPV DNA throughout Section 6.5.2.) was generated by digesting pPPV-6 with Acc I, filling in the cohesive ends using DNA polymerase Klenow fragment (resulting in blunt ends) and then digesting with BglII (which generates BglII cohesive ends). The resulting 730 b.p. blunt end/BglII PPV DNA fragment was isolated by gel

0117767

purification as described in Section 6.1.5. The expression vector, pJS413, was digested with SmaI which generates a SmaI blunt end) followed by BamHI which generates a BamHI cohesive end).

The 730 b.p. blunt end/BglII pPPV-6 fragment and the 6.5 kb SmaI/BamHI pJS413 were annealed in a 5:1 molar ratio and ligated using T4 DNA ligase resulting in the generation of the recombinant expression plasmid pPP405 (FIG. 5); [N.B., the BglII and BamHI single-stranded or staggered ends are complementary in base composition and, thus, are compatible cohesive ends that will anneal and ligate; in addition, the SmaI blunt end of the vector will ligate to the blunt end of the 730 b.p. PPV DNA fragment. However, both the BamHI and BglII sites and both the SmaI and AccI sites are destroyed after ligation (i.e., the sequences resulting from ligation are no longer recognized by these enzymes)].

The recombinant plasmid, pPP406, (or pPPV 1000t) was constructed from pPP405 in the following manner: pPP405 was digested with BglII (i.e., the BglII cloning site of the pJS413 vector was cleaved) resulting in BglII cohesive ends which were then filled in using DNA polymerase Klenow fragment to produce blunt ends. The cleaved, blunt-ended plasmid was then religated (by blunt-end ligation) using T4 DNA ligase. As a result of this process, the cleaved BglII site was destroyed.

pPP510

The construction of this plasmid is depicted in FIG. 6. A 1686 b.p. PPV DNA fragment was generated by a partial digestion of pPPV-6 with BglII followed by digestion with PvuII (generating a PvuII blunt end). The PvuII/BglII 1683 b.p. fragment was gel purified and the BglII cohesive end was filled in using DNA polymerase Klenow fragment (resulting in blunt ends at both termini of the DNA molecule). After digestion with HindIII (which generates HindIII cohesive ends) a 907 b.p. HindIII/blunt end PPV DNA fragment was gel purified. The expression vector, pHK413, was digested with SmaI (generating a SmaI blunt end) followed by HindIII (generating a HindIII cohesive end).

The 907 b.p. HindIII/blunt end pPPV-6 fragment and the 6.5 kb HindIII/SmaI pHK413 were annealed in a 5:1 molar ratio and ligated using T4 DNA ligase (FIG. 6). Thus, after ligation the HindIII site was restored but the SmaI site and the BglII site were destroyed.

pPP410

A 1686 b.p. DNA fragment containing a PPV sequence was generated by a partial digestion of pPPV-6 with BglII followed by digestion with PvuII. The 1686 b.p. PvuII/BglII DNA fragment which was gel purified, was then digested with AccI, and the cohesive ends were filled in using DNA polymerase Klenow fragment. The resulting 1296 b.p. blunt ended DNA fragment containing PPV sequences was gel purified.

The vector pHK413 was digested with SmaI (resulting in a blunt ended cleaved vector). The 1296 b.p. blunt ended pPPV-6 DNA fragment and the cleaved 6.5 kb blunt ended pHK413 vector were directly ligated (i.e., or blunt end ligated) in a 5:1 molar ratio using T4 DNA ligase. As a result of this process the AccI site and the SmaI site were destroyed.

pPP420 and pPP421

A 402 b.p. DNA fragment was generated by digesting pPP405 with HindIII, filling in the cohesive ends using DNA polymerase Klenow fragment (resulting in blunt ends), and then digesting the cleaved pPP405 with BglII (i.e., the BglII site of the pHK413 cloning vector was cleaved). The resulting 402 BglII/blunt end b.p. DNA fragment (gel purified) contained 393 b.p. of PPV DNA. The expression vector pHK413 was digested with SmaI (resulting in a blunt end) followed by BglII.

The 402 b.p. BglII/blunt end pPP405 DNA fragment and the 6.5 kb BglII/SmaI pHK413 were annealed in a 5:1 molar ratio and ligated using T4 DNA ligase. As a result of this process, the HindIII site and the SmaI site were destroyed.

The recombinant plasmid, pPP421, was constructed from pPP420 in the following manner: pPP420 was digested with BglII (i.e., the BglII site of the pHK413 cloning vector was cleaved). The resulting BglII cohesive ends were then filled in using DNA polymerase Klenow fragment to produce blunt ends. The cleaved, blunt ended plasmid was then religated using T4 DNA ligase. As a result of this process, the BglII site was destroyed.

pPP501

A 341 b.p. PPV DNA fragment was generated by digesting pPPV-6 with HindIII and BglII. The resulting 341 b.p. HindIII/BglII PPV DNA fragment was gel purified. The expression vector pHK413 was digested with HindIII followed by BamHI.

The 341 b.p. HindIII/BglII pPPV-6 DNA fragment and the 6.5 kb HindIII/BamHI pHK413 were annealed in a 5:1 molar ratio and ligated using T4 DNA ligase. In this case, the HindIII site is restored. As explained previously, the BglII and BamHI single-stranded or staggered ends are complementary in base composition and, therefore, are compatible cohesive ends which will anneal and ligate; however, both the BamHI and the BglII site are destroyed after ligation (i.e., the sequence resulting from ligation is no longer recognized by either enzyme).

pPP520

A 184 b.p. PPV DNA fragment was generated by digesting pPP501 with HinfI, filling in the cohesive ends using DNA polymerase Klenow fragment (resulting in blunt ends), and then digesting the cleaved pPP501 with HindIII. The resulting 184 b.p. HindIII/blunt end PPV DNA fragment was gel purified. The expression vector pHK413 was digested with SmaI (resulting in a blunt end) followed by HindIII.

The 184 b.p. HindIII/blunt end pPP501 fragment and the 6.5 kb HindIII/SmaI pHK413 were annealed in a 5:1 molar ratio and ligated using T4 DNA ligase. As a result of this process the HindIII site was restored, however, the HinfI site and the SmaI site were destroyed.

pPP540 and pPP541

A 480 b.p. PPV DNA fragment was generated by digesting pPPV-6 with HaeIII (which generates HaeIII blunt ends) and HindIII. The resulting 480 b.p. HindIII/HaeIII PPV DNA fragment was gel purified. The expression vector pHK413 was digested with SmaI followed by HindIII.

The 480 b.p. HindIII/HaeIII pPPV-6 fragment and the 6.5 kb HindIII/SmaI pHK413 were annealed in a 5:1 molar ratio and ligated using T4 DNA ligase to form the recombinant plasmid pPP540. As a result of this process the HindIII site was restored, however, the HaeIII site and the SmaI site were destroyed.

The recombinant plasmid, pPP541, was constructed from pPP540 in the following manner: pPP540 was digested with BamHI (i.e., the BamHI site of the pHK413 portion of pPP540 was cleaved) resulting in BamHI cohesive ends which were then filled in using DNA polymerase Klenow fragment to produce blunt ends. The cleaved, blunt ended plasmid was then religated using T4 DNA ligase. As a result of this process the cleaved BamHI site was destroyed.

pPP700

A 255 b.p. PPV DNA fragment was generated by digesting pPPV-6 with BstEII, filling in the cohesive ends using DNA polymerase Klenow fragment (resulting in blunt ends) and then digesting the cleaved pPPV-6 with BglII. The resulting 255 b.p. blunt end/BglII PPV DNA fragment was gel purified. The expression vector pHK413 was digested with SmaI (resulting in a blunt end) followed by BamHI.

The 255 b.p. blunt end/BglII pPPV-6 fragment and the 6.5 kb SmaI/BamHI pHK413 were annealed in a 5:1 molar ratio and ligated using T4 DNA ligase. Since the BamHI cohesive ends and the BglII cohesive ends are complementary, annealing and ligation occured; however both the BamHI site and the BglII site were destroyed. In addition, the BstEII site and the SmaI site were destroyed.

pPP800

A 570 b.p. PPV DNA fragment was generated by digesting pPP510 with BglII, filling in the cohesive ends using DNA polymerase Klenow fragment (resulting in blunt ends), and then digesting the cleaved pPP510 with BamHI (thus the BamHI site of pHK413 portion of pPP510 was cleaved). The resulting 570 b.p. blunt end/BamHI PPV DNA fragment was gel purified. The expression vector pHK413 was digested with BglII, and the cohesive ends were filled in using DNA polymerase Klenow fragment. This blunt ended vector was then digested with BamHI.

The 570 b.p. blunt end/BamHI pPP510 fragment and the 6.5 kb blunt end/BamHI pHK413 cleaved vector were annealed in a 5:1 molar ratio and ligated using T4 DNA ligase. As a result of this process the BamHI site was restored, however the two cleaved BglII sites were destroyed.

pPP900

A 1116 b.p. PPV DNA fragment was generated by digestion of pPPV-6 with BglII followed by digestion with PvuII and gel purification. A 102 b.p. PPV DNA fragment was generated by digesting the 1116 b.p. PvuII/BglII PPV DNA fragment with RsaI (which generates RsaI blunt ends). The resulting 102 b.p. RsaI/BglII PPV DNA fragment was gel purified. The expression vector pHK413 was digested with SmaI (resulting in a SmaI blunt end) and BamHI.

The 102 b.p. RsaI/BglII pPPV-6 fragment and the 6.5 kb SmaI/BamHI pHK413 were annealed in a 5:1 molar ratio and ligated using T4 DNA ligase. Since the BamHI cohesive ends and the BglII cohesive ends are complementary, annealing and ligation occurred; however both the BamHI site and the BglII site were destroyed. In addition, the RsaI site and the SmaI site were destroyed.

p1000

The 1116 b.p. PvuII/BglII fragment of pPPV-6 was prepared and digested with RsaI (which generates blunt ends) as described above for pPP900. This RsaI digestion resulted in five PPV DNA fragments: 517 b.p. PvuII/RsaI, 36 b.p. RsaI/RsaI, 156 b.p. RsaI/RsaI, 306 b.p RsaI/RsaI, and 102 b.p. RsaI/BglII. These 5 fragments were added to a ligation reaction mixture containing the expression vector pHK413 which had been previously cleaved with SmaI and BamHI. One of the recombinant plasmids resulting from this ligation reaction was designated pPP1000, which contains two of the five PPV-DNA fragments: the 156 b.p. RsaI/RsaI PPV DNA fragment and the 102 b.p. RsaI/BglII PPV DNA fragment which ligated to form a 258 b.p. RsaI/BglII PPV DNA insert in the 6.5 kb SmaI/BamHI pHK413. See FIG. 7 which depicts the arrangement of these fragments within the expression vector.

Since the BamHI cohesive ends and the BglII cohesive ends are complementary, annealing and ligation occurred; however, both the BamHI site and the BglII site were destroyed, In addition, the terminal RsaI site of the pPPV-6 fragment and the SmaI site of pHK413 were destroyed; however the internal RsaI site of the 258 b.p. PPV DNA insert was restored.

pPP1100

The recombinant plasmid, pPP1000, was digested with BglII (i.e., the BglII site of the pHK413 cloning vector was cleaved) and *Rsa*I. A 168 b.p. *Bgl*II/*Rsa*I fragment was isolated by gel purification. This 168 b.p. *Bgl*II/*Rsa*I fragment of pPP1000 contained the 156 b.p. pPPV-6 *Rsa*I/*Rsa*I fragment.

The expression vector pHK413 was cleaved with *Hin*dIII, the resulting cohesive ends were filled in using DNA polymerase Klenow fragment (resulting in blunt ends) and the cleaved plasmid was then digested with *Bgl*II.

The 168 b.p. *Bgl*II/*Rsa*I fragment of pPP1000 and the *Bgl*II/blunt end pHK413 were annealed and ligated in a 5:1 molar ratio using T4 DNA ligase. As a result of this procedure, the *Bgl*II site was restored but the *Hin*dIII site of pHK413 and the *Rsa*I site of the pPP1000 fragment were destroyed.

pPP405t, pPP406t, pPP1000t

The construction of pPP405t and pPP406t from pPP405 is depicted in FIG. 8A. The plasmid pPP1000t (or pPPV1000t) was constructed similarly using pPP1000 as the starting plasmid (FIG. 8B).

The recombinant plasmids pPP405 and pPP1000 were each digested with *Pst*I and then treated with *BAL* 31 in order to cleave the plasmids and remove a total of approximately 400 b.p. in either direction from the *Pst*I site; thus the *amp*$^r$ gene was removed and the cleaved plasmids were blunt ended.

The plasmid pBR322 was digested with *Eco*RI followed by *Ava*I. The resulting 1430 b.p. fragment containing the *tet*$^r$ gene was gel purified. The *Eco*RI

0117767

and *Ava*I single-stranded ends were filled in using DNA polymerase Klenow fragment.

The 1430 b.p. pBR322 DNA fragment containing the *tet*$^r$ gene was ligated (in a 5:1 molar ratio) to each of the cleaved blunt-ended pPP405 and pPP1000 using T4 DNA ligase. The resulting plasmids pPP405t and pPP1000t (respectively) contained the *tet*$^r$ gene in the former location of the *amp*$^r$ gene.

The recombinant plasmid pPP406t was constructed from pPP405t by digesting pPP405t with *Bgl*II, filling in the *Bgl*II cohesive ends using DNA polymerase Klenow fragment and religating the blunt-ended plasmid using T4 DNA ligase. As a result of this process, the cleaved *Bgl*II site was destroyed.

pPP605

The construction of pPP605, shown in FIG. 9, was accomplished as follows: pPP405 was digested with *Pst*I followed by *Hin*dIII. The resulting 1353 b.p. *Pst*I/*Hin*dIII fragment which contained the *lac* promoter elements, SD$^2$ SD$^{cro}$, the *cro* ATG and a portion of the PPV genetic sequence was gel purified (FIG. 9A).

The recombinant plasmid pPPV-6 was digested with *Hin*dIII and the resulting 2550 *Hin*dIII/*Hin*dIII PPV DNA fragment was gel purified (FIG. 9A).

Finally, pBR322 was digested with *Pst*I followed by *Hin*dIII and the 3.6 kb *Hin*dIII/*Pst*I pBR322 DNA containing the *tet*$^r$ gene was gel purified (FIG. 9A).

All three fragments were ligated in a 1:1:1 molar ratio and resulted in recombinant plasmid pPP605 (FIG. 9B).

TRANSFORMATION OF E. COLI AND
EXPRESSION OF FUSION PROTEINS

All pPP recombinant plasmids described supra were used to transform *E. coli* strain NF1829.

The *E. coli* strain NF1829 is a K-12 MC1000 derivative carrying an F'-*lac* episome with the *lac* $i^q$ mutation for *lac* repressor overproduction. Thus, in strain NF1829, the *lac* promoter must be induced in order to obtain expression of a gene inserted into the pJS413 or pHK413 plasmid. The *lac*Z-gene encoding ß-galactosidase present on the F'-*lac* episome is inactivated by a *Tn* 5 (transposon) insertion.

Host cell clones were analyzed to determine whether the predicted PPV/ß-galactosidase fusion polypeptides were expressed. The drug resistant colonies (i.e., pPP transformants express ampicillin resistance, the "t" series of pPP transformants express tetracycline resistance, and the pPP600's may be screened for either) were examined for fusion protein production by analyzing indicator agar plates for colonies producing ß-galactosidase activity (Miller, 1972, Experiments in Molecular Genetics, Cold Spring Harbor Press, New York, N.Y.). The positive colonies (transformants containing pPP405, pPP406, pPP405t, pPP406t, pPP410, pPP420, pPP421, pPP501, pPP510, pPP520, pPP541, pPP700, pPP800, pPP900, pPP1000, pPP1000t, and pPP1100) were then tested for the presence of the PPV/ß-galactosidase fusion protein. Two approaches were routinely used (these are described in the following subsections): direct analysis of total *E. coli*

proteins by SDS-polyacrylamide gel electrophoresis (SDS-PAGE) followed by Coomassie brillant blue staining, and specific immunoprecipitation of $^{35}$S-methionine radiolabeled E. coli polypeptides using antisera directed against authentic PPV viral structural proteins.

In both approaches the polypeptide production of transformants grown under lactose operon repressed or derepressed conditions was determined. Since the lac promoter was transcriptionally inactive in NF1829 (due to the overproduction of the lac repressor) the fusion protein could only be detected upon induction, i.e. derepressing, of the promoter with either 1mM IPTG or 1% lactose. All transformants so analyzed produced multiple fusion proteins (except for p1000 which produced only 1 major fusion protein). A number of the expression plasmids, i.e., pPP405, pPP405t, pPP410, and pPP420, which contain PPV DNA inserted in phase (in the same reading frame) with the z-gene but not with the cro ATG of the expression plasmid direct the production of fusion proteins. The molecular weight of these fusion proteins is lower than that predicted for a fusion protein resulting from the expression of the PPV DNA sequence insert ligated in phase with both the cro and z gene sequences. Such protein synthesis is perhaps due to a "restart" phenomenon which occurs during translation. That is, host cell translation of the transcribed mRNA probably initiates at the cro AUG codon which is out of phase with ß-galactosidase transcript, and then later reinitiates at internal codons (e.g., AUG, GUG) of the PPV transcribed sequence which are in phase with the ß-galactosidase sequence. Alternatively, it may be that the sequences transcribed in pPP405, pPP405t, pPP410, and pPP420, and which are also present in the PPV DNA sequences of many of the other recombinant expression plasmids, contain signals for the initiation of translation. These signals may be utilized by the host cell in addition to those supplied by the expression vector. Finally, the lower molecular weight of the polypeptides produced may be due to specific proteolytic cleavage.

The recombinant plasmid pPP540 (described in Section 6.5.2.) did not direct the expression of a fusion protein. In pPP540 the inserted PPV DNA sequence is in phase with the cro ATG but out of phase with ß-galactosidase. When pPP540 was converted to pPP541, the PPV DNA sequence was placed in phase with the ß-galactosidase sequence; pPP541 directs the production of a fusion protein. Proteins produced by a transformant containing pPP605 have not yet been analyzed.

ANALYSIS OF TOTAL E. COLI PROTEINS

All transformants were grown in 1 mℓ overnight cultures (stationary phase) at 30°C in L-broth containing, as appropriate, either 100 µg/mℓ ampicillin or 20 µg/mℓ tetracycline. To assay these cultures for expression of the fusion proteins, cultures were grown in parallel cultures in the presence of an inducer. Induction was accomplished by the inclusion of 1mM IPTG or 1% lactose in the growth medium. In order to examine the protein content of these cells, the cultures were centrifuged and the cell pellets were resuspended in an appropriate volume of lysis buffer (SDS gel sample buffer) consisting of 70mM Tris-HCl(pH6.8), 3% SDS, 11% glycerol, 5% ß-ME, and 0.01% bromophenol blue. After boiling for 2-5 minutes the samples were centrifuged in a microcentrifuge at 16,000 x g for 5 minutes. The resultant supernatants were then analyzed by SDS-polyacrylamide gel electrophoresis in 7.5% polyacrylamide gels (Laemmli, 1970, Nature (London) 227:680).

Results of SDS-PAGE protein analyses (data not shown) of a number of clones transformed with the pPP plasmids (pPP700, pPP520, pPP501, pPP406, pPP405, pPP421, pPP420, pPP1000, pPP900 and pPP110) demonstrated that all induced *E. coli* transformed hosts produced fusion proteins; most clones produced several fusion proteins of different sizes.

ANALYSIS OF IMMUNOREACTIVE E. COLI PROTEINS

The positive transformants were then analyzed by immunoprecipitation of $^{35}S$-methionine radiolabeled *E. coli* polypeptides using antibodies directed against the PPV A protein, B protein, C protein, and/or whole PPV. The results of the experiments described below demonstrated that the pPP transformants produced fusion proteins that were immunoreactive with antisera directed against PPV.

The antisera used for these analyses were a gift from T. Molitor and were prepared as described (Molitor *et al.*, 1983, J. Virol., 45:842-854). Briefly, highly purified virus was isolated by cesium chloride equilibrium density centrifugation. The purified virus was disrupted by boiling in SDS-PAGE sample buffer and then subjected to electrophoresis in SDS-10% polyacrylamide gels. The A, B, and C protein bands separated according to size, and were localized and excised from the gel. Each protein was subjected to electrophoresis in a second SDS-10% polyacrylamide gel. After localization and excision from the second polyacrylamide gel each polypeptide was eluted from the gel slices by placing the gel slices in an ammonium carbonate/SDS solution which was then lyophilized. The lyophilized material was dissolved in a small volume of Tris-HCl/EDTA buffer and then dialyzed extensively to remove SDS. These immunogen preparations (in 50 µg aliquots) were each mixed separately with an equal volume of Freund's complete adjuvant and injected into rabbits subcutaneously at multiple sites in the back. The rabbits were reinjected (boosted) with the same immunogen preparation mixed with incomplete Freund's adjuvant after 3 weeks and after 7 weeks. After this the rabbits were bled and the antisera was assayed for PPV specific antibody using techniques described in Section 6.8. (Molitor _et al_., 1983, J. Virol., _45_:842-854).

Immunoprecipitation of expressed polypeptides in pPP _E. coli_ transformants was accomplished as follows: 1 mℓ overnight cultures were grown at 30°C in L-broth containing, as appropriate, either 100 µg/mℓ ampicillin or 20 µg/mℓ tetracycline. Induction was accomplished by adding 1mM IPTG or 1% lactose to the growth medium, and the host cell polypeptides were radiolabeled by the addition of 100 µCi of $^{35}$S-methionine (controls were radiolabeled but not induced). After incubation at 30°C for 2 hours, the cells were pelleted and resuspended in 0.5 mℓ cold TE-10 (20mM Tris-HCl, pH8.0, 10mM EDTA). In order to lyse the cells and release the cell contents, the cells were pelleted again and then resuspended in 50 µℓ TE-10 to which 0.5 mg lysozyme was added. After 15 minutes at 4°C the following was added: 400 µℓ TE-Triton (100mM Tris-HCl, pH8.0, 100mM EDTA, 0.2% Triton X-100 or polyoxyethylene (9-10) p-tert-octyl-phenol) and 500 µℓ 2 x RIPA (1 x RIPA is 150mM NaCl, 10mM Tris-HCl, pH7.2, 1% Triton X-100, 1% deoxycholate, 0.1% SDS; 2 x RIPA is twice the concentration of each ingredient). After 10 minutes at 4°C, 200 µℓ activated _Staph_ A protein (Pansorbin, a 10% suspension of Staphylococcus aureus cells containing protein A, Calbiochem-Behring Corp., LaJolla, Cal.) was added in order to remove all components that nonspecifically adsorbed to Staph A protein. After 10 minutes at 4°C the cell lysates were centrifuged in a microcentrifuge for at 16,000 x g 15 minutes. The supernatant was removed, adjusted to a total volume of 2 mℓ with a buffer consisting of 100mM NaCl, 2mM EDTA, 10mM Tris-HCl (pH7.2), 1% NP-40 (i.e., polyoxyethylene (9) p-tert-octylphenol), 0.5% deoxycholate and 1% aprotinin (a protease inhibitor). Each supernatant was divided in half and 4-10 μℓ of antisera was added to one half while 4-10 μℓ of pre-immune sera was added to the other half (control). The mixtures were allowed to incubate for at least 30 minutes at 4°C at which time 80-200 μℓ activated Staphylococcus aureus cells were added. After 15 minutes at 4°C, the immune complexes were pelleted by centrifugation. The pellets were washed once with 1 mℓ 1M buffer (1M NaCl, 10mM Tris-HCl, pH7.2, 1% NP-40) and twice with 1 mℓ RIPA.

The washed pellets were then prepared for SDS-PAGE as follows: the washed pellets were resuspended in 50 μℓ SB (0.07M Tris-HCl, pH6.8, 11.2% glycerol, 3% SDS, 0.01% bromophenol blue, 5% ß-ME). After heating at 95°C for 2 minutes and removing insoluble components by centrifugation, 25 μℓ aliquots of the supernatants were electrophoresed on SDS-polyacrylamide gels (Laemmli, 1970, Nature 227:680). After electrophoresis the proteins were stained with Coomassie brilliant blue dye, treated with sodium salicylate and dried for fluorography (Chamberlain, 1979, Anal. Biochem. 98:132).

Fusion proteins produced by pPP405, pPP501, and pPP700 transformants were precipitated with porcine antisera directed against PPV, however, normal porcine serum did not precipitate these fusion proteins. Similar results for clones transformed with the other pPP recombinant plasmids demonstrated that all transformants produced fusion proteins that were immunoreactive with PPV-specific antisera.

A fluorogram (not shown) of $^{35}$S-methionine labeled immunoprecipitated polypeptides of induced E. coli transformed by pPP406 indicated that the fusion proteins produced by the pPP406 transformant (with approximate molecular weights of 147,000, 138,000, and 122,000 daltons) were immunoprecipitated with antisera directed against the PPV A protein, PPV B protein, PPV C protein, and against whole PPV. The predicted size of the fusion protein produced by pPP406 based on the size of the DNA fragment inserted is 147,000 daltons.

PURIFICATION OF FUSION PROTEINS

The fusion proteins produced by E. coli transformants were isolated and purified using two methods. One involved the method for isolating aggregate-forming proteins which is outlined in detail in Section 5.5., *supra*. Briefly, L-broth containing, as appropriate, either 100 µg/mℓ ampicillin or 20 µg/mℓ tetracycline and 1% lactose or 1mM IPTG was inoculated with select transformants. After incubation at 30°C overnight the cells were pelleted and quick frozen in dry ice/methanol. The fusion protein was then isolated according to the embodiment described in Section 5.5.

An alternate method used for purifying fusion proteins involved polyacrylamide gel purification. After induction and growth as described above, the bacteria were pelleted. The cell pellet was resuspended in a volume of SDS-gel sample buffer equal to one-quarter of the original volume (Laemmli, 1970, Nature 227:680). The suspension was boiled for 10 minutes thus solubilizing the fusion protein. The insoluble debris was pelleted by centrifugation at 90,000 x g for 20 minutes. The cleared supernatant was then subjected to preparative SDS-PAGE (7.5% polyacrylamide gel). The PPV fusion protein bands were localized in the gel by dansylation (Tijssen and Kurstak, 1981, J. Virol. 37:17) and were excised from the gel. The protein was then isolated from the gel slices by repeated elution in buffer containing 0.05M $NH_4HCO_3$, 0.1% SDS at 37°C, concentrated by lyophilization, resuspended in a small volume of Tris-HCl EDTA buffer, and extensively dialzyed against buffer containing 10mM potassium phosphate (pH 6.8), 40mM NaCl, 1mM EDTA, 1mM ß-ME, and 50% glycerol (Molitor et al., 1983, J. Virol., 45:842-854).

## IMMUNIZATION OF LABORATORY ANIMALS WITH PREPARATIONS OF PPV/ß-GALACTOSIDASE FUSION PROTEINS

As described above, many plasmid constructions were generated that resulted in the expression of inducible fusion proteins containing antigenic sites recognized specifically by antisera directed against the PPV structural proteins. To determine if such bacterial fusion protein products were able to elicit PPV-specific immune responses when injected into animals (i.e., act as immunogens) polyacrylamide gel purified fusion polypeptides were used to immunize laboratory rabbits.

All preparations were started by culturing select transformants as follows: 10 mℓ of L-broth containing as appropriate either 100 μg/mℓ ampicillin or 20 μg/mℓ

tetracycline was inoculated with a single transformant and the inoculum was incubated with agitation at 37°C overnight; 5-6 mℓ of this culture was used to inoculate 1 liter of LB medium containing 1% lactose and as appropriate either 100 μg/mℓ ampicillin or 20 μg/mℓ tetracycline which was incubated with agitation at 30°C overnight.

Rabbit 013 and 015 were immunized with a fusion protein preparation derived from E. coli pPP405 transformants. The fusion protein was purified according to the polyacrylamide gel protein purification method described in Sections 6.6.2. Rabbits were bled to obtain pre-immune sera (pre-bled) and then injected with 50-100 μg of fusion protein mixed in a 1:1 ratio with Freund's complete adjuvant. After 28 days, rabbits were bled and then boosted with 40-50 μg of fusion protein mixed in a 1:1 ratio with Freund's incomplete adjuvant. Seven to 10 days after the booster injection, animals were bled and sera (antisera) prepared.

## ANALYSIS OF ANTISERA FOR PPV-SPECIFIC IMMUNE RESPONSE

The various antisera were analyzed for their specificity for PPV antigens and ability to neutralize viral infectivity in vitro. Antisera specificity was analyzed by immunoprecipitation and hemagglutination inhibition (HAI) assays (since PPV agglutinates red blood cells, inhibition of hemagglutination by antiserum indicates that the antiserum is specific for PPV antigens). Two other assays were used to evaluate the antisera reactivity: serum neutralization-HA (SN-HA), and serum neutralization-direct fluorescent antibody assay (SN-DFA). Each assay is described in the following subsections and the results of each assay are shown in Table 1.

TABLE 1

| Rabbit Antisera | PPV Serology HAI[1] | SN-HA[2] | SN-DFA[3] |
|---|---|---|---|
| 013 Pre-bleed | 8 | 4 | 4 |
| 013 1st bleed | 64 | 64 | 16 |
| 013 2nd bleed | 64 | 64 | 16 |
| 015 Pre-bleed | 8 | 4 | 4 |
| 015 1st bleed | 128 | 256 | 16 |
| 015 2nd bleed | 64 | 64 | 16 |

[1] HAI is expressed as the reciprocal of the highest serum dilution which resulted in 50% inhibition of hemagglutination (see Section 6.8.2.).

[2] SN-HA is expressed as the reciprocal of the highest serum dilution which resulted in complete inhibition of infection of susceptible tissue culture cells (see Section 6.8.3.).

[3] SN-DFA is expressed as the reciprocal of the highest dilution which prevented the appearance of PPV infection-specific fluorescence of cultured cells (see Section 6.8.4.).

IMMUNOPRECIPITATION OF RADIOLABELED PPV-INFECTED PORCINE CELL LYSATES

To test the specificity of antisera from rabbits 013 and 015 for PPV structural proteins, the antisera were

0117767

used to immunoprecipitate $^{35}S$-methionine radiolabeled PPV-infected ST (Swine Testicle) cell lysates. Recently trypsinized, subconfluent ST cell cultures grown in Dulbecco's minimal essential medium supplemented with 5% fetal calf serum, 0.25% lactoalbumin hydrolysate and penicillin (100 µg/mℓ) were inoculated with 0.2 mℓ of PPV, strain NADL-8 (approximately 1024 hemagglutination units/50 µℓ), incubated for 90 minutes at 37°C, washed, and cultured in fresh growth medium. About 36-48 hours post-infection, growth medium was removed and the cultures were washed three times with methionine-free minimal essential medium. Fresh methionine-free medium containing 100 µCi/mℓ $^{35}S$-methionine was added, and the cell cultures were incubated for 2-3 hours at 37°C. Cells were then harvested, washed twice with cold STE buffer (150mM NaCl, 10mM Tris-HCl, pH 7.2, 1mM EDTA), and the cell pellet was stored at -20°C until needed. Radiolabeled cell pellets were resuspended in lysis buffer (100mM NaCl. 2mM EDTA, 10mM Tris-HCl, pH 7.2, 1% NP-40, 0.5% deoxycholate, 1% aprotinin). The lysed cell suspension was then cleared by centrifugation at 90,000 x g for 30 minutes. Antiserum (3-6 µℓ aliquots) was added to each cleared lysate and incubated at 4°C for 30 minutes. Pansorbin (10% suspension of protein A-containing Staphylococcus aureus cells, Calbiochem Inc.) was added in an amount equal to 20 times the volume of antiserum used and the mixtures were incubated at 4°C for 10 minutes. The immune complexes were pelleted and washed once with 1M buffer (1M NaCl, 10mM Tris-HCl, pH 7.2, 0.1% NP-40) and twice with RIPA buffer (150mM NaCl, 10mM Tris-HCl, pH 7.2, 1% Triton X-100, 1% deoxycholate, 0.1% SDS). The washed pellets were resuspended in 1X sample buffer, (5% ß-mercaptoethanol, 0.07M Tris-HCl, pH 6.8, 11.2% glycerol, 3% SDS and 0.01% bromophenol blue) and boiled for one minute. After removal of insoluble debris by centrifugation in a microcentrifuge, the material was electrophoresed in SDS polyacrylamide gels (10% polyacrylamide) (Laemmli, 1970, Nature 227:680). The gels were prepared for fluorography by soaking in sodium salicylate (Chamberlain, 1979, Anal. Biochem. 98:132) and the dried gels were exposed to Dupont Cronex 4 X-ray film at -70°C. Rabbit antisera 013 and 015 each immunoprecipitated the PPV structural proteins while pre-immune sera did not (data not shown) thus demonstrating the specificity of these antisera for PPV.

HEMAGGLUTINATION INHIBITION (HAI)

The specificity of antisera 013 and 015 for PPV and the antibody titers were determined and quantitated in a hemagglutination inhibition test (Joo et al., 1976, Aust. Vet. J. 52:422). Briefly, serum complement was heat inactivated (56°C for 30 minutes) and the heat inactivated sera (rabbit antisera 013, 015 and the corresponding pre-immune control sera) were first incubated with 25% kaolin for 20 minutes, centrifuged and then incubated with 50% guinea pig red blood cells for 60 minutes. This served to remove nonspecific hemagglutinins. Sera were then diluted in PBS (phosphate buffered saline) in microtiter plates. A standard amount of PPV (8 hemagglutination units/50 µℓ) was added to each microtiter well and allowed to incubate for 3 hours at room temperature or overnight at 4°C. Then a 0.6% suspension of guinea pig red blood cells was added to each well and incubated at 22°C for 2 hours. The endpoint for the HAI assay was expressed as the reciprocal of the highest dilution of serum which resulted in 50% inhibition of hemagglutination. Table 1 indicates that antisera 013 and 015, each directed against a PPV/ß-galactosidase

fusion protein product produced as described herein, were capable of inhibiting hemagglutination by PPV.

SERUM NEUTRALIZATION-HEMAGGUTINATION ASSAY

The serum neutralization (SN) assay (Joo et al., 1975, Arch. Virol. 47: 337) was performed as follows: briefly, serum complement was heat inactivated (56°C for 30 minutes) and the inactivated sera (rabbit antisera 013 and 015 and the corresponding pre-immune sera controls) were serially diluted in phosphate buffered saline (PBS). The inactivated diluted sera were incubated with a standard amount of PPV (as described in Section 6.8.2.) for 2 hours at 37°C. Aliquots of the sera-PPV mixture were added to ST cell monolayers in microtiter plates (i.e., growth media was removed from the microtiter wells and 0.2 mℓ of the serum-virus mixtures were added to each well). These inoculums were allowed to adsorb for 90-120 minutes after which the cultures were washed with PBS and fresh growth medium was added. After seven days at 37°C a 0.6% suspension of guinea pig red blood cells was added to all wells and incubated at 22°C for 2 hours; the presence of PPV in the media surrounding the ST cells (i.e., as a result of virus infection) could be detected by hemagglutination (HA). No HA is found if the virus was neutralized by the antisera. Neutralization of virus infection by each antiserum tested was recorded as the reciprocal of the highest dilution giving no HA. The results in Table 1 indicate that both rabbit antisera 013 and 015 were capable of neutralizing virus infectivity.

SERUM NEUTRALIZATION-DIRECT FLUORESCENT ANTIBODY ASSAY

Direct fluorescent antibody assays (DFA) (Bommelli et al., 1980, Vet. Imm. Immunopath. 1:179) were performed in triplicate. Serial dilutions of sera (each antiserum and pre-immune control serum) in PBS (total volume 0.3 mℓ) were mixed and incubated with 0.3 mℓ of virus (8 hemagglutination units/50 μℓ) for 60 minutes at 22°C. Two hundred microliters of this mixture was then added to Leighton tubes containing recently trypsinized ST cells previously washed with PBS. Controls included known positive sera, known negative sera (i.e., pre-immune sera), no serum, and no virus. The inocula were allowed to adsorb for 90-120 minutes, after which the cultures were washed with PBS and fresh growth medium added. Four days following inoculation, the Leighton slips were removed, dried in acetone, stained directly with fluorescent antibody (FA) conjugate, washed, and observed under a fluorescent microscope. The FA conjugate consisted of fluorescent isothiocyonate conjugated to porcine anti-PPV antibodies produced in gnotobiotic pigs (Mengeling, 1972, Am. J. Vet. Res. 33:2239). Virus infection of cells was considered positive when several foci of fluorescently labeled cells were apparent on any given Leighton slip. Neutralization of virus infection was indicated by the absence of these fluorescent foci. Table 1 indicates that rabbit antisera 013 and 015 were each capable of inhibiting virus infection of the ST cells.

## CONSTRUCTION OF pPP450A WHICH PRODUCES BOTH cro/PPV AND cro/PPV/ß-GALACTOSIDASE FUSION PROTEIN

A recombinant plasmid, pPP450A, was constructed which can direct the production of both a fused (i.e., fused to ß-galactosidase) and an unfused PPV protein (i.e., not fused to ß-galactosidase) in appropriate host cells. Both proteins were fused to cro sequences.

0117767

The pPP450A plasmid was derived from a series of recombinant plasmids which contain PPV sequences and an expression vector which contains unique restriction endonuclease recognition sequences located at the junction of the cro gene and the z-gene. Details of the procedure are described below.

pPP530

The construction of this plasmid is depicted in FIG. 10. An approximately 1.7 kb PPV DNA fragment was generated by digestion of pPPV-6 with PstI followed by treatment with mung-bean nuclease in order to remove the single stranded end at the PstI site and thus to create blunt ends. After digestion with HindIII, the 1.7 kb HindIII/blunt ended PPV DNA fragment was purified by agarose gel electrophoreis. The expression vector pHK413 was digested with HindIII and SmaI and the 6.5 kb backbone was gel purified. The 1.7 kb fragment and the 6.5 kb backbone were annealed in a 5:1 molar ratio and ligated with T4 DNA ligase. As a result of this process, the HindIII site was restored, however, the PstI and SmaI sites were destroyed. Unexpectedly, the recombinant plasmid pPP530 was missing approximately 400 b.p. of PPV DNA sequence at the PstI site and consequently contained 1.3 kb of PPV sequence.

pPP550

The recombinant plasmid pPP550 was constructed from pPP530 and pHK413 in the following manner: pPP530 was digested with BamHI and then treated with nuclease BAL 31 in order to remove approximately 150 b.p., thus removing the stop codon. The plasmid was then digested with PstI. The resulting 2.1 kb PstI/blunt ended fragment was then purified by agarose gel electrophoresis. The expression vector pHK413 was digested with SmaI and PstI and the 5.5 kb SmaI/PstI fragment which contained the site of origin was gel purified. The 2.1 kb PstI/blunt ended fragment and the 5.5 kb SmaI/PstI fragment were annealed in 5:1 molar ratio and ligated using T4 DNA ligase as shown in FIG. 11. As a result of this process, the PstI site was restored, however, the BamHI (from pPP530) and SmaI (from pHK413) sites were destroyed. Transformants were screened for the ability to form red colonies on MacConkey agar. One transformant, pPP550, produced a PPV/ß-galactosidase protein.

pPP450

Plasmid pPP450 was constructed from pPP406 (described supra) and pPP550 as described in FIG. 12. The plasmid pPP406 was digested with PstI and HindIII. The resulting 1.3 kb PstI/HindIII fragment was gel purified. The plasmid pPP550 was digested with PstI and HindIII. The resulting 6.8 kb fragment was gel purified. The 1.3 kb fragment and the 6.8 kb fragment were annealed in a 5:1 molar ratio and ligated with T4 DNA ligase. As a result of this process, the HindIII and PstI sites were restored.

PHK414-7X

In order to introduce an amber chain termination sequence between the cro sequence and the z-gene, the plasmid pHK414 was first digested with HindIII and BamHI and the 6.5 kb backbone was purified by agarose gel electrophoresis. DNA linkers which have HindIII/BamHI cohesive ends, an internal XbaI site and an amber sequence (TAG) were annealed in a 10:1 molar ratio to the linearized plasmid and ligated using T4 DNA ligase (see FIG. 13). The complementary strands of the DNA linker were synthesized by the solid phase method of Chow et al., 1981, Nucleic Acids Res. 9 (12):2807-2817. As a result of

this process, the *Bam*HI site was restored, however, the *Hin*dIII site was destroyed.

pPP450A

The construction of plasmid pPP450A is shown in FIG. 14. Plasmid pPP450 was digested with *Pst*I and *Bam*HI. The 2.5 kb *Pst*I/*Bam*HI fragment was purified by gel electrophoresis. The vector pHK414-7X was digested with *Pst*I and *Bgl*II, and the 5.5 kb *Pst*I/*Bgl*II fragment was purified by gel electrophoresis.

The 2.5 kb *Pst*I/*Bam*HI fragment and the 5.5 kb *Pst*I/*Bgl*II pHK414-7X fragment were annealed in a 5:1 molar ratio and ligated using T4 DNA ligase. As a result of this process the *Pst*I site was restored, however the *Bgl*II and *Bam*HI sites were destroyed. In addition, the amber sequence of the DNA linker was in phase with the translational reading frame of the PPV and the z-gene.

pPP450A TRANSFORMANTS

The pPP450A plasmid was used to transform *E. coli* NF1829. When induced with lactose, the ampicillin transformants synthesized no detectable levels of fusion protein (*i.e.*, no red colonies on MacConkey indicator agar plates). The transformants were then infected with the lysogenic transducing phage, Ø80SuIII, which carries an amber suppressor tRNA gene. The lysogenized transformants produced pink colonies on MacConkey agar plates, indicating fusion protein production.

The pPP450A plasmid was also used to transform *E. coli* LE392 which carries two amber suppressor (supE and supF) but does not have the *laci*$^q$ mutation of NF1829 and therefore does not oversynthesize lac repressor. These transformants produced both a fusion protein and an unfused protein in the presence or absence of the inducer lactose or IPTG.

## IDENTIFICATION OF TRANSFORMANTS THAT PRODUCE FUSION AND UNFUSED PROTEINS

To determine if transformants were capable of synthesizing the predicted fusion and unfused protein, proteins were isolated and analyzed by SDS-PAGE followed by Coomassie Brilliant Blue staining.

Either one of the following mini-aggregate procedures was used to isolate aggregate proteins for screening analysis:

(1) Duplicate culture tubes containing 5 mℓ of fresh Luria broth containing 100μg/mℓ ampicillin were inoculated with 200μℓ of cells obtained from an overnight culture and grown for 90 minutes at 37°C. One of each duplicate was induced by the addition of 1mM IPTG (final concentration). The inocula were then grown with shaking, for a further 5 hours at 37°C.

(2) The cells contained in a 3 mℓ aliquot of the culture were pelleted by centrifugation in a microcentrifuge (12,000 x g) for 2 minutes. (N.B., the total volume of a microcentrifuge tube is 1.5 mℓ, therefore, a 1.5 mℓ aliquot was first pelleted and the supernatant was drawn off; another 1.5 mℓ aliquot was added to the same microcentrifuge tube and pelleted in the same tube.)

(3) The cell pellet was resuspended in 50μℓ of 25% sucrose containing 50mM Tris-HCl, pH8, and the suspension was frozen by placing the tube in a dry ice/ethanol bath.

(4) The frozen suspension was thawed, 50μℓ of 10 mg/mℓ lysozyme in 0.25M Tris-HCl pH8 was added, and the suspension was incubated for 10 to 15 minutes on ice.

(5) After the 10 to 15 minute incubation 400μℓ of TET buffer (100mM Tris-HCl pH8, 50mM EDTA, 2% Triton X-100; TritonX-100 is a non-ionic detergent: polyoxyethylene (9-10) p-*tert*-octyl phenol) was added, the suspension was gently mixed and incubated for 5 minutes on ice. Then 500μℓ of 2xRIPA (2xRIPA is 20mM Tris-HCl, pH7.4, 300mM NaCl, 2% sodium deoxycholate, 2% NP-40, 0.2% SDS) was added and the suspension was mixed gently and incubated for 5 minutes on ice.

(6) The cells in the suspension were then sonicated for 10 seconds using a microprobe, and the suspension was cleared by centrifugation in a Sorvall SS34 rotor for 30 minutes at 20,000 r.p.m. (47,800 x g).

(7) The supernatant was decanted and the pellet, which contains the co-aggregate proteins, was resuspended in 50μℓ distilled $H_2O$ to which 50μℓ of 2x Sample Buffer (2x Sample Buffer is composed of 10% SDS, 40mM Tris-HCl, [pH6.8, 2% ß-ME, and 0.02 volumes saturated solution of bromphenol blue) was added and mixed well.] The mixture was boiled for 5 minutes and 25μℓ aliquots were applied to 7.5% or 10% SDS-polyacrylamide gels.

The proteins produced were of the size predicted for fusion (approximately 173,000 daltons) and unfused proteins (approximately 55,500 daltons).

Alternatively aggregate proteins were isolated and analyzed as follows:

(1) Duplicate culture tubes of 10 mℓ of Luria broth were inoculated with a single colony. One of each duplicate was induced by the addition of 20% lactose (final concentration 1%). The inocula were then grown with shaking for 6-24 hours at 30°C or 37°C.

(2) The cells were pelleted by centrifugation in a Sorvall SS34 rotor at 9148 x g for 2-5 minutes.

(3) The cell pellet was resuspended in 200 µℓ of 25% sucrose containing 50mM Tris-HCl (pH 8.0), and the suspension was frozen. In some instances 1-10mM EDTA was added to the solution.

(4) The frozen suspension was thawed, 200 µℓ of 10 mg/mℓ lysozyme in 0.25M Tris-HCl pH 8.0 was added, and the suspension was incubated for 10 minutes on ice.

(5) After incubation 1.6 mℓ of TET buffer was added, the suspension was gently mixed and incubated for 5 minutes on ice. Then 2.0 mℓ of 2x RIPA was added and the suspension was mixed and incubated for 5 minutes on ice.

(6) The suspension was then sonicated for 10 seconds using a microprobe as described <u>supra</u>.

(7) The supernatant was decanted and the pellet which contains the co-aggregate proteins was resuspended

in 50-100 μℓ distilled $H_2O$ and 2x Sample buffer and electrophoresed on SDS-polyacrylamide gels as described supra.

## IMMUNIZATION OF PIGS WITH BACTERIALLY DERIVED PPV PROTEIN

The immune response of pigs injected with bacterially derived PPV protein was studied. Twenty pigs were randomly split into 4 groups of 5. Two groups served as controls. While one of the control groups was not injected, the other received 2 doses of a commercial vaccine. The other two groups were injected with either whole cells or purified PPV protein encoded by pPP450A protein which had been treated with formalin. Pigs were injected intramuscularly (IM).

*E. coli* LE392 transformed with pPP450A was grown in L-Broth containing 1% lactose and 100 μg/mℓ ampicillin for 24 hours while shaking at 37°C. The culture was placed in a 68°C water bath and an equal volume of saline (100°C) was added with mixing. After 30 minutes in the water bath, the cells were harvested and resuspended in sterile saline. The cells were concentrated 40 fold and resuspended in saline. 2.5 mℓ was used for each dose.

Purified PPV protein was obtained from *E. coli* LE392 transformed with pPP450A and grown in L-broth containing 1% lactose and 100 μg/mℓ ampicillin for 24 hours at 37°C while shaking. The proteins were purified by the modification of the aggregate purification procedure described supra in Section 5.5.

Before injection each of these preparations was shown to be sterile and passed the USDA mouse safety test. Pigs were injected on day 0 and 21 with purified (aggregate) or unpurified (cells) fusion protein which was

0117767

sterilized with formalin (0.4%) at room temperature overnight with agitation and mixed with an equal volume of a sterilized aluminum hydroxide (Rehsorptar, Reheis Chemical Co., Berkeley Heights, N.J.) solution, diluted as necessary to achieve a final concentration of 5 and 15%, respectively. Two hours after the addition of formalin Tris-HCl (1M, pH7.6) was added to a final concentration of 20mM. Each dose of whole cells contained approximately 2.5 mg of unfused protein and 6 mg of fusion protein. Each dose of purified protein consisted of approximately 5 mg of unfused protein cro/PPV and 12.5 mg of cro/PPV/ß-galactosidase fusion protein.

All animals, to varying degrees, responded to the formalin treated preparations (see Table 2). None of the uninoculated control animals seroconverted while several of the pigs which received the commercial vaccine did.

TABLE 2

RESPONSE OF PIGS TO BACTERIALLY DERIVED PPV STRUCTURAL PROTEIN ENCODED BY pPP450A

| Antigen | Reciprocal of Mean of SN-DFA[1] Pre | Reciprocal of Mean of Titer Post |
|---|---|---|
| Aggregate Protein | 8 | 769 |
| Cells | 4 | 678 |
| Commercial Vaccine | 9 | 616 |
| None | 10 | 14 |

[1] SN-DFA is expressed as the reciprocal of the highest serum dilution which prevented the appearance of PPV infection-specific fluorescence of cultured cells. The procedure used is identical to that described in Section 6.8.4. except that the experiments were done in microtiter plates rather than in Leighton tubes.

0117767

The results in Table 2 indicate that bacterially derived PPV structural protein encoded by pPP450A were capable of eliciting neutralizing antibody.

CHARACTERIZATION OF IMMUNE RESPONSE OF RABBITS TO FORMALIN TREATED AGGREGATE PPV FUSION PROTEIN

To characterize the immune response of rabbits to PPV fusion protein, rabbits were injected with 2 doses of 15-20 mg of PPV bacterially derived proteins expressed by *E. coli* LE392 transformed with pPP450A on day 0 and 35. Each dose contained 5 mg of cro/PPV protein and 12.5 to 15 mg of the cro/PPV/ß-galactosidase fusion protein. The aggregate protein (isolated by the modification of the aggregate purification procedure described *supra* in Section 5.5.) was suspended in water, treated with formalin at room temperature or 4°C for 6-18 hours, and mixed with 2.5 mg L-121 (Hunter *et al.*, 1981, J. of Immunol. 127(3):1244-1250; BASF Wyandotte Corporation, Wyandotte, Mich.) adjuvant either immediately or several hours prior to injection. As a control, animals were injected with untreated bacterially derived proteins mixed with adjuvant (2.5 mg L-121).

Antisera were analyzed for its ability to immunoprecipitate (IP) viral proteins from radiolabeled infected cell lysates. Results are reported below in Table 3 as the number of animals positive/total number injected.

TABLE 3

IMMUNE RESPONSE OF
RABBITS TO FORMALIN TREATED AGGREGATE PPV
BACTERIALLY DERIVED FUSION PROTEIN

| Immunogen | Final Concentration Formalin (%) | Adjuvant | Characterization of Immune Response IP |
|---|---|---|---|
| cro/PPV and cro/PPV/ß-galactosidase | 0.0 | L-121 | 0/3[1] |
| cro/PPV and cro/PPV/ß-galactosidase | 0.2 | L-121 | 3/3[1] |

[1] Antisera were drawn 1 week post-boost.

To characterize the immune response of rabbits to formalin treated aggregate PPV bacterially derived fusion protein, the antisera were used to immunoprecipitate $^{35}S$-methionine radiolabeled PPV-infected ST cell lysates as described in detail in Section 6.8.1.

Samples were electrophoresed in SDS polyacrylamide gels (10%) and the gels were prepared for fluorography as described supra. Rabbit antisera to formalin treated aggregate PPV bacterially derived fusion protein immunoprecipitated the PPV structural genes while antisera of rabbits not treated with formalin did not immunoprecipitate PPV structural proteins.

## CONSTRUCTION OF ptac CLONES

Various "tac" or trp-lac promoter/ operator region clones were constructed. The hybrid promoter was constructed by combining the -35 region and the -10 region (the sequences which are the RNA polymerase binding site) of the tryptophan and the lactose promoters.

## CONSTRUCTION OF pPP450tc

The plasmid pDR540 (obtained from PL-Biochemicals, Milwaukee, WI) contains the strong tac (or trp-lac) promoter. This plasmid was linearized by digestion with HindIII and the cohesive ends were filled-in using the Klenow fragment of DNA polymerase I. The reaction mixture was phenol extracted, precipitated, and the DNA was religated and used to transform E. coli NF1829. One clone, p809 was selected.

Plasmid p809 (see FIG. 15) was digested with BamHI, filled-in with the Klenow fragment of DNA polymerase I, and the plasmid backbone was isolated in low melting point agarose. In order to isolate a cro (ATG) sequence, followed by BglII and HindIII sites for cloning, plasmid pPP520 (described supra) was digested with BstEII, the cohesive ends were filled-in using the Klenow fragment of DNA polymerase I, and then cleaved with RsaI. The fragments were resolved in a 5% acrylamide gel and the approximately 170 b.p. fragment which contained cro (ATG)/PPV sequences was eluted from the gel, and the DNA was precipitated with ethanol. The RsaI/BstEII fragment of pPP520 and the filled-in linearized backbone of p809 were annealed in a 5:1 molar ratio and ligated using T4 DNA ligase. The ligation mixture was used to transform E. coli NF1829. Transformants were selected for ampicillin resistance and plasmids were screened by restriction endonuclease digestion with EcoRI and BglII to identify a plasmid containing the insert in the correct orientation, i.e., the cro sequences ligated to the tac promoter of p809 with no intervening PPV sequences (See FIG. 15). One clone selected, p830, contained the ptac/$SD^{cro}$ (ATG)/PPV sequences in the proper orientation.

Plasmid p830 was digested with NcoI/BglII (see FIG. 16) and the 4 kb ptac bearing fragment was isolated by agarose gel electrophoresis. Plasmid pPP405 (described supra) was digested with BglII/NcoI and an approximately 475 b.p. fragment was isolated. The 475 b.p. BglII/NcoI fragment and the 4 kb tac fragment were annealed in a 5:1 molar ratio and ligated with T4 DNA ligase, and plasmid p830/405 was isolated.

In order to place the PPV sequences in phase with the cro sequences, the plasmid p830/405 was digested with BglII, filled-in with the Klenow fragment of DNA polymerase I and religated. Plasmid p989 was isolated as described in FIG. 16 (N.B. plasmids p803/405 and p989 did not contain z sequences).

Plasmid p989 was digested with PstI and HindIII (see FIG. 17) and the ptac bearing fragment was isolated by agarose gel electrophoresis. Plasmid pPP450 was also digested with HindIII/PstI and the lacz bearing fragment was isolated by gel electrophoresis. The ptac fragment of p989 and the HindIII/PstI fragment of pPP450 were annealed in a 5:1 molar ratio and ligated.

The ligation mixture was used to transform E. coli NF1829. Plasmid pPP450tc was identified by protein analysis by SDS-PAGE of induced E. coli transformants.

CONSTRUCTION OF pPP450Atc

Plasmid pPP450Atc was constructed to produce the cro/PPV and cro/PPV/ß-galactosidase proteins using the tac promoter. Plasmid pPP450tc was digested with HindIII and PstI and the 1.6 kb ptac fragment was isolated by gel electrophoresis (see FIG. 18). An approximately 7.0 kb HindIII/PstI fragment of pPP450A which contained PPV amber i-z sequences was isolated and the two fragments were annealed in a 5:1 molar ratio and ligated with T4 DNA ligase. The ligation mixture was used to transform E. coli strain F' LE392 (F factor plasmid which carries on it the $i^q$ mutation which leads to the overproduction of the lac repressor protein). These transformants produced both cro/PPV (53,000 daltons) and cro/PPV/ß-galactosidase (173,000 daltons) proteins in the presence or absence of inducer lactose.

Proteins were identified after isolation by either of the mini-aggregate procedures described supra (see Section 6.9.2.) or by the modification of the aggregate purification procedure described in Section 5.5. supra and analysis by SDS-PAGE.

EXAMPLE: CANINE PARVOVIRUS

The canine parvovirus (CPV) genetic sequences were obtained from RF DNA of CPV, Cornell, virulent strain. The double-stranded RF DNA was cleaved with restriction enzymes and ligated directly into expression vectors possessing complementary termini and were used to transform host E. coli. Alternatively, the RF DNA was tailed with terminal transferase. The CPV gene products which were isolated as fusion proteins were formulated for use as immunogens. The procedure is described in detail below.

## GENERAL PROCEDURES USED FOR PREPARATION OF THE PLASMIDS

The procedures used for host cell transformation, DNA isolation, enzyme reactions, DNA modification, gel purification and DNA ligation reactions are identical to those described in Section 6.1. and its subsections. Unless otherwise indicated in the text, these procedures were utilized in the practice of this example of the present invention.

## PREPARATION OF REPLICATIVE FORM DNA (RF)

The RF CPV DNA was isolated from infected cells as discussed in Section 5.1. according to the procedure of Hirt (1967, J. Mol. Biol. 26:365 and Spalholz *et al.*, in Tumor Viruses and Differentiation, eds. Scolnick, E.M. and Lebiner, A.J., 1983, Alan R. Liss, Inc., N.Y.). Briefly, Norden Feline Kidney cells were grown in Autopow (Flow) monolayer medium supplemented with 10% fetal calf serum, sodium pyruvate, non-essential amino acids, glutamine, penicillin and streptomycin. Cells were seeded to give 1 x $10^7$ cells per 150 mm dish at the start of infection. Monolayers were infected by removing medium and replacing with 10 mℓs of medium containing CPV strain Cornell 916-4 calculated to produce complete monolayer destruction in 48 hours. After incubation at 37°C for 8 hours, 15 mls of medium was added. Cells were harvested at 26 hours post infection, washed in 0.15M NaCl, 20mM Tris-HCl pH 7.5, 10mM EDTA and resuspended at less than $10^8$ cells per mℓ. Four volumes of lysis buffer (0.75% SDS, 1.25M NaCl, 20mM Tris-HCl pH 7.5, 10mM EDTA containing 250 μg/mℓ

proteinase K) was added to the cells and the mixture was incubated for 2 hours at 37°C then for 8 hours at 0°C. The lysed cell suspension was cleared by centrifugation and the supernatant was ethanol precipitated. The pellet was resuspended in 10mM Tris-HCl pH 7.5, 1mM EDTA and treated with 50-100 mg/mℓ RNase A for 1 hour at 37°C, phenol extracted, and dialysed against 10mM Tris-HCl pH 7.5, 1mM EDTA. At this point the DNA contained a mixture of single stranded viral DNA released from virions and RF duplex monomers, dimers, tetramers and replicative intermediates containing single stranded tails. To remove DNA with any single stranded regions the mixture was passed through BD cellex (Biorad, Richmond, Ca.) in 1M NaCl, allowing single stranded DNA to attach to the column and duplex DNA to flow through. The RF monomer of approximately 4900 b.p. was further purified by agarose gel electrophoresis.

## ISOLATION AND MOLECULAR CLONING OF CPV GENETIC SEQUENCES

Based on the CPV restriction map reported by McMaster et al., 1981, J. Virol. 38(1):368, fragments of CPV DNA containing DNA sequences from the middle towards the 5'-end of the CPV genome were generated by digestion of the RF CPV DNA with appropriate restriction enzymes; see FIG. 21 for a diagrammatic representation of the restriction map of the CPV genome (only relevant restriction sites are indicated). These CPV DNA fragments were inserted into the expression vector pHK413 (described in Section 6.5.1). Other DNA fragments, derived from various portions of the CPV genome were inserted into pBR322. The various recombinant plasmids are depicted in FIG. 21 and described in the text that follows. All base pair sizes of DNA fragments in this example are estimates based on the relative electrophoretic mobilities of the various restriction fragments generated. DNA fragments are indicated by their base pair sizes and terminal restriction sites, as previously described in Section 6.5.2. except that b.p. sizes are estimates.

THE pCPV RECOMBINANT PLASMIDS

The RF CPV DNA was dC tailed using TdT as follows: 1.5 µg RF CPV DNA was sliced out of a LMP agarose gel and placed at 65°C for 15 minutes to melt the agarose slice. This was incubated in a 50 µℓ reaction mixture (at a final pH 6.9) containing 140mM sodium cacodylate, 30mM Tris-HCl, 1mM cobalt chloride, 0.1mM DTT, 1.0mM dCTP and 20 units TdT. After incubation at 37°C for 5 minutes the tailing reaction was terminated by the addition of 3 µℓ of 0.2M EDTA and by heating at 65°C for 3 minutes. Since the RF CPV DNA was not treated with a DNA polymerase to fill in the nicks or gaps, tailing reactions resulted in a mixed population of dC tailed double-stranded DNA molecules derived from different portions of the CPV genome. This population of DNA molecules was then annealed to pBR322 which previously had been digested with PstI, gel purified and dG tailed using TdT as follows: 500 ng of the PstI cleaved pBR322 was incubated in a 50 µℓ reaction mixture (at a final pH 6.9) containing 140mM sodium cacodylate, 30mM Tris-HCl, 1mM cobalt chloride, 0.1mM DTT, 1.0mM dGTP and 10 units of TdT. After incubation at 37°C for 1 minute the reaction was terminated by the addition of 3 µℓ of 0.2M EDTA and by heating at 65°C for 3 minutes. The annealing reaction mixture was first incubated at 65°C for 5 minutes followed by 42°C for 2 hours. After the annealing reaction mixture was allowed to come to room temperature, the mixture was used to transform *E. coli* K12 strain MC1000 and ampicillin- sensitive, tetracycline-resistant colonies were selected as previously described (Section 6.4.).

Plasmid DNA was isolated from several clones and screened for DNA insert size by digestion with PstI and BglII or PstI and EcoRI followed by agarose gel electrophoresis (restriction mapping). The plasmid DNA of three ampicillin-sensitive, tetracycline-resistant clones, designated pCPV-2, pCPV-9, and pCPV-10 are diagrammatically represented in FIG. 19; however, the restriction enzyme recognition sites which are located within these CPV DNA inserts have not been fully mapped and thus are not indicated in the figure. In each of these recombinant plasmids the CPV DNA insert is flanked by PstI restriction enzyme recognition sites (the dC/dG-tailing reactions restore the PstI recognition sequence).

THE pCP EXPRESSION PLASMIDS

The RF CPV DNA, isolated as described previously, was completely digested with PvuII and partially digested with HindIII. The DNA fragments that were generated were gel purified by electrophoresis in LMP agarose gels and pooled. This generated a number of different DNA fragments derived from a region containing sequences extending from the middle towards the 5'-end of the CPV genome (i.e., see FIG. 19; the fragments were derived from the region approximately between positions 1600 and 3800 of the 5000 b.p. genome). Thus, a 2200 b.p. HindIII/PvuII DNA fragment, a 1500 b.p. HindIII/PvuII DNA fragment, and a 700 b.p. HindIII/HindIII DNA fragment were generated.

The expression vector, pHK413, was first digested with SmaI followed by HindIII. The pooled gel purified CPV DNA fragments were added to the cleaved pHK413. This mixture was ligated using T4 DNA ligase and used to transform _E. coli_ strain NF1829. Three clones designated pCP100, pCP110, and pCP200 (shown in FIG. 19) contain the 2200 b.p. _Hin_dIII/_Pvu_II DNA fragment, the 700 b.p. _Hin_dIII/_Hin_dIII DNA fragment, and the 1500 b.p. _Hin_dIII/_Pvu_III DNA fragment, respectively. Presumably, pCP110 resulted from the annealing of the 700 b.p. _Hin_dIII/_Hin_dIII CPV DNA fragment to a pHK413 vector that had been cleaved only by _Hin_dIII and not by _Sma_I.

The recombinant plasmids pCP300 and pCP310 were constructed from pCP100, whereas pCP400 was constructed from pCP200. Each is described below and depicted in FIG. 19.

pCP300

The recombinant plasmid pCP100 was digested with _Bgl_II. The _Bgl_II cohesive ends were filled in using DNA polymerase Klenow fragment and then the large blunt ended molecule was gel purified and religated using T4 DNA ligase. This resulted in pCP300 wherein the 1800 b.p. _Bgl_II/_Bgl_II DNA fragment of pCP100 was deleted.

pCP310

The recombinant plasmid pCP100 was digested with _Bgl_II and then treated with 1 µℓ of mung-bean nuclease [_N.B._ 1 µℓ of the P.L. Biochemicals mung-bean nuclease preparation was used regardless of its concentration] per 1 to 10 µg DNA for 30 minutes at room temperature in the following buffer: 0.03M sodium acetate (pH 4.6), 5% glycerol, 0.05M NaCl, and 1mM $ZnCl_2$.

After the linearized plasmid was treated with mung-bean nuclease in order to remove the single-stranded ends of the BglII site, the cleaved plasmid was electrophoresed and the larger molecule was purified from LMP agarose prior to religation using T4 DNA ligase. The ligated molecules were used to transform E. coli NF1829. One of the resulting transformants, pCP310, was found to have had more than the single-stranded end of the CPV DNA insert removed, however, at least 200 b.p. of the CPV DNA insert was retained by the transformant (see FIG. 19).

pCP400

The recombinant plasmid pCP200 was digested to completion with HincII (resulting in HincII blunt ends) followed by total digestion with BamHI. A HincII/BamHI fragment (approximately 750 b.p.) was gel purified. The expression vector pHK413 was cleaved with HindIII. After the resulting HindIII cohesive ends were filled in using T4 DNA polymerase Klenow fragment, the vector was cleaved with BamHI. The 750 b.p. HincII/BamHI pCP200 DNA fragment and the 6.5 kb blunt ended/BamHI pHK413 were annealed in a 5:1 molar ratio and ligated using T4 DNA ligase; thus forming pCP400. Sequence data indicated that a b.p. of the HindII restriction site was lost during this process.

NUCLEOTIDE SEQUENCING

The nucleotide sequence of portions of each DNA strand of a segment of the CPV DNA sequence of pCP200 was determined by methods previously described in Section 6.4.3. The DNA sequence extends for 722 b.p. and ends near the PvuII site at the 5'-end of the CPV genome (see FIGS. 19 and 20).

## TRANSFORMATION OF E. COLI AND EXPRESSION OF FUSION PROTEINS

All pCP recombinant plasmids were used to transform *E. coli* strain NF1829. Plasmid DNA was isolated from the resultant ampicillin resistant transformants, and CPV clones were identified by restriction mapping. The clones were streaked on indicator plates to determine whether or not under induced conditions ß-galactosidase activity, *i.e.*, fusion protein production, could be detected (Miller, 1972, Experiments in Molecular Genetics, Cold Spring Harbor Press, New York, N.Y.).

Ampicillin resistant transformants containing pCP100, pCP110, pCP200, pCP300, pCP310, and pCP400 were identified by restriction mapping; and pCP300, pCP310, and pCP400 were screened for the production of the CPV/ß-galactosidase fusion protein. Two approaches were routinely used to further analyze fusion proteins produced: direct analysis of total *E. coli* proteins by SDS-PAGE and SDS-PAGE analysis of radiolabeled *E. coli* proteins which are immunoprecipitated by convalscent canine antisera. Accordingly, pCP300, pCP310 and pCP400 were found to produce fusion proteins.

## ANALYSIS OF TOTAL E. COLI PROTEINS

The pCP300, pCP310, and pCP400 transformants were cultured under induced and uninduced conditions and cell lysates were prepared for SDS-PAGE as previously described in Section 6.6.1.

Transformants containing pCP300, pCP310, and pCP400 each produce a fusion protein with a molecular weight of 135,000 daltons, 125,000 daltons and 145,000

0117767

daltons respectively (data not shown). As described previously, other fusion proteins were also present.

ANALYSIS OF IMMUNOREACTIVE E. COLI PROTEINS

The pCP300 transformants were then analyzed by immunoprecipitation of $^{35}$S-methionine radiolabeled E. coli polypeptides using convalescent canine antisera (i.e., antisera directed against antigenic determinants of CPV). Immunoprecipitation and SDS-PAGE of expressed polypeptides in pCP300 E. coli transformants was accomplished by methods described previously in Section 6.6.2.

A fluorogram demonstrating the results of the SDS-PAGE analysis of immunoprecipitated proteins was made (data not shown). Lysates of uninduced pCP300 transformants, induced pCP300 transformants, induced pHK413 transformants, and induced pDN520 transformants were run on SDS-PAGE [N.B., pDN520 is a pHK413 derivative wherein the coding sequence for the z-gene is in phase with the plasmid cro ATG.]

The lysates of these transformants were immunoprecipitated with convalescent canine serum, or normal canine serum, or rabbit antiserum directed against the B protein of PPV. The results clearly demonstrated that induced transformants containing pCP300 produced a fusion protein that was immunoprecipitated with antisera directed against CPV and with antisera directed against PPV. This cross-immunoreactivity demonstrated by the CPV/ß-galactosidase fusion protein for both covalescent canine sera (i.e., anti-CPV sera) and for anti-PPV sera indicates the possibility of using the CPV fusion protein in a vaccine formulation to protect the recipient against either CPV or PPV infection.

## IMMUNIZATION OF LABORATORY ANIMALS WITH PREPARATIONS OF CPV/ß-GALACTOSIDASE FUSION PROTEINS

Purified CPV/ß-galactosidase fusion proteins were used to immunize rabbits according to the protocol described below. Fusion proteins produced by host cells transformed with pCP300 were gel purified using methods described in Section 6.1.5. except that the fusion protein bands were not localized in the gels by dansylation. Rather, strips were sliced from each side of the gel and stained with Coomassie Brilliant Blue. Alignment of these strips to the gel allowed localization of the fusion protein band within the remainder of the unstained gel. The accuracy of excision was established by staining the remainder of the gel. The gel slice containing the fusion protein was processed for injection by repeated passage through an 18 gauge needle and suspension in an appropriate volume of PBS (Lane and Roberts, 1978, Virology 87:182). Three rabbits were immunized with fusion proteins derived from pCP300 transformants prepared as described above. Rabbits were pre-bled and then injected (subcutaneously in the back) with 250 µg fusion protein mixed in a 1:1 ratio with Freund's complete adjuvant. After 21 days the rabbits were boosted with 50 µg fusion protein mixed in a 1:1 ratio with Freund's incomplete adjuvant. After a total of 27 days the rabbits were bled (designated as 1st bleed). The rabbits were boosted a total of five times at weekly intervals; rabbits were bled six days after each boost.

The specificity of the antisera was analyzed by the ability of the antisera to specifically immunoprecipitate proteins from lysates of tissue culture cells infected with CPV. The method used is similar to the method employed in Section 6.8.1. except that Feline Kidney Cells were used as the cell line. Results of these tests demonstrated that two out of three rabbit antisera directed against the CPV/ß-galactosidase fusion protein produced by pCP300 transformants specifically immunoprecipitated a protein of approximately 63,000 dalton from CPV-infected cell lysates.

The foregoing results indicate that bacterially produced CPV/ß-galactosidase fusion protein possesses antigenic determinants capable of being recognized by convalescent canine antisera containing virus neutralizing antibodies to CPV, and further demonstrate that these fusion proteins are able to elicit a CPV-specific immune response in animals when such proteins are used as immunogens.

### CONSTRUCTION OF pCP450A WHICH PRODUCES BOTH cro/CPV AND cro/PPV/ß-GALACTOSIDASE FUSION PROTEIN

A recombinant plasmid pCP450A was constructed which can direct the production of both a fused and an unfused CPV protein in appropriate host cells. (N.B. Both proteins were fused to cro sequences.) Details of the procedure are described below.

pCP322

The plasmid pCPV-2 (described supra) was digested with PstI and treated with the Klenow fragment of DNA polymerase I in order to remove the single stranded end at the PstI site. The plasmid was then digested with BglII and the 1.4 kb BglII/PstI fragment was gel purified (See FIG. 21). The expression vector pHK413 was digested with BglII and SmaI and the 6.5 kb SmaI/BglII cleaved vector was gel purified.

The 1.4 kb BglII/PstI fragment and the 6.5 kb SmaI/BglII fragment were annealed in a 5:1 molar ratio and ligated using T4 DNA ligase. As a result of this process, the BglII site was restored, however, the PstI and SmaI sites were destroyed.

pCP422

The recombinant plasmid pCP400 (described *supra*) was partially digested with BglII. The plasmid was then digested with PstI. The resulting 1.3 kb PstI/BglII fragment was purified by gel electrophoresis. The plasmid pCP322 was digested with BglII and PstI and the 6.9 kb fragment was gel purified. The 1.3 kb PstI/BglII fragment and the 6.9 kb PstI/BglII fragment were annealed in a 5:1 molar ratio and ligated using T4 DNA ligase. As a result of this process, the PstI and BglII sites were restored (see FIG. 22).

pCP450

The construction of this plasmid is depicted in FIG. 23. Plasmid pCP422 was digested with BamHI and treated with BAL 31 in order to remove approximately 200-300 b.p. The plasmid was then digested with PstI and DNA fragments of approximately 2300-2400 b.p. were gel purified. The expression plasmid pHK414 was digested with BglII, filled-in with the Klenow fragment of DNA polymerase I, and then digested with PstI. The 5.5 kb DNA fragment was gel purified. The 2.4 kb fragment and the 5.5 kb fragment were annealed in 5:1 molar ratio and ligated with T4 DNA ligase. The ligation mixture was used to transform E. coli NF1829. Transformants were screened for the ability to form red colonies on MacConkey agar plates. One transformant, pCP450, produced a CPV/ß-galactosidase fusion protein.

pCP450A

In order to introduce an amber chain termination sequence between the CPV sequence and the z-gene of the pCP450 plasmid, pCP450 was cleaved with HindIII followed by BamHI resulting in cleavage of the plasmid at its unique restriction endonuclease site located at the junction between the CPV gene and the z-gene. DNA linkers which have HindIII/BamHI cohesive ends, an internal XbaI site, and an amber sequence (TAG) were annealed in a 10:1 molar ratio to the linearized plasmid and ligated using T4 DNA ligase (see FIG. 24). The complementary strands of the DNA linker were synthesized by the solid phase method described supra (section 6.9.). As a result of this process, the BamHI site was restored, however, the HindIII site was destroyed.

After ligation of the linker to the cleaved plasmid, the resulting recombinant plasmids were cleaved with XbaI, the linear DNA fragments were isolated by agarose gel electrophoresis and recircularized by ligation with T4 DNA ligase (this process selected for the pCP450 plasmids that contained the linker sequence ligated between the HindIII and BamHI sites at the CPV/z junction and a single XbaI site on the plasmid. As a result of ligation, the amber sequence of the DNA linker was in phase with the translational reading frame of CPV and the z-gene. The resulting plasmid was designated pCP450A.

## pCP450A TRANSFORMANTS

The pCP450A plasmid was used to transform *E. coli* NF1829. When induced with lactose, the ampicillin transformants synthesized no detectable levels of fusion protein (*i.e.*, no red colonies on MacConkey indicator agar plates). The transformants were then infected with the lysogenic transducing phage, Ø80SuIII, which carries an amber suppressor tRNA gene. The lysogenized transformants produced pink colonies on MacConkey agar plates, indicating fusion protein production.

The pCP450A plasmid was also used to transform *E. coli* LE392 which carries two amber suppressor (supE and supF) but does not have the *laci*$^q$ mutation of NF1829 and therefore does not oversynthesize *lac* repressor. These transformants produced an unfused protein and a fusion protein in the presence or absence of the inducer IPTG or lactose.

## IDENTIFICATION OF TRANSFORMANTS THAT PRODUCE FUSION AND UNFUSED PROTEINS

Either of the mini-aggregate procedures (as described in Section 6.9.2.) were used to isolate aggregate proteins for screening analysis.

The proteins produced were of the size predicted for fusion (approximately 170,000 daltons) and unfused (approximately 49,500 daltons) proteins.

## CHARACTERIZATION OF IMMUNE RESPONSE OF RABBITS TO FORMALIN TREATED AGGREGATE CPV FUSION PROTEIN

To characterize the immune response of rabbits to CPV fusion protein, rabbits were injected with 2 doses of 15-20 mg of CPV bacterially derived proteins expressed by *E. coli* LE392/pCP450A on day 0 and 28. Each dose contained 5 mg of cro/CPV protein and 12.5 to 15 mg of the cro/CPV/ß-galactosidase fusion protein. The aggregate protein (isolated by the modification of the aggregate purification procedure described *supra* in Section 5.5.) was suspended in water, treated with formalin at room temperature or 4°C for 6-18 hours, mixed with adjuvant either immediately or several hours prior to injection. Aluminum hydroxide gel (5 mg of $Al(OH)_3$ was used per dose) was added to stimulate the immune system rather than to bind protein. As a control, animals were injected with untreated bacterially derived proteins mixed with adjuvant (5 mg of $Al(OH)_3$).

Antisera were analyzed for its ability to immunoprecipitate (IP) viral proteins from radiolabeled infected cell lysates. Results are reported below in Table 4 as the number of animals positive/total number injected.

TABLE 4

IMMUNE RESPONSE OF RABBITS TO FORMALIN TREATED AGGREGATE CPV BACTERIAL DERIVED FUSION PROTEIN

| Immunogen[1] | Final Concentration Formalin (%) | Adjuvant | Characterization of Immune Response IP |
|---|---|---|---|
| cro/CPV and cro/CPV/ß-galactosidase | 0.0 | $Al(OH)_3$gel | 1/3[1] |
| cro/CPV and cro/CPV/ß-galactosidase | 0.2 | $Al(OH)_3$gel | 3/3[1] |

[1] Antisera were drawn 1 week post-boost.

To characterize the immune response of rabbits to formalin treated aggregate CPV bacterial derived fusion protein, the antisera were used to immunoprecipitate $^{35}$S-methionine radiolabeled CPV infected ST cell lysates as described supra in Section 6.8.1.

Samples were electrophoresed in SDS polyacrylamide gels (10%), and the gels were prepared for fluorography as described supra. Rabbit antisera to formalin treated aggregate CPV bacterial derived fusion protein immunoprecipitated the CPV structural genes in all cases whereas the CPV structural protein was immunoprecipitated by 1 out of 3 rabbits which did not receive formalin treated CPV protein.

## CONSTRUCTION OF ptac CLONES

Tac promoter/operator region vectors were constructed as described in Section 6.10 for PPV.

### pCP450tc

Plasmid p830 (described supra) was digested with PstI and BglII and the approximately 1.3 kb fragment was isolated by agarose gel electrophoresis. Plasmid pCP450 was digested with PstI and BglII and the approximately 6.5 kb fragment containing lacz sequences was isolated by gel electrophoresis (See FIG. 25). The 1.3 kb fragment and the 6.5 kb fragment were annealed in a 5:1 molar ratio, ligated with T4 DNA ligase, and used to transform E. coli NF1829. A clone p881 (which lacks the PPV sequences) was selected.

Plasmid p881 was then digested with BglII and treated with calf intestinal phosphatase and electrophoresed in low melting point (LMP) agarose to purify the linearized plasmid. Plasmid pCP450 was digested with BglII and an approximately 300 b.p. BglII/BglII fragment was isolated and purified by gel electrophoresis in LMP agarose as shown in FIG. 26. The 300 kb fragment and the 7.8 kb fragment were annealed in a 5:1 molar ratio and ligated with T4 DNA ligase. The ligation mixture was used to transform E. coli NF1829. Since selection of orientation of the BglII insert was not possible, a transformant which contained the BglII insert in the correct orientation and thus produced CPV protein was identified only after induction with lactose and protein analyses by SDS-PAGE. Plasmid pCP450tc was found to express the identical proteins as pCP450 but in greater quantity (an increase of 5-fold).

pCP450Atc

In order to introduce the tac promoter fragment into pCP450A, plasmid pCP450tc was first digested with PstI and partially digested with BglII. An approximately 1350 b.p. fragment containing the promoter and an internal BglII site was annealed and ligated to a BglII/PstI fragment of pCP450A which carried the lacz sequences of the expression vector (see FIG. 27). E. coli strain F' LE392 (described supra) was transformed with the ligation mixture and clone pCP450Atc was selected by plating on LB agar plates containing 100 µg/mℓ ampicillin. The clone containing pCP450Atc produced cro/CPV and cro/CPV/ß-galactosidase proteins in the presence of inducer.

The pCP450Atc plasmid was also used to transform E. coli NF1829. When induced with 1% lactose, the ampicillin resistant transformants synthesized an approximately 49,500 dalton cro/CPV protein. Proteins were identified after isolation by the mini-aggregate procedure (described supra in Section 6.9.2.) or by the modification of the aggregate purification procedure described in Section 5.5. supra) and analysis by SDS-PAGE.

pCP450AtcF3

An approximately 625 b.p. CPV fragment was isolated by digestion of pCP450Atc with PvuII (which generates blunt ends) and BamHI followed by gel purification (See FIG. 28). A second preparation of pCP450Atc was digested with BglII, filled-in with the Klenow fragment of DNA polymerase I, and then digested with BamHI. The 6.8 kb ptac containing fragment was isolated by gel electrophoresis and annealed and ligated to the 625 b.p. CPV fragment. E. coli strain F' LE392 was transformed with the ligation mixture and clone pCP450AtcF3 was selected as described supra. Transformants produced proteins with molecular weights of 143,000 daltons and 25,000 daltons corresponding to the predicted molecular weight for cro/CPV/ß-galactosidase and cro/CPV proteins.

## DEPOSIT OF MICROORGANISMS

It is to be understood that all base pair sizes given for nucleotide sequences are approximate and are used for purposes of description. While the nucleotide sequences reported herein are believed to be accurate, any changes which do not significantly affect the processes or activity of the products described herein are intended to be included within the scope of the claimed invention. Furthermore, it is apparent that many modifications and variations of this invention as hereinbefore set forth may be made without departing from the spirit and scope thereof. The specific embodiments described are given by way of example only and the invention is limited only by the appended claims.

The following *E. coli* strains carrying the listed plasmids have been deposited with the Agricultural Research Culture Collection (NRRL), Peoria, IL and have been assigned the listed accession numbers:

| | E. coli Strain | Plasmid | Accession Numbers |
|---|---|---|---|
| (1) | K-12, HB101 | pPPV-6 | B-15226 |
| (2) | K-12, MC1000 | pCPV-2 | B-15227 |
| (3) | K-12, MC1000 | pCPV-9 | B-15257 |
| (4) | K-12, NF1829 | pPP406 | B-15228 |
| (5) | K-12, NF1829 | pCP100 | B-15229 |
| (6) | K-12, NF1829 | pCP300 | B-15230 |
| (7) | K-12, NF1829 | pPP450 | B-15729 |
| (8) | K-12, F'LE392 | pPP450Atc | B-15731 |
| (9) | K-12, NF1829 | pCP450 | B-15730 |
| (10) | K-12, F'LE392 | pCP450Atc | B-15732 |

A culture of the deposited microorganisms will be made available to the public upon the grant of patent based upon the present application. It is to be understood that the availability of a deposit does not constitute a license to practice the subject invention in derogation of patent rights granted by the United States government. Furthermore, the present invention is not to be limited in scope by the microorganism deposited, since the deposited embodiment is intended as a single illustration of one aspect of the invention. Indeed, various modifications of the invention in addition to those shown and described herein will become apparent to those skilled in the art from the foregoing description and accompanying drawings. Such modifications are intended to fall within the scope of the appended claims.

WE CLAIM:

1. A process for producing a polypeptide having an immunoreactive and antigenic determinant of a Parvovirus protein, comprising:

(a) culturing a unicellular organism containing a recombinant vector comprising a DNA sequence coding for a polypeptide having an immunoreactive and antigenic determinant of a Parvovirus protein and capable of being replicated, transcribed and translated in said unicellular organism; and

(b) isolating said polypeptide from the culture.

2. The process according to claim 1, wherein said polypeptide is immunogenic.

3. The process according to claim 1, wherein the recombinant vector was introduced into the unicellular organism by transformation.

4. The process according to claim 1, wherein the recombinant vector was introduced into the unicellular organism by transduction.

5. The process according to claim 1, wherein the recombinant vector was introduced into the unicellular organism by transfection.

6. The process according to claim 1, wherein the information contained in the DNA sequence is obtained from a Parvovirus genome.

7. The process according to claim 6, wherein the information contained in the DNA sequence is obtained from Porcine Parvovirus.

8. The process according to claim 6, wherein the information contained in the DNA sequence is obtained from Canine Parvovirus.

9. The process according to claim 1, wherein the information contained in the DNA sequence is obtained by isolating mRNA coding for a Parvovirus protein and using reverse transcriptase to construct said DNA sequence having one strand complementary to the isolated mRNA.

10. The process according to claim 1, wherein the DNA sequence is obtained from a DNA vector containing a Parvovirus DNA sequence.

11. The process according to claim 1, wherein the DNA sequence codes for a structural protein of a Parvovirus.

12. The process according to claim 1, wherein the DNA sequence codes for a structural protein of a Porcine Parvovirus.

13. The process according to claim 1, wherein the DNA sequence codes for a structural protein of a Canine Parvovirus.

14. The process according to claim 1, wherein the unicellular organism is a eucaryotic organism.

15. The process according to claim 1, wherein the unicellular organism is a procaryotic organism.

16. The process according to claim 15, wherein the procaryotic organism is _Escherichia coli_.

17. The process according to claim 16, wherein the _Escherichia coli_ has accession NRRL No. B-15226.

18. The process according to claim 16, wherein the _Escherichia coli_ has accession NRRL No. B-15227.

19. The process according to claim 16, wherein the _Escherichia coli_ has accession NRRL No. B-15257.

20. The process according to claim 16, wherein the _Escherichia coli_ has accession NRRL No. B-15228.

21. The process according to claim 16, wherein the _Escherichia coli_ has accession NRRL No. B-15229.

22. The process according to claim 16, wherein the _Escherichia coli_ has accession NRRL No. B-15230.

23. The process according to claim 16, wherein the _Escherichia coli_ has accession NRRL No. B-15729.

24. The process according to claim 16, wherein the _Escherichia coli_ has accession NRRL No. B-15731.

25. The process according to claim 16, wherein the _Escherichia coli_ has accession NRRL No. B-15730.

26. The process according to claim 16, wherein the _Escherichia coli_ has accession NRRL No. B-15732.

27. A process for preparing a unicellular organism having a DNA sequence coding for a polypeptide having an immunoreactive and antigenic determinant of a Parvovirus protein, comprising: introducing a recombinant vector into a unicellular organism, said recombinant vector comprising a DNA sequence coding for a polypeptide having an immunoreactive and antigenic determinant of a Parvovirus protein and capable of being replicated, transcribed and translated in the unicellular organism.

28. A recombinant DNA vector, comprising: a DNA sequence coding for an immunoreactive and antigenic determinant of a Parvovirus protein.

29. The recombinant DNA vector according to claim 28, wherein the Parvovirus is Porcine Parvovirus.

30. The recombinant DNA vector according to claim 28 wherein the DNA sequence codes for a Porcine Parvovirus structural protein as depicted in FIG. 3, or any portion thereof.

31. The recombinant DNA vector according to claim 30 wherein the vector is pPPV-6, or a mutant, recombinant, or genetically engineered derivative thereof.

32. The recombinant DNA vector according to claim 29, wherein said DNA sequence is under the control of expression control elements.

33. The recombinant DNA vector according to claim 32 wherein the vector is pPP406, or a mutant, recombinant, or genetically engineered derivative thereof.

34. The recombinant DNA vector according to claim 29, further comprising the essential portions of the pBR322 replicon.

35. The recombinant DNA vector according to claim 29, wherein said DNA sequence is connected in the correct translational reading frame to a second DNA sequence coding for a protein.

36. The recombinant DNA vector according to claim 35 wherein the vector is pPP450 or a mutant, recombinant, or genetically engineered derivative thereof.

37. The recombinant DNA vector according to claim 35 wherein a chain termination signal is located between said first and second DNA sequences in the correct translational reading frame.

38. The recombinant DNA vector according to claim 37, wherein the vector is pPP450A, or a mutant, recombinant, or genetically engineered derivative thereof.

39. The recombinant DNA vector of claim 37 wherein the vector is pPP450Atc, or a mutant, recombinant or genetically engineered derivative thereof.

40. A unicellular organism containing the recombinant DNA vector of claim 28.

41. A unicellular organism containing the recombinant DNA vector of claim 29.

42. A unicellular organism containing the recombinant DNA vector of claim 30.

43. A unicellular organism containing the recombinant DNA vector of claim 32.

0117767

44. A unicellular organism containing the recombinant DNA vector of claim 34.

45. A unicellular organism containing the recombinant DNA vector of claim 35.

46. A unicellular organism containing the recombinant DNA vector of claim 37.

47. A unicellular organism containing the recombinant vector of claim 37 and a corresponding chain terminator suppressor tRNA gene.

48. An _Escherichia coli_ bacterium containing the recombinant DNA vector of claim 28.

49. An _Escherichia coli_ bacterium containing the recombinant DNA vector of claim 29.

50. An _Escherichia coli_ bacterium containing the recombinant DNA vector of claim 30.

51. An _Escherichia coli_ bacterium containing the recombinant DNA vector of claim 32.

52. An _Escherichia coli_ bacterium containing the recombinant DNA vector of claim 34.

53. An _Escherichia coli_ bacterium containing the recombinant DNA vector of claim 35.

54. An _Escherichia coli_ bacterium containing the recombinant DNA vector of claim 37.

55. An *Escherichia coli* bacterium containing the recombinant vector of claim 37 and a corresponding chain terminator suppressor tRNA gene.

56. An *Escherichia coli* bacterium of claim 49 deposited with the NRRL and assigned NRRL accession No. B-15226, and mutants, recombinants, and genetically engineered derivatives thereof.

57. An *Escherichia coli* bacterium of claim 51 deposited with the NRRL and assigned NRRL accession No. B-15228, and mutants, recombinants, and genetically engineered derivatives thereof.

58. An *Escherichia coli* of claim 53 deposited with the NRRL and assigned NRRL accession No. B-15729, and mutants, recombinants, and genetically engineered derivates thereof.

59. An *Escherichia coli* of claim 55 deposited with the NRRL and assigned NRRL accession No. B-15731, and mutants, recombinants, and genetically engineered derivates thereof.

60. The recombinant DNA vector according to claim 28, wherein the Parvovirus is Canine Parvovirus.

61. The recombinant DNA vector according to claim 28, wherein the DNA sequence codes for a Canine Parvovirus structural protein as depicted in FIG. 20, or any portion thereof.

62. The recombinant DNA vector according to claim 61 wherein the vector is pCPV-2, or a mutant, recombinant, or genetically engineered derivative thereof.

0117767

63. The recombinant DNA vector according to claim 61 wherein the vector is pCPV-9, or a mutant, recombinant, or genetically engineered derivative thereof.

64. The recombinant DNA vector according to claim 60, wherein said DNA sequence is under the control of expression control elements.

65. The recombinant DNA vector according to claim 64 wherein the vector is pCP100, or a mutant, recombinant, or genetically engineered derivative thereof.

66. The recombinant DNA vector according to claim 64 wherein the vector is pCP300, or a mutant, recombinant, or genetically engineered derivative thereof.

67. The recombinant DNA vector according to claim 60, further comprising the essential portions of the pBR322 replicon.

68. The recombinant DNA vector according to claim 60, wherein said DNA sequence is connected in the correct translational reading frame to a second DNA sequence coding for a protein.

69. The recombinant DNA vector according to claim 68 wherein the vector is pCP450, or a mutant, recombinant or genetically engineered derivative thereof.

70. The recombinant DNA vector according to claim 68 wherein a chain termination signal is located between said first and second DNA sequences in the correct translational reading frame.

71. The recombinant DNA vector according to claim 70 wherein the vector is pCP450A, or a mutant, recombinant, or genetically engineered derivative thereof.

72. The recombinant DNA vector according to claim 70 wherein the vector is pCP450Atc, or a mutant, recombinant, or genetically engineered derivative thereof.

73. A unicellular organism containing the recombinant DNA vector of claim 60.

74. A unicellular organism containing the recombinant DNA vector of claim 61.

75. A unicellular organism containing the recombinant DNA vector of claim 64.

76. A unicellular organism containing the recombinant DNA vector of claim 67.

77. A unicellular organism containing the recombinant DNA vector of claim 68.

78. A unicellular organism containing the recombinant DNA vector of claim 70.

79. A unicellular organism containing the recombinant DNA vector of claim 70 and a corresponding chain terminator suppressor tRNA gene.

80. An *Escherichia coli* bacterium containing the recombinant DNA vector of claim 60.

81. An *Escherichia coli* bacterium containing the recombinant DNA vector of claim 61.

82. A unicellular organism containing the recombinant DNA vector of claim 64.

83. A unicellular organism containing the recombinant DNA vector of claim 67.

84. A unicellular organism containing the recombinant DNA vector of claim 68.

85. A unicellular organism containing the recombinant DNA vector of claim 70.

86. An _Escherichia_ _coli_ bacterium containing the recombinant vector of claim 70 and a corresponding chain terminator suppressor tRNA gene.

87. An _Escherichia_ _coli_ bacterium of claim 80 deposited with the NRRL and assigned NRRL accession No. B-15227, and mutants, recombinants and genetically engineered derivatives thereof.

88. An _Escherichia_ _coli_ bacterium of claim 80, deposited with the NRRL and assigned NRRL accession No. B-15257, and mutants, recombinants, and genetically engineered derivatives thereof.

89. An _Escherichia_ _coli_ bacterium of claim 82 deposited with the NRRL and assigned NRRL accession No. B-15229, and mutants, recombinants, and genetically engineered derivatives thereof.

90. An _Escherichia_ _coli_ bacterium of claim 82 deposited with the NRRL and assigned NRRL accession No. B-15230, and mutants, recombinants, and genetically engineered derivatives thereof.

91. An <u>Escherichia</u> <u>coli</u> bacterium of claim 84 deposited with the NRRL and assigned NRRL accession No. B-15730, and mutants, recombinants, and genetically engineered derivatives thereof.

92. An <u>Escherichia</u> <u>coli</u> bacterium of claim 86 deposited with the NRRL and assigned NRRL accession No. B-15732, and mutants, recombinants, and genetically engineered derivatives thereof.

93. A polypeptide having an immunoreactive and antigenic determinant of a parvovirus protein produced by <u>Escherichia</u> <u>coli</u> of claim 57.

94. A polypeptide having an immunoreactive and antigenic determinant of a parvovirus protein produced by <u>Escherichia</u> <u>coli</u> of claim 58.

95. A polypeptide having an immunoreactive and antigenic determinant of a parvovirus protein produced by <u>Escherichia</u> <u>coli</u> of claim 59.

96. A polypeptide having an immunoreactive and antigenic determinant of a parvovirus protein produced by <u>Escherichia</u> <u>coli</u> of claim 90.

97. A polypeptide having an immunoreactive and antigenic determinant of a parvovirus protein produced by <u>Escherichia</u> <u>coli</u> of claim 91.

98. A polypeptide having an immunoreactive and antigenic determinant of a parvovirus protein produced by <u>Escherichia</u> <u>coli</u> of claim 92

0117767

99. A polypeptide produced according to the process of claim 2.

100. A polypeptide of claim 99 which, when used as an immunogen, is capable of eliciting antibodies that neutralize virus infectivity.

101. A vaccine formulation comprising the polypeptide of claim 100 and a compatible pharmaceutical carrier therefor.

FIG. 1

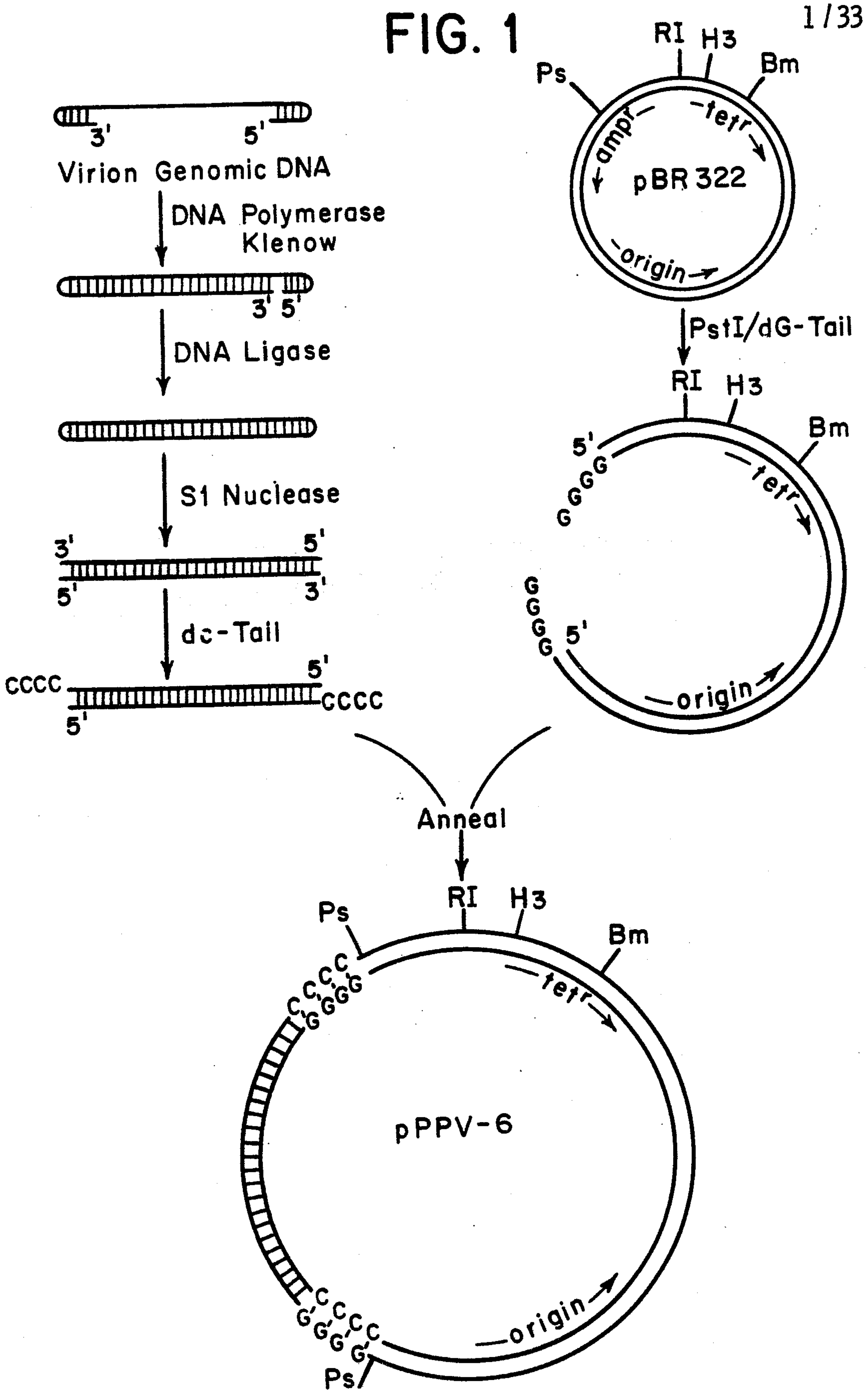

3'
5'
Virion Genomic DNA
DNA Polymerase Klenow
3' 5'
DNA Ligase
S1 Nuclease
3'
5'
5'
3'
dc-Tail
CCCC
5'
CCCC
5'
RI
H3
Bm
Ps
ampr
tetr
pBR 322
origin
PstI/dG-Tail
RI
H3
Bm
5'
GGGG
tetr
GGGG
5'
origin
Anneal
RI
H3
Ps
Bm
CCCC
GGGG
tetr
pPPV-6
CCCC
GGGG
origin
Ps

FIG.2A

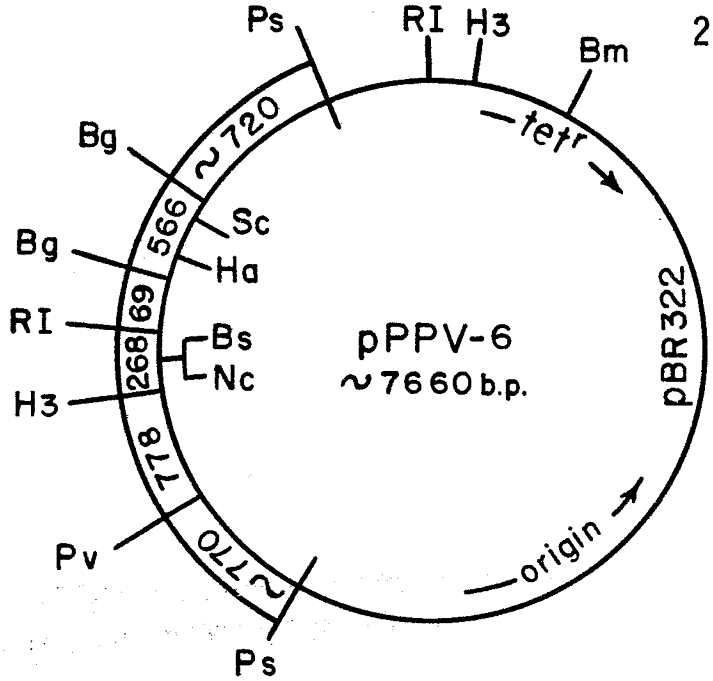
Ps
RI
H3
Bm
tetr
Bg
~720
566
Sc
Bg
Ha
69
RI
Bs
268
Nc
H3
pPPV-6
~7660 b.p.
pBR322
778
Pv
~770
origin
Ps

FIG.2B

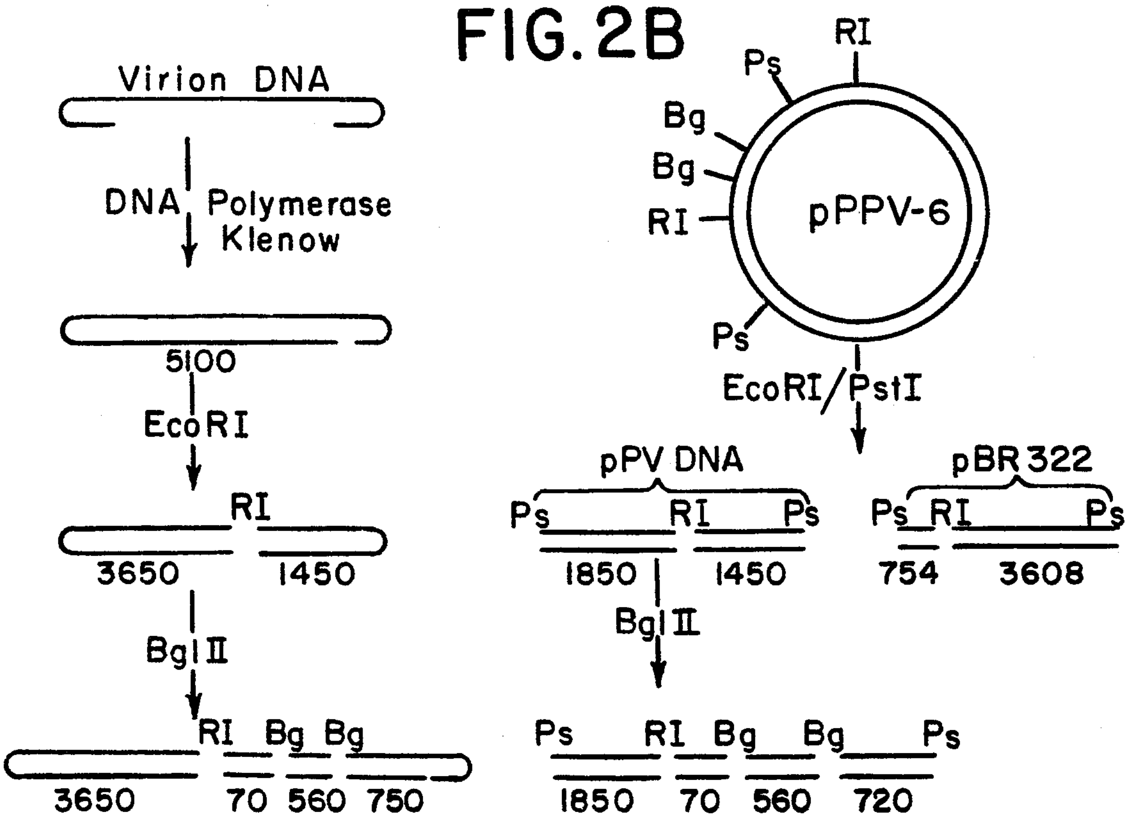
Virion DNA
DNA Polymerase
Klenow
5100
EcoRI
RI
3650
1450
BglII
RI Bg Bg
3650
70 560 750
Ps
RI
Bg
Bg
RI
pPPV-6
Ps
EcoRI/PstI
pPV DNA
Ps RI Ps
1850
1450
pBR322
Ps RI Ps
754
3608
BglII
Ps RI Bg Bg Ps
1850 70 560 720

# FIG. 3



PvuII
CT

GAT GAA AAA TTC ATA AAA GAA ACT GAA CAC GCA AAA GAC TAC GGA GGT AAA ATT GGA CAT 62
1 Asp Glu Lys Phe Ile Lys Glu Thr Glu His Ala Lys Asp Tyr Gly Gly Lys Ile Gly His

TAC TTC TTC AGA GCA AAG CGT GCC TTT GCT CCA AAA CTC TCA GAA ACA GAC TCA CCA ACT 122
21 Tyr Phe Phe Arg Ala Lys Arg Ala Phe Ala Pro Lys Leu Ser Glu Thr Asp Ser Pro Thr

ACA TCT CAA CAA CCA GAG GTA AGA AGA TCG CCG AGA AAA CAC CCA GGG TCT AAA CCA CCA 182
41 Thr Ser Gln Gln Pro Glu Val Arg Arg Ser Pro Arg Lys His Pro Gly Ser Lys Pro Pro

GGA AAA AGA CCT GCT CCA AGA CAT ATT TTT ATA AAC TTA GCT AAA AAA AAA GCT AAA GGG 242
61 Gly Lys Arg Pro Ala Pro Arg His Ile Phe Ile Asn Leu Ala Lys Lys Lys Ala Lys Gly

ACA TCT AAT ACA AAC TCT AAC TCA ATG AGT GAA AAT GTG GAA CAA CAC AAC CCT ATT AAT 302
81 Thr Ser Asn Thr Asn Ser Asn Ser Met Ser Glu Asn Val Glu Gln His Asn Pro Ile Asn

GCA GGC ACT GAA TTG TCT GCA ACA GGA AAT GAA TCT GGG GGT GGG GGC GGC GGT GGC GGG 362
101 Ala Gly Thr Glu Leu Ser Ala Thr Gly Asn Glu Ser Gly Gly Gly Gly Gly Gly Gly Gly

AccI
GGT AGG GGT GCT GGG GGG GTT GGT GTG TCT ACA GGT AGT TTC AAT AAT CAA ACA GAA TTT 422
121 Gly Arg Gly Ala Gly Gly Val Gly Val Ser Thr Gly Ser Phe Asn Asn Gln Thr Glu Phe

CAA TAC TTG GGG GAG GGC TTG GTT AGA ATC ACT GCA CAC GCA TCA AGA CTC ATA CAT CTA 482
141 Gln Tyr Leu Gly Glu Gly Leu Val Arg Ile Thr Ala His Ala Ser Arg Leu Ile His Leu

RsaI
AAT ATG CCA GAA CAC GAA ACA TAC AAA AGA ATA CAT GTA CTA AAT TCA GAA TCA GGG GTG 542
161 Asn Met Pro Glu His Glu Thr Tyr Lys Arg Ile His Val Leu Asn Ser Glu Ser Gly Val

RsaI
GGG GGA CAA ATG GTA CAA GAC GAT GCA CAC ACA CAA ATG GTA ACA CCT TGG TCA CTA ATA 602
181 Gly Gly Gln Met Val Gln Asp Asp Ala His Thr Gln Met Val Thr Pro Trp Ser Leu Ile

GAT GCT AAC GCA TGG GGA GTG TGG TTC AAT CCA GCG GAC TGG CAG TTA ATA TCC AAC AAC 662
201 Asp Ala Asn Ala Trp Gly Val Trp Phe Asn Pro Ala Asp Trp Gln Leu Ile Ser Asn Asn

RsaI
ATG ACA GAA ATA AAC TTA GTT AGT TTT GAA CAA GCA ATA TTC AAT GTA GTA CTT AAA ACA 722
221 Met Thr Glu Ile Asn Leu Val Ser Phe Glu Gln Ala Ile Phe Asn Val Val Leu Lys Thr

HindIII
ATT ACA GAA TCA GCA ACC TCA CCA CCA ACC AAA ATA TAT AAT AAT GAT CTA ACT GCA AGC 782
241 Ile Thr Glu Ser Ala Thr Ser Pro Pro Thr Lys Ile Tyr Asn Asn Asp Leu Thr Ala Ser

TTA ATG GTC GCA CTA GAC ACC AAT AAC ACA CTT CCA TAC ACA CCA GCA GCA CCT AGA AGT 842
261 Leu Met Val Ala Leu Asp Thr Asn Asn Thr Leu Pro Tyr Thr Pro Ala Ala Pro Arg Ser

NcoI BstEII
GAA ACA CTT GGT TTT TAT CCA TGG TTA CCT ACA AAA CCA ACT CAA TAC AGA TAT TAC CTA 902
281 Glu Thr Leu Gly Phe Tyr Pro Trp Leu Pro Thr Lys Pro Thr Gln Tyr Arg Tyr Tyr Leu

HinfI
TCA TGC ATC AGA AAC CTA AAT CCA CCA ACA TAC ACT GGA CAA TCA CAA CAA ATA ACA GAC 962
301 Ser Cys Ile Arg Asn Leu Asn Pro Pro Thr Tyr Thr Gly Gln Ser Gln Gln Ile Thr Asp

RsaI
TCA ATA CAA ACA GGA CTA CAC AGT GAC ATT ATG TTC TAC ACA ATA GAA AAT GCA GTA CCA 1022
321 Ser Ile Gln Thr Gly Leu His Ser Asp Ile Met Phe Tyr Thr Ile Glu Asn Ala Val Pro

# FIG. 3A

EcoRI
ATT CAT CTT CTA AGA ACA GGA GAT GAA TTC TCC ACA GGA ATA TAT CAC TTT GAC ACA AAA 1082
341 Ile His Leu Leu Arg Thr Gly Asp Glu Phe Ser Thr Gly Ile Tyr His Phe Asp Thr Lys

BglII
CCA CTA AAA TTA ACT CAC TCA TGG CAA ACA AAC AGA TCT CTA GGA CTG CCT CCA AAA CTA 1142
361 Pro Leu Lys Leu Thr His Ser Trp Gln Thr Asn Arg Ser Leu Gly Leu Pro Pro Lys Leu

CTA ACT GAA CCT ACC ACA GAA GGA GAC CAA CAC CCA GGA ACA CTA CCA GCA GCT AAC ACA 1202
381 Leu Thr Glu Pro Thr Thr Glu Gly Asp Gln His Pro Gly Thr Leu Pro Ala Ala Asn Thr

HaeIII
AGA AAA GGT TAT CAC CAA ACA ATT AAT AAT AGC TAC ACA GAA GCA ACA GCA ATT AGG CCA 1262
401 Arg Lys Gly Tyr His Gln Thr Ile Asn Asn Ser Tyr Thr Glu Ala Thr Ala Ile Arg Pro

GCT CAG GTA GGA TAT AAT ACA CCA TAC ATG AAT TTT GAA TAC TCC AAT GGT GGA CCA TTT 1322
421 Ala Gln Val Gly Tyr Asn Thr Pro Tyr Met Asn Phe Glu Tyr Ser Asn Gly Gly Pro Phe

CTA ACT CCT ATA GTA CCA ACA GCA GAC ACA CAA TAT TAT GAT GAT GAA CCA AAT GGT GCT 1382
441 Leu Thr Pro Ile Val Pro Thr Ala Asp Thr Gln Tyr Tyr Asp Asp Glu Pro Asn Gly Ala

ATA AGA TTT ACA ATG GGT TAC CAA CAT GGA CAA TTA ACC ACA TCT TCA CAA GAG CTA GAA 1442
461 Ile Arg Phe Thr Met Gly Tyr Gln His Gly Gln Leu Thr Thr Ser Ser Gln Glu Leu Glu

SacI
AGA TAC ACA TTC AAT CCA CAA AGT AAA TGT GGA AGA GCT CCA AAG CAA CAA TTT AAT CAA 1502
481 Arg Tyr Thr Phe Asn Pro Gln Ser Lys Cys Gly Arg Ala Pro Lys Gln Gln Phe Asn Gln

CAG GCA CCA CTA AAC CTA GAA AAT ACA AAT AAT GGA ACA CTT TTA CCT TCA GAT CCA ATA 1562
501 Gln Ala Pro Leu Asn Leu Glu Asn Thr Asn Asn Gly Thr Leu Leu Pro Ser Asp Pro Ile

GGA GGG AAA TCT AAC ATG CAT TTC ATG AAT ACA CTC AAT ACA TAT GGA CCA TTA ACA GCA 1622
521 Gly Gly Lys Ser Asn Met His Phe Met Asn Thr Leu Asn Thr Tyr Gly Pro Leu Thr Ala

CTA AAC AAT ACT GCA CCT GTA TTT CCA AAT GGT CAA ATA TGG GAT AAA GAA CTT GAT ACA 1682
541 Leu Asn Asn Thr Ala Pro Val Phe Pro Asn Gly Gln Ile Trp Asp Lys Glu Leu Asp Thr

BglII
GAT CTA AAA CCT AGA CTA CAT GTT ACA GCT CCA TTT GTT TGT AAA AAC AAT CCA CCA GGA 1742
561 Asp Leu Lys Pro Arg Leu His Val Thr Ala Pro Phe Val Cys Lys Asn Asn Pro Pro Gly

CAA CTA TTT GTA AAA ATA GCA CCA AAC CTA ACA GAT GAT TTC AAT GCT GAC TCT CCT CAA 1802
581 Gln Leu Phe Val Lys Ile Ala Pro Asn Leu Thr Asp Asp Phe Asn Ala Asp Ser Pro Gln

CAA CCT AGA ATA ATA ACT TAT TCA AAC TTT TGG TGG AAA GGA ACA CTA ACA TTC ACA GCA 1862
601 Gln Pro Arg Ile Ile Thr Tyr Ser Asn Phe Trp Trp Lys Gly Thr Leu Thr Phe Thr Ala

AAA ATG AGA TCC AGT AAT ATG TGG AAC CCT ATT CAA CAA CAC ACA ACA ACA GCA GAA AAC 1922
621 Lys Met Arg Ser Ser Asn Met Trp Asn Pro Ile Gln Gln His Thr Thr Thr Ala Glu Asn

ATT GGT AAA TAT ATT CCT ACA AAT ATT GGT GGC ATA AAA ATG TTT CCA GAA TAT TCA CAA 1982
641 Ile Gly Lys Tyr Ile Pro Thr Asn Ile Gly Gly Ile Lys Met Phe Pro Glu Tyr Ser Gln

CTT ATA CCA AGA AAA TTA TAC TAG AAA TAA CTC TGT AAA TAA AAA CTC AGT TAC TTG GTT 2042
661 Leu Ile Pro Arg Lys Leu Tyr *** Lys *** Leu Cys Lys *** Lys Leu Ser Tyr Leu Val

AAT CAT GTA CTA CTA TCA TG 2062
681 Asn His Val Leu Leu Ser

FIG. 4A



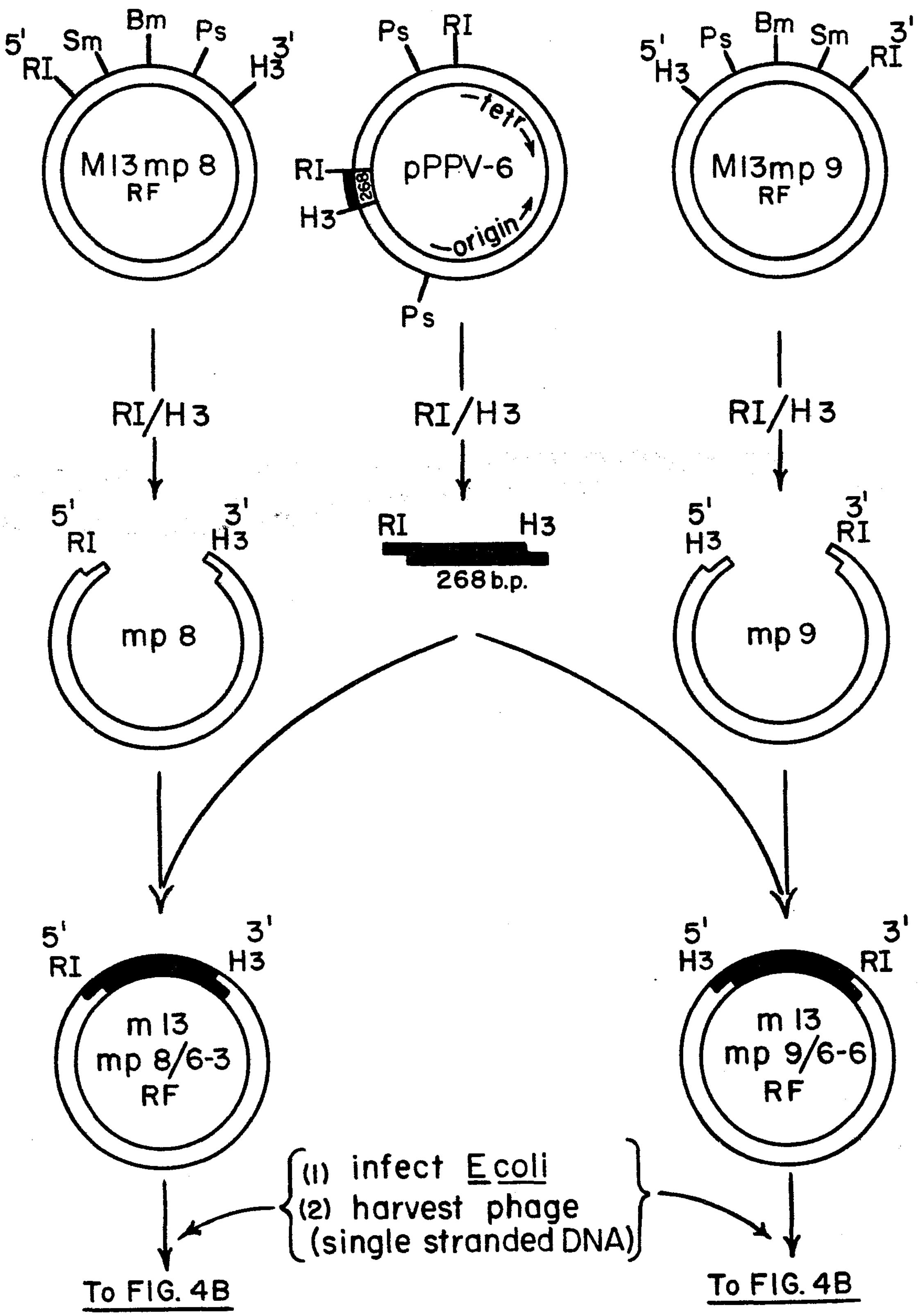
5'
RI
Sm
Bm
Ps
3'
H3
M13mp 8
RF
Ps
RI
tetr
RI
268
H3
pPPV-6
origin
Ps
5'
H3
Ps
Bm
Sm
3'
RI
M13mp 9
RF
RI/H3
RI/H3
RI/H3
5'
RI
3'
H3
mp 8
RI
H3
268 b.p.
5'
H3
3'
RI
mp 9
5'
RI
3'
H3
m 13
mp 8/6-3
RF
5'
H3
3'
RI
m 13
mp 9/6-6
RF
(1) infect E coli
(2) harvest phage
(single stranded DNA)
To FIG. 4B
To FIG. 4B

FIG. 4B

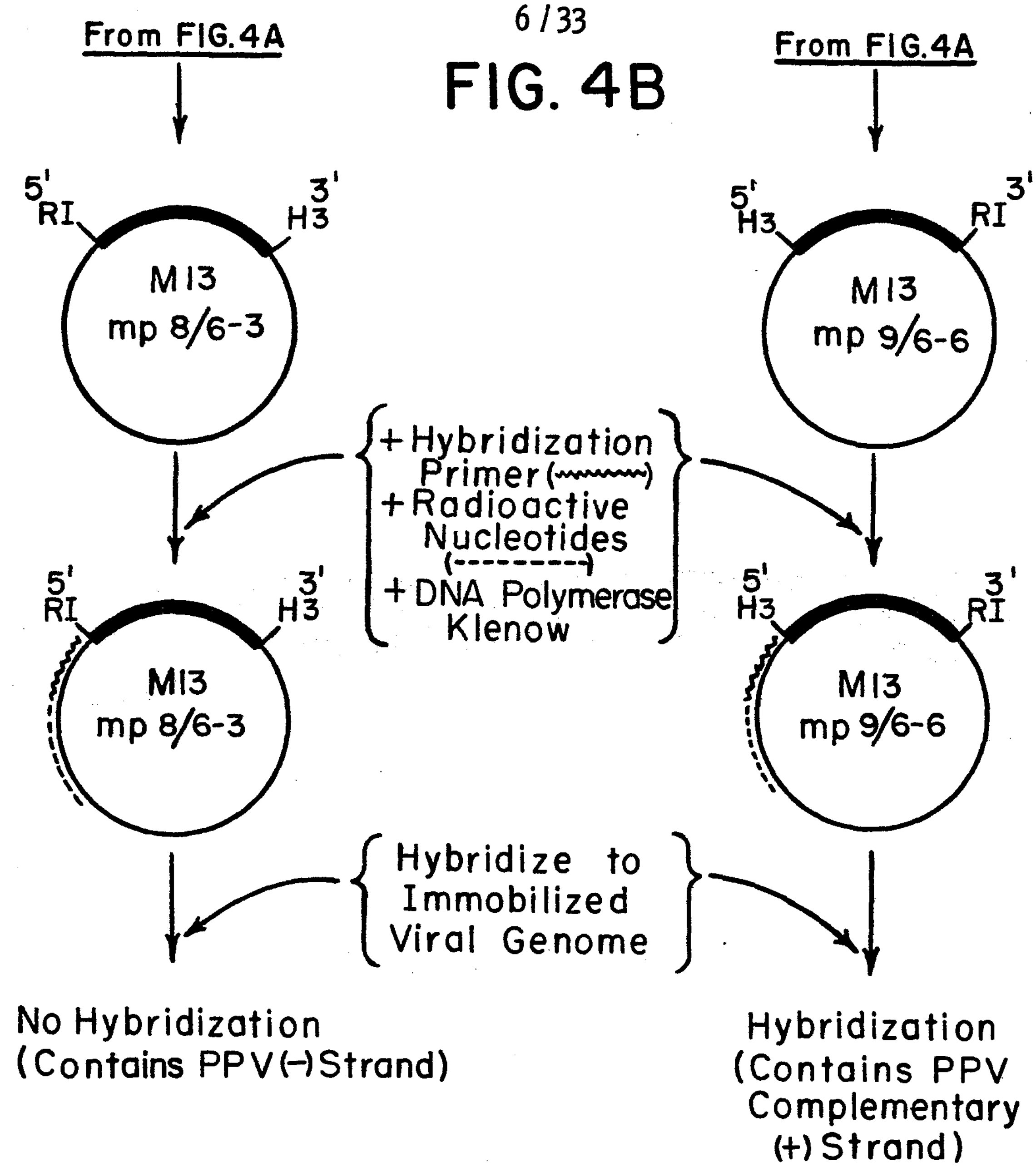

From FIG.4A
From FIG.4A
5'
RI
3'
H3
M13
mp 8/6-3
5'
H3
3'
RI
M13
mp 9/6-6
+ Hybridization
Primer
+ Radioactive
Nucleotides
+ DNA Polymerase
Klenow
M13
mp 8/6-3
M13
mp 9/6-6
Hybridize to
Immobilized
Viral Genome
No Hybridization
(Contains PPV (-) Strand)
Hybridization
(Contains PPV
Complementary
(+) Strand)


FIG. 4C

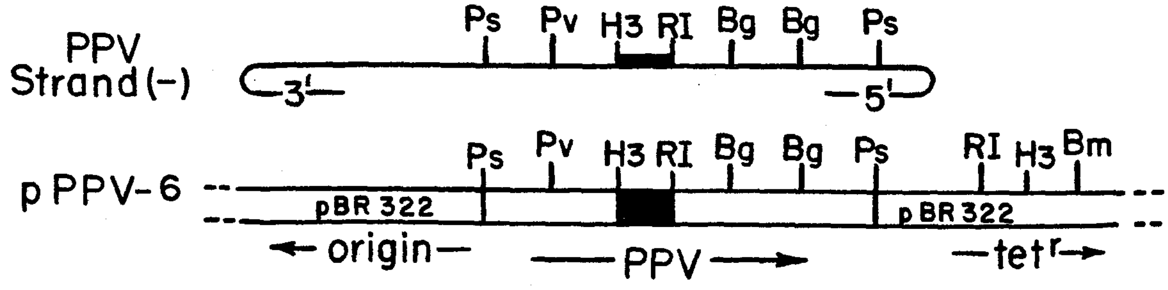

PPV
Strand (-)
Ps
Pv
H3
RI
Bg
Bg
Ps
3'
5'
p PPV-6
Ps
Pv
H3
RI
Bg
Bg
Ps
RI
H3
Bm
pBR 322
pBR 322
origin
PPV
tet^r

FIG. 5



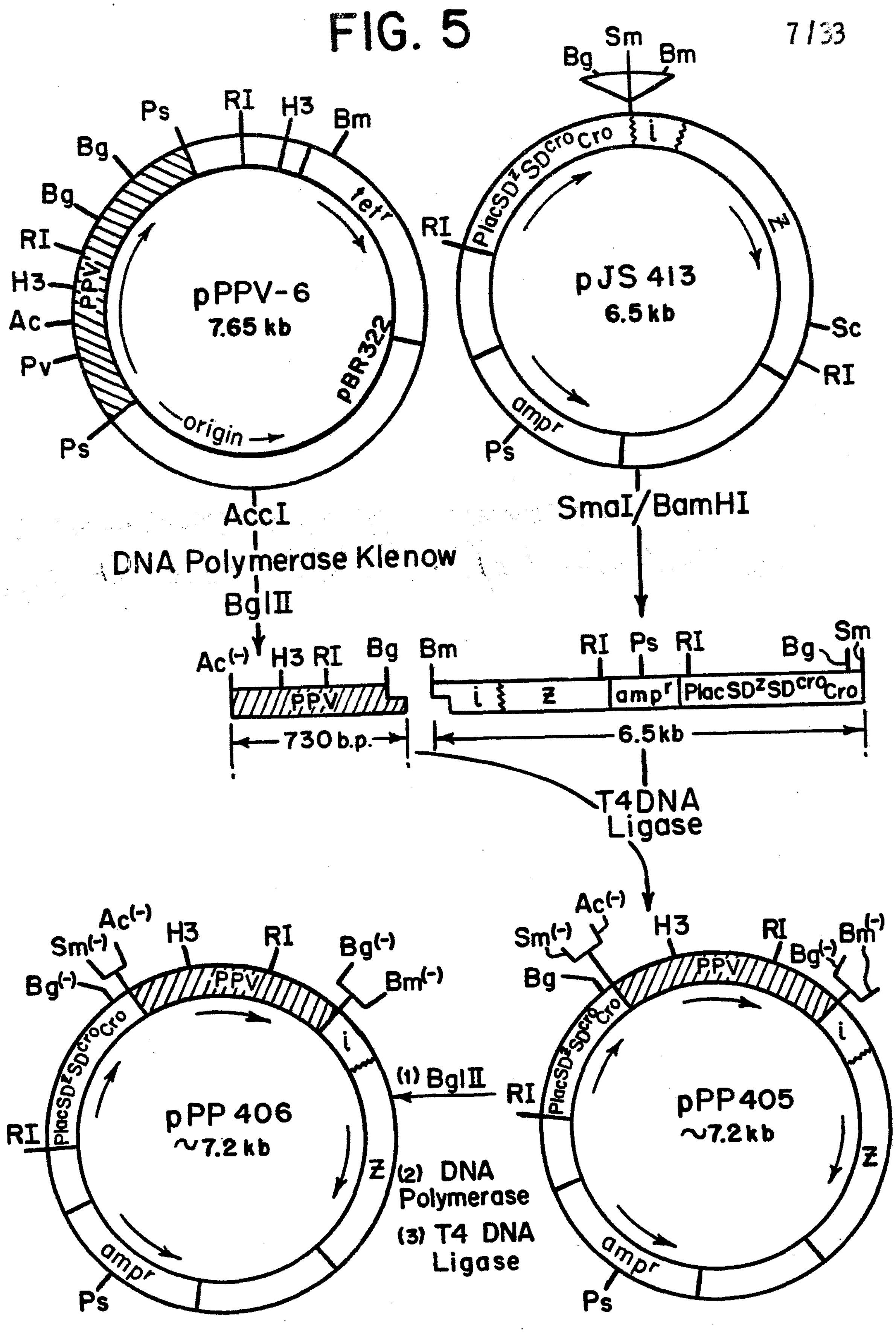
pPPV-6
7.65 kb
Ps
RI
H3
Bm
Bg
Bg
RI
H3
Ac
Pv
Ps
PPV
tetr
pBR322
origin
pJS 413
6.5 kb
Bg
Sm
Bm
PlacSDzSDcroCro
i
z
RI
Sc
RI
ampr
Ps
AccI
DNA Polymerase Klenow
BglII
SmaI/BamHI
Ac(-)
H3
RI
Bg
PPV
730 b.p.
Bm
RI
Ps
RI
Bg
Sm
i
z
ampr
PlacSDzSDcroCro
6.5 kb
T4DNA
Ligase
pPP406
~7.2 kb
Sm(-)
Ac(-)
H3
RI
Bg(-)
Bg(-)
Bm(-)
PPV
i
z
RI
PlacSDzSDcroCro
ampr
Ps
(1) BglII
(2) DNA
Polymerase
(3) T4 DNA
Ligase
pPP405
~7.2 kb
Ac(-)
Sm(-)
H3
RI
Bg(-)
Bm(-)
Bg
PPV
i
z
RI
PlacSDzSDcroCro
ampr
Ps

FIG. 6



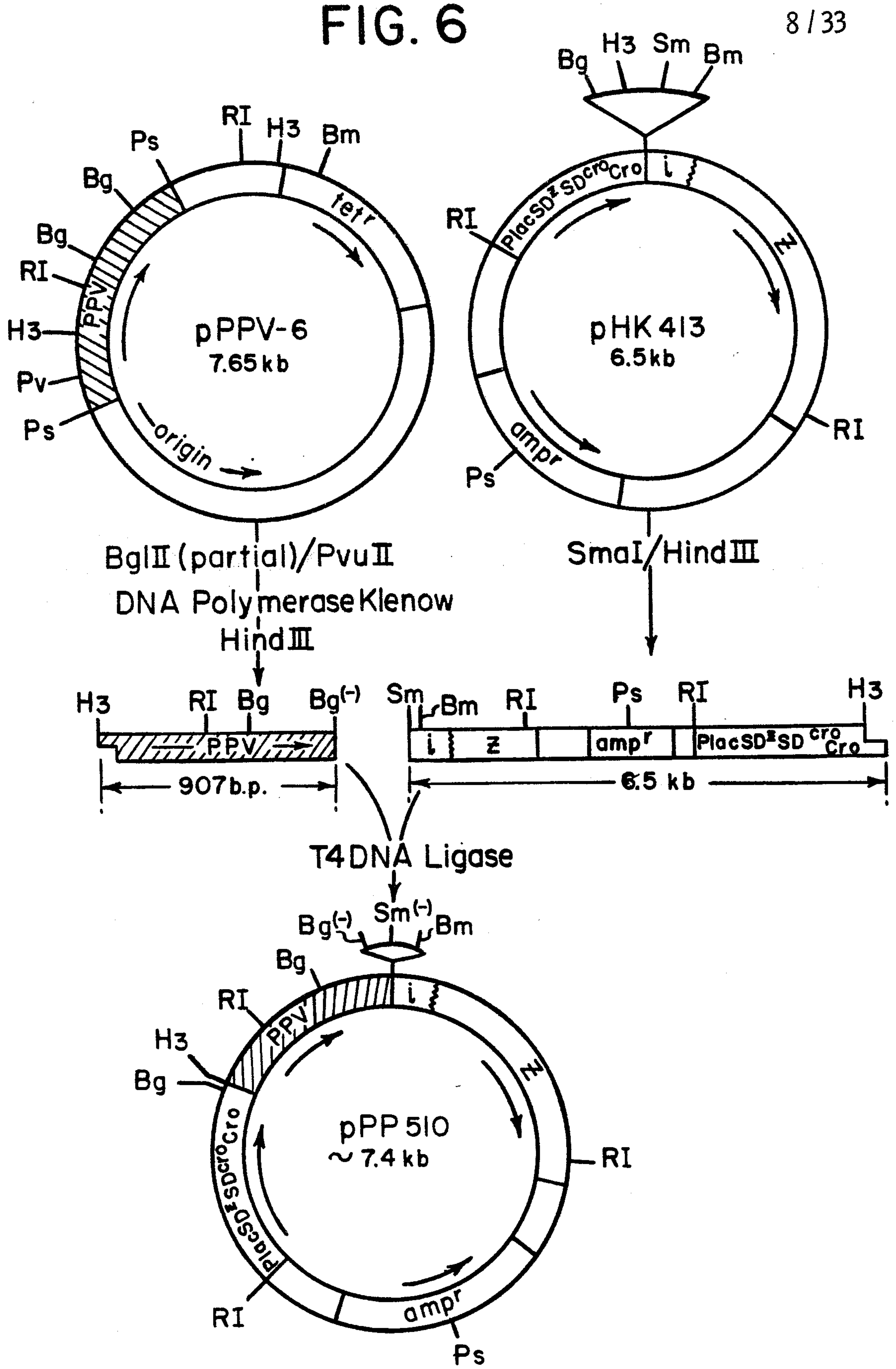
pPPV-6
7.65 kb
RI
H3
Bm
Ps
Bg
Bg
RI
H3
Pv
Ps
PPV
tetr
origin
pHK 413
6.5 kb
Bg
H3
Sm
Bm
PlacSD SDcroCro
i
z
RI
RI
Ps
ampr
BglII (partial)/PvuII
DNA Polymerase Klenow
HindIII
SmaI/HindIII
H3
RI
Bg
Bg(-)
PPV
907 b.p.
Sm
Bm
RI
Ps
RI
H3
i
z
ampr
PlacSD SD cro Cro
6.5 kb
T4 DNA Ligase
Bg(-)
Sm(-)
Bm
Bg
RI
H3
Bg
PPV
i
z
pPP 510
~ 7.4 kb
RI
PlacSD SDcroCro
RI
ampr
Ps

FIG. 7



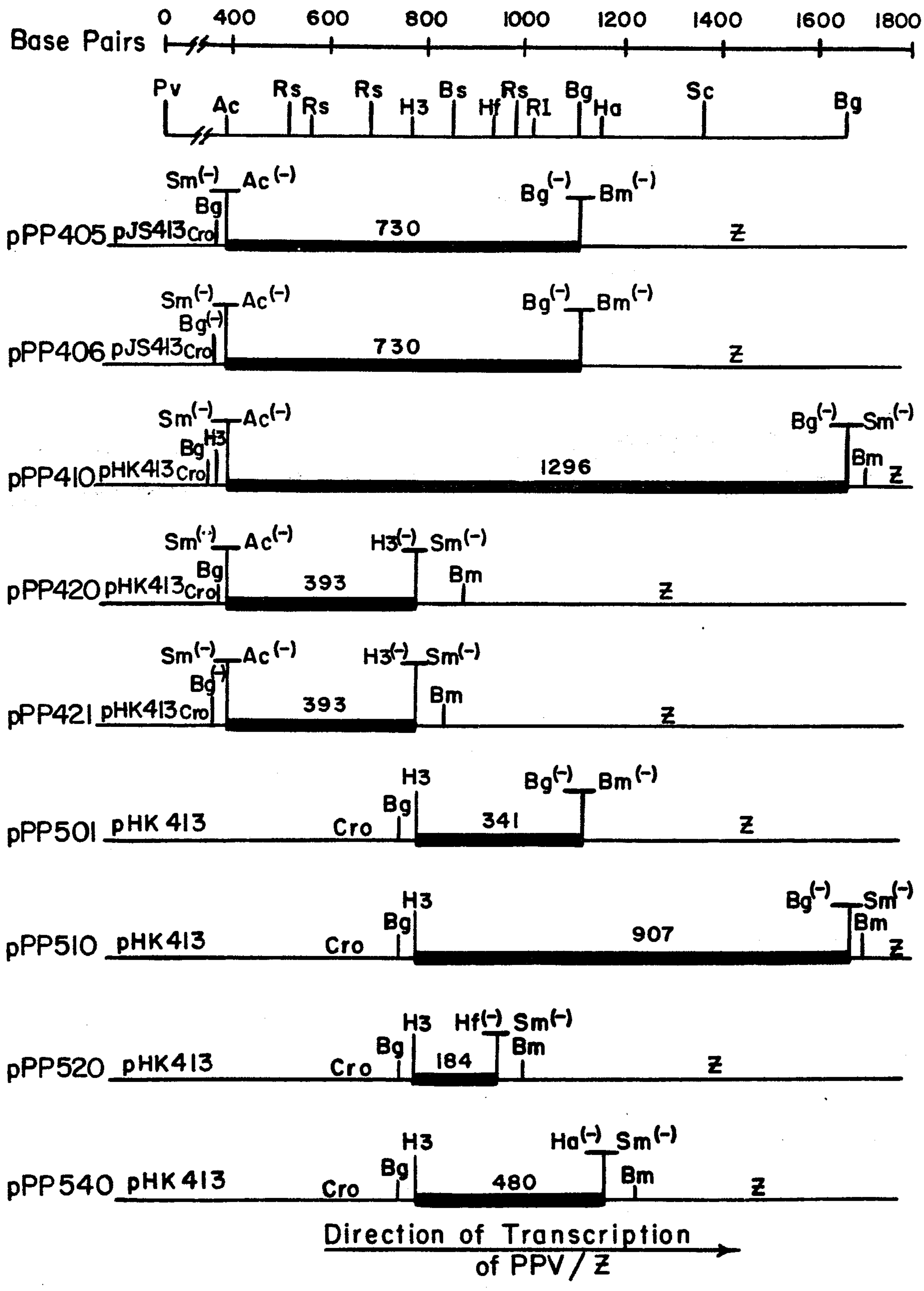
Base Pairs
0
400
600
800
1000
1200
1400
1600
1800
Pv
Ac
Rs
Rs
Rs
H3
Bs
Hf
Rs
R1
Bg
Ha
Sc
Bg
pPP405
pJS413
Cro
Sm(-)
Bg
Ac(-)
730
Bg(-)
Bm(-)
Z
pPP406
pJS413
Cro
Sm(-)
Bg(-)
Ac(-)
730
Bg(-)
Bm(-)
Z
pPP410
pHK413
Cro
Sm(-)
Bg
H3
Ac(-)
1296
Bg(-)
Sm(-)
Bm
Z
pPP420
pHK413
Cro
Sm(-)
Bg
Ac(-)
393
H3(-)
Sm(-)
Bm
Z
pPP421
pHK413
Cro
Sm(-)
Bg(-)
Ac(-)
393
H3(-)
Sm(-)
Bm
Z
pPP501
pHK 413
Cro
Bg
H3
341
Bg(-)
Bm(-)
Z
pPP510
pHK413
Cro
Bg
H3
907
Bg(-)
Sm(-)
Bm
Z
pPP520
pHK413
Cro
Bg
H3
184
Hf(-)
Sm(-)
Bm
Z
pPP540
pHK 413
Cro
Bg
H3
480
Ha(-)
Sm(-)
Bm
Z
Direction of Transcription
of PPV/Z

FIG. 7A



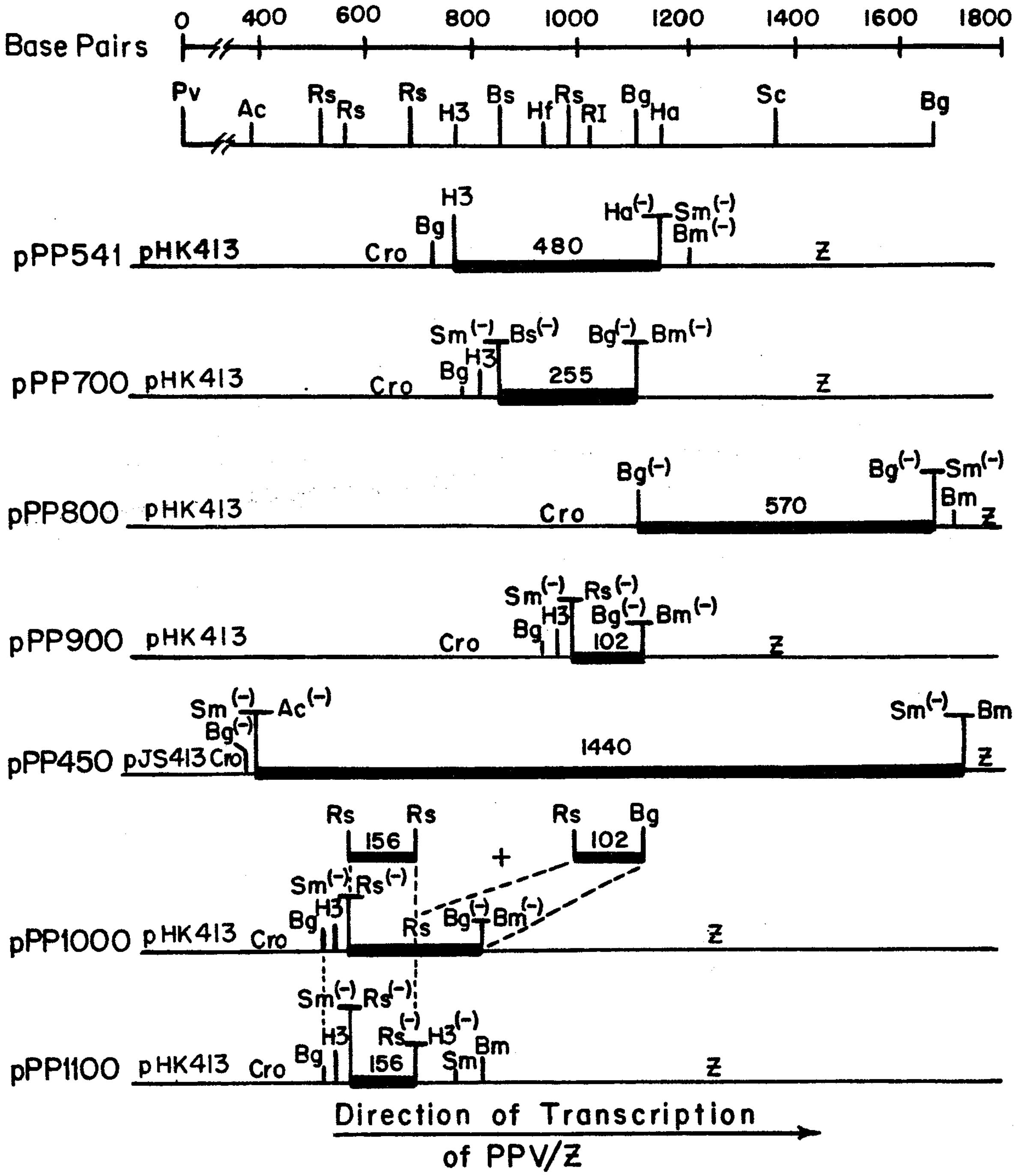


Legend

Ac = AccI Ha=HaeIII Rs=RsaI
Bg = BglII Hf=HinfI Sc=SacI
Bm = BamHI H3=HindIII Sm=SmaI
Bs = BstEII RI=EcoRI

# FIG.8A



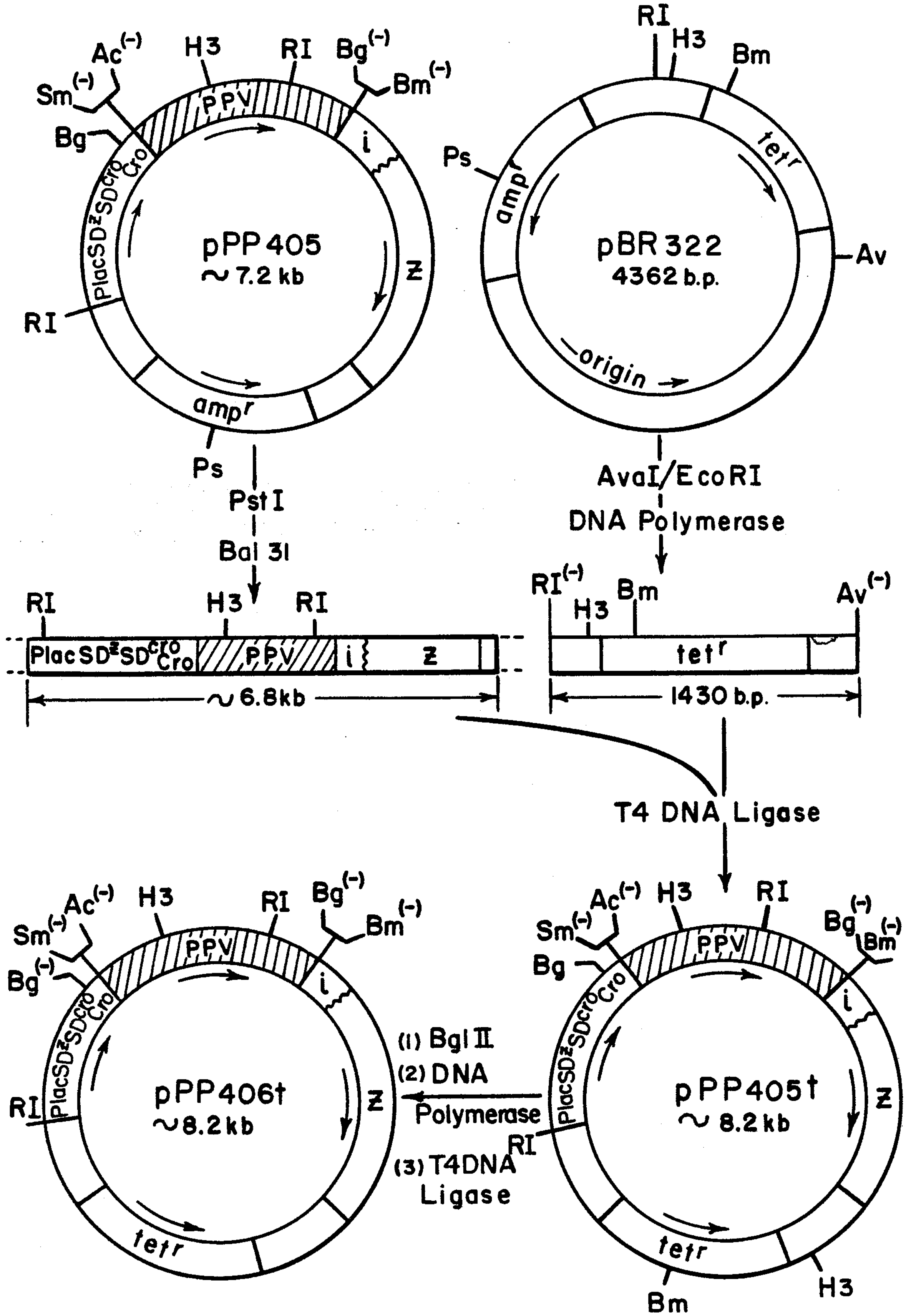

Ac(-)
Sm(-)
Bg
H3
RI
Bg(-)
Bm(-)
PPV
i
Z
PlacSDzSDcro Cro
pPP405
~7.2 kb
RI
ampr
Ps
PstI
Bal 31
RI
H3
Bm
tetr
Ps
ampr
pBR322
4362 b.p.
Av
origin
AvaI/EcoRI
DNA Polymerase
RI
H3
RI
PlacSDzSDcro Cro
PPV
i
Z
~6.8kb
RI(-)
H3
Bm
Av(-)
tetr
1430 b.p.
T4 DNA Ligase
Sm(-)
Ac(-)
H3
RI
Bg(-)
Bm(-)
Bg(-)
PPV
i
PlacSDzSDcro Cro
RI
pPP406t
~8.2 kb
Z
tetr
(1) BglII
(2) DNA Polymerase
(3) T4DNA Ligase
Ac(-)
H3
RI
Sm(-)
Bg
Bg(-)
Bm(-)
PPV
i
PlacSDzSDcro Cro
pPP405t
~8.2 kb
Z
RI
tetr
Bm
H3

# FIG. 8B



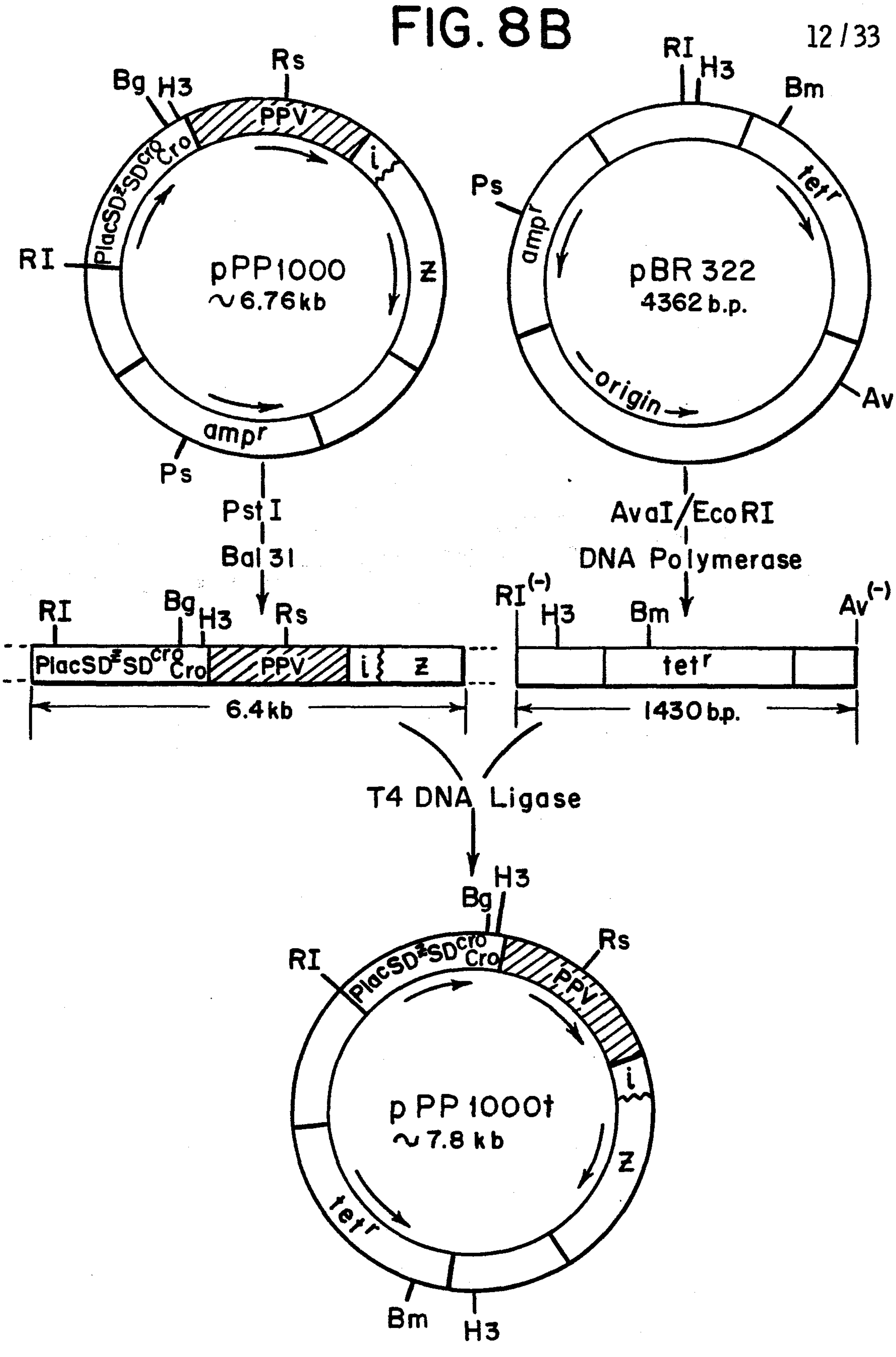
Rs
Bg
H3
PPV
PlacSDᶻSDᶜʳᵒ Cro
i
RI
pPP1000
~6.76kb
Z
ampʳ
Ps
PstI
Bal 31
RI
H3
Bm
tetʳ
Ps
ampʳ
pBR 322
4362 b.p.
origin
Av
AvaI/EcoRI
DNA Polymerase
RI⁽⁻⁾
H3
Bm
Av⁽⁻⁾
tetʳ
6.4kb
1430 b.p.
T4 DNA Ligase
Bg
H3
RI
Rs
pPP1000t
~7.8 kb
Z
tetʳ
Bm
H3

FIG. 9A

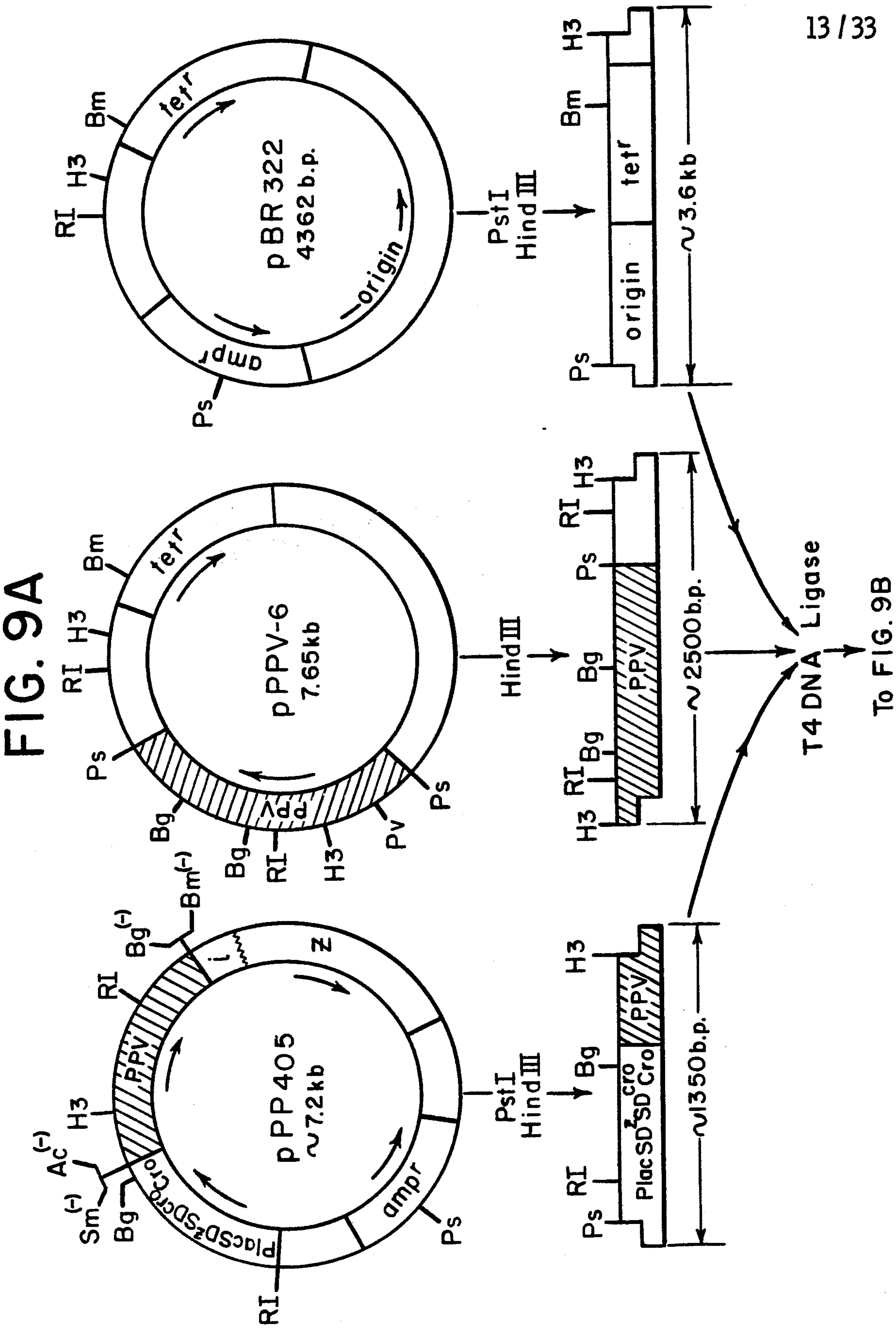
pBR 322
4362 b.p.
RI
H3
Bm
tet^r
origin
amp^r
Ps
PstI
HindIII
Ps
origin
tet^r
Bm
H3
~3.6 kb
pPPV-6
7.65 kb
RI
H3
Bm
tet^r
Ps
PPV
Bg
Bg
RI
H3
Pv
Ps
HindIII
H3
RI
Bg
Bg
PPV
Ps
RI
H3
~2500 b.p.
pPP405
~7.2 kb
Sm^(-)
Ac^(-)
Bg
H3
PPV
RI
Bg^(-)
Bm^(-)
i
Z
PlacSD^zSDcro
ampr
Ps
RI
PstI
HindIII
Ps
RI
PlacSD^zSD^cro Cro
Bg
PPV
H3
~1350 b.p.
T4 DNA Ligase
To FIG. 9B

From
FIG.9A

FIG. 9B

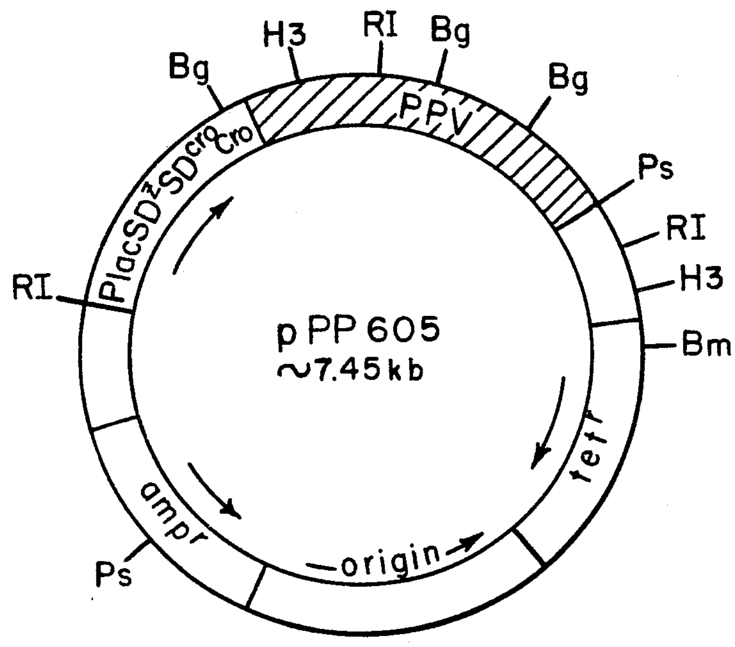

FIG. 10



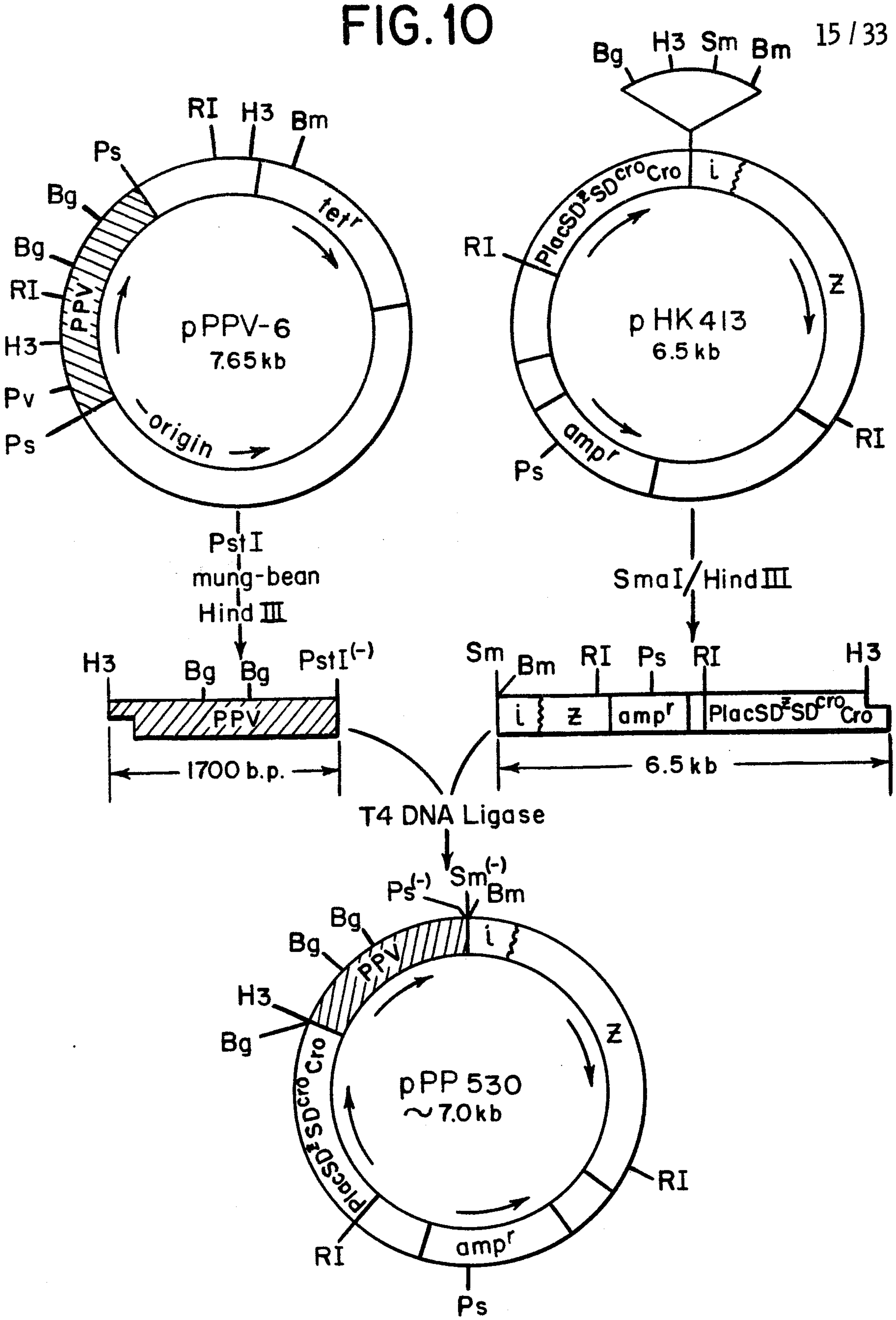
RI
H3
Bm
Ps
Bg
Bg
RI
H3
Pv
Ps
PPV
tet^r
pPPV-6
7.65 kb
origin
Bg
H3
Sm
Bm
PlacSD^z SD^cro Cro
i
Z
RI
pHK413
6.5 kb
amp^r
Ps
RI
PstI
mung-bean
Hind III
SmaI/HindIII
H3
Bg
Bg
PstI^(-)
PPV
1700 b.p.
Sm
Bm
RI
Ps
RI
H3
i
Z
amp^r
PlacSD^z SD^cro Cro
6.5 kb
T4 DNA Ligase
Ps^(-)
Sm^(-)
Bm
Bg
Bg
H3
Bg
PPV
i
Z
Cro
PlacSD^z SD^cro
pPP 530
~7.0 kb
RI
RI
amp^r
Ps

FIG. 11

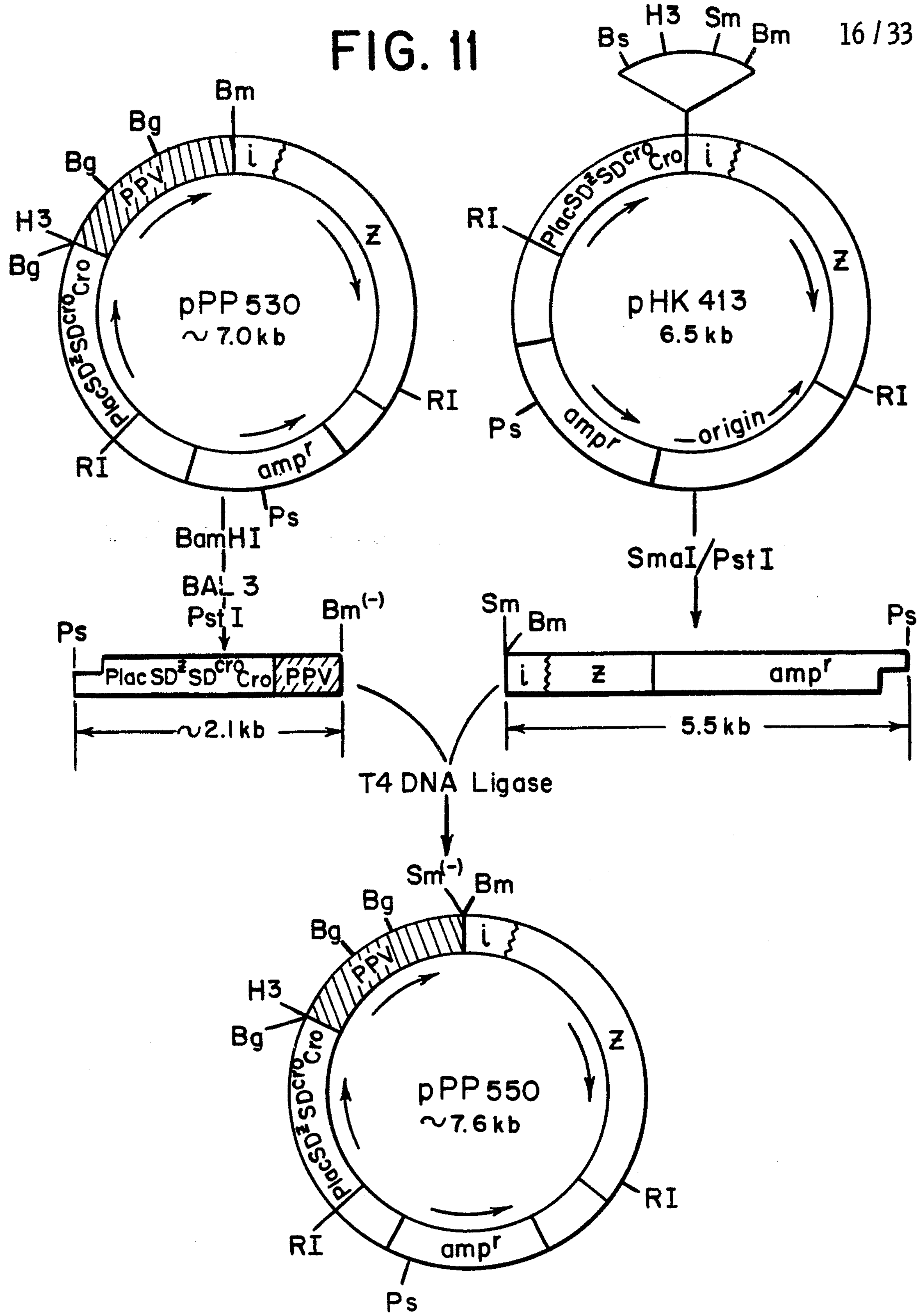
Bm
Bg
Bg
H3
Bg
PPV
i
z
Cro
PlacSD$^z$SD$^{cro}$
pPP 530
~ 7.0 kb
RI
RI
amp$^r$
Ps
Bs
H3
Sm
Bm
PlacSD$^z$SD$^{cro}$Cro
i
RI
z
pHK 413
6.5 kb
RI
Ps
amp$^r$
origin
BamHI
BAL 3
Pst I
SmaI/Pst I
Ps
Bm$^{(-)}$
Plac SD$^z$ SD$^{cro}$ Cro
PPV
~2.1 kb
Sm
Bm
Ps
i
z
amp$^r$
5.5 kb
T4 DNA Ligase
Sm$^{(-)}$
Bm
Bg
Bg
PPV
H3
Bg
i
z
PlacSD$^z$SD$^{cro}$Cro
pPP 550
~7.6 kb
RI
RI
amp$^r$
Ps

# FIG. 12



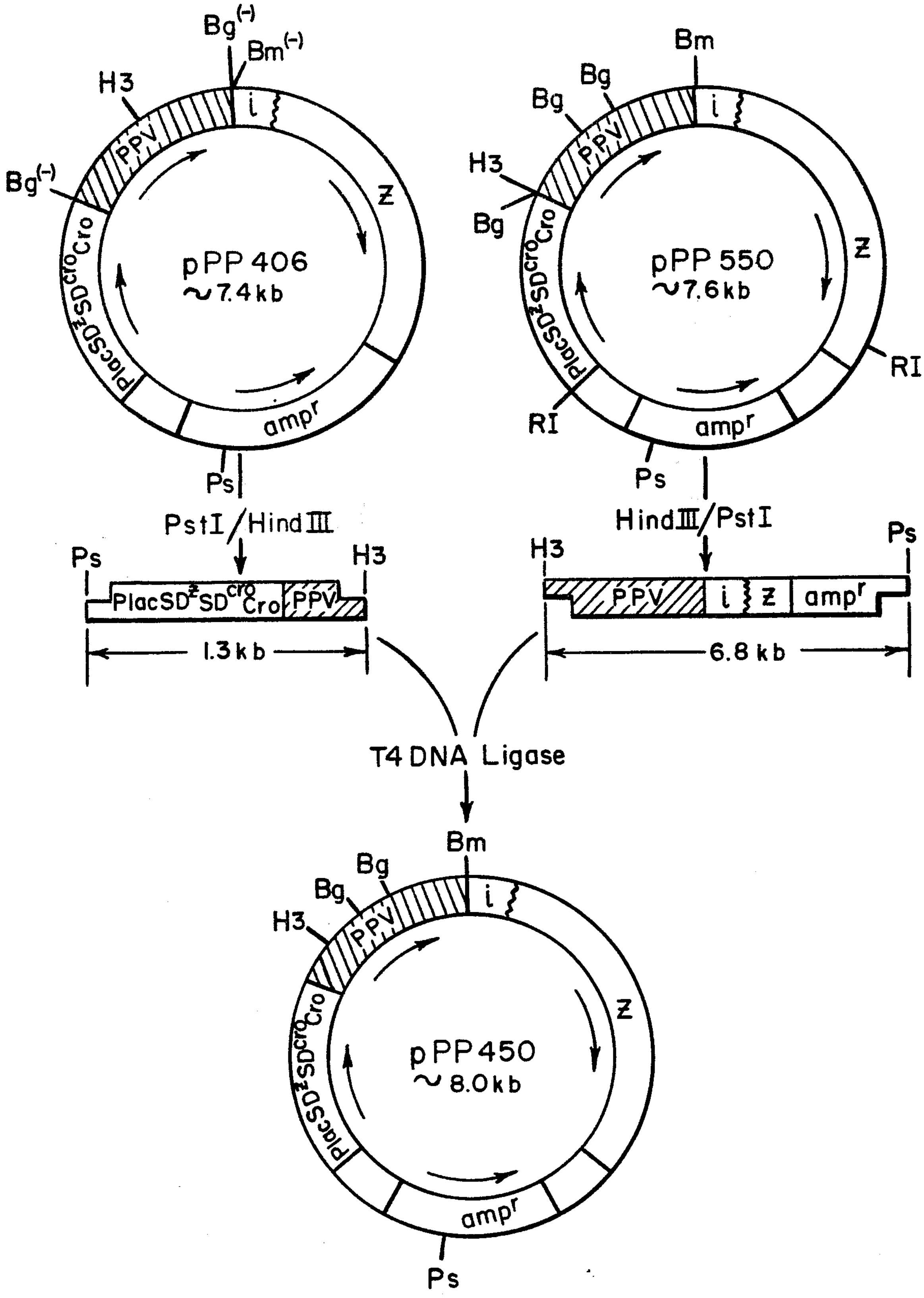
Bg(-)
Bm(-)
H3
PPV
i
Bg(-)
Z
pPP 406
~7.4 kb
PlacSDzSDcroCro
ampr
Ps
PstI / HindIII
Bm
Bg
Bg
H3
Bg
pPP 550
~7.6 kb
RI
RI
Ps
HindIII / PstI
Ps
H3
PlacSDzSDcroCro
1.3 kb
H3
Ps
PPV
i
Z
ampr
6.8 kb
T4 DNA Ligase
Bm
Bg
Bg
H3
pPP 450
~8.0 kb
Ps

FIG. 13

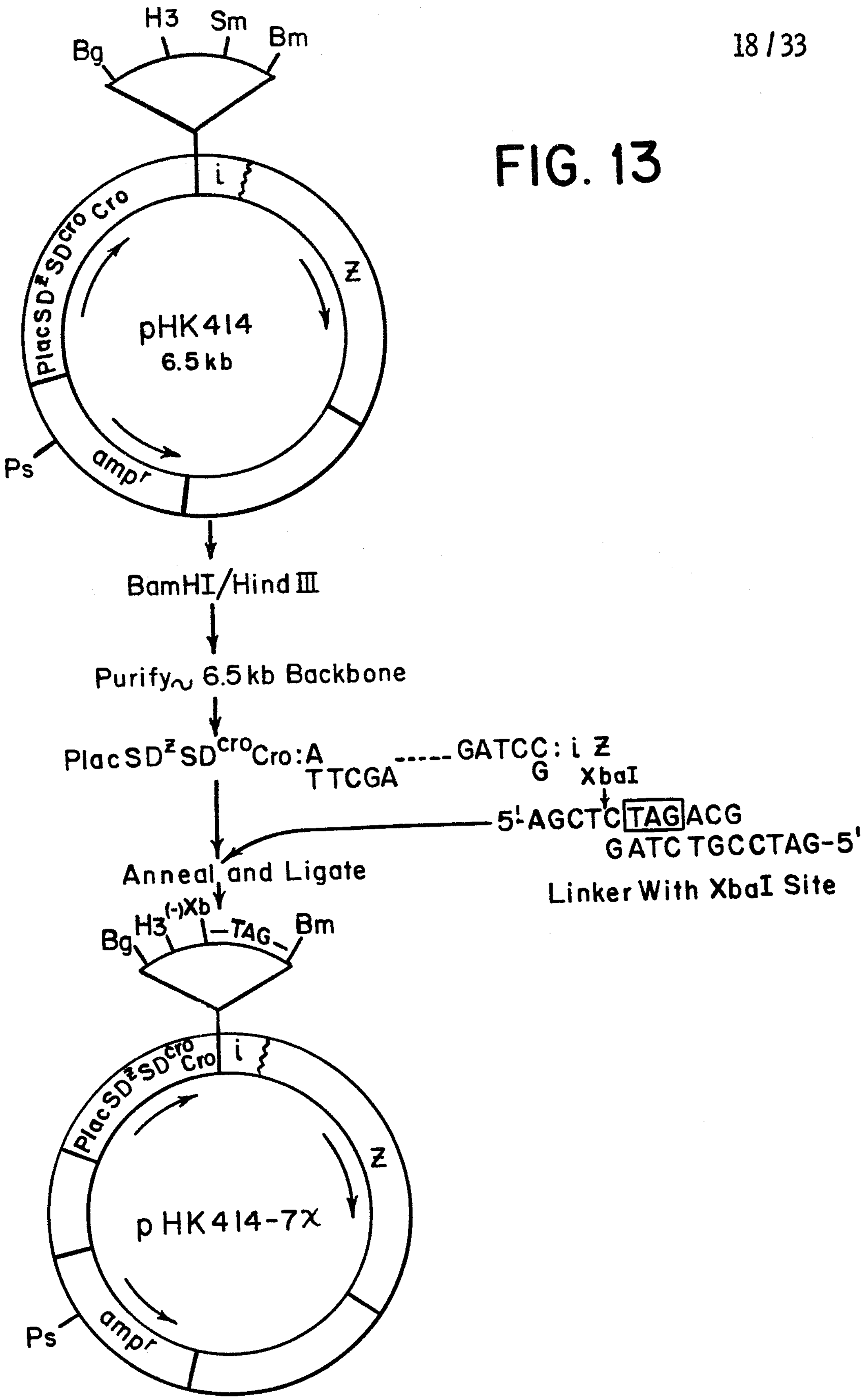
Bg
H3
Sm
Bm
i
PlacSD^z SD^cro Cro
Z
pHK414
6.5 kb
Ps
amp^r
BamHI/Hind III
Purify ~ 6.5 kb Backbone
PlacSD^z SD^cro Cro:A
TTCGA
-----GATCC: i Z
G
XbaI
5'-AGCTC TAG ACG
GATC TGCCTAG-5'
Linker With XbaI Site
Anneal and Ligate
Bg
H3
(-)Xb
TAG
Bm
PlacSD^z SD^cro Cro
i
Z
pHK414-7X
Ps
amp^r

FIG. 14



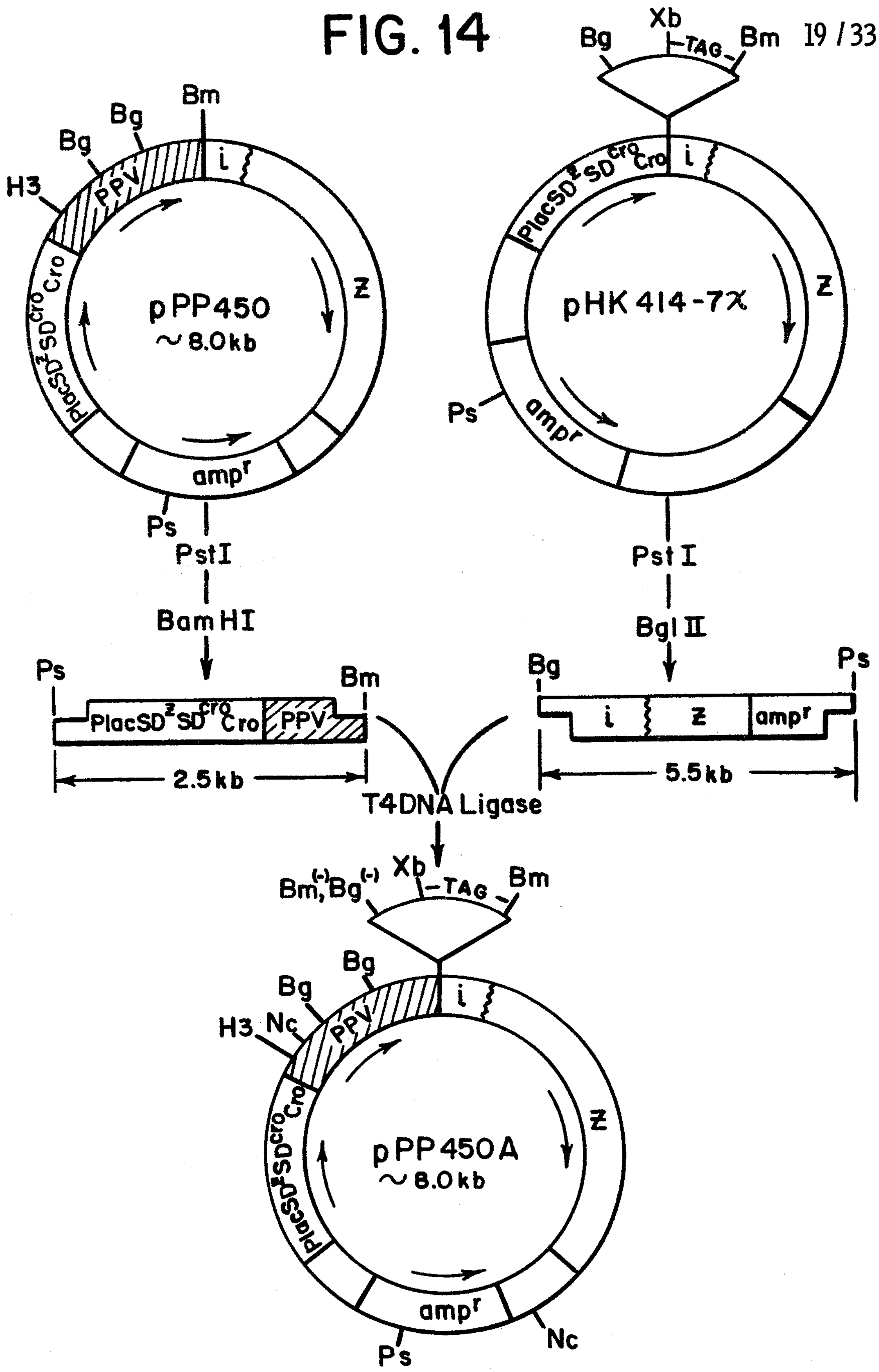

Bg
Xb
-TAG-
Bm
Bm
Bg
Bg
H3
i
PPV
Cro
PlacSD^z SD^cro
pPP450
~8.0kb
Z
amp^r
Ps
PstI
BamHI
PlacSD^z SD^cro Cro
i
pHK414-7X
Z
amp^r
Ps
Pst I
Bgl II
Ps
Bm
PlacSD^z SD^cro Cro
PPV
2.5kb
Bg
Ps
i
Z
amp^r
5.5kb
T4DNA Ligase
Bm,(-)Bg(-)
Xb
-TAG
Bm
Bg
Bg
H3
Nc
PPV
i
Cro
PlacSD^z SD^cro
pPP450A
~8.0kb
Z
amp^r
Ps
Nc

FIG. 15



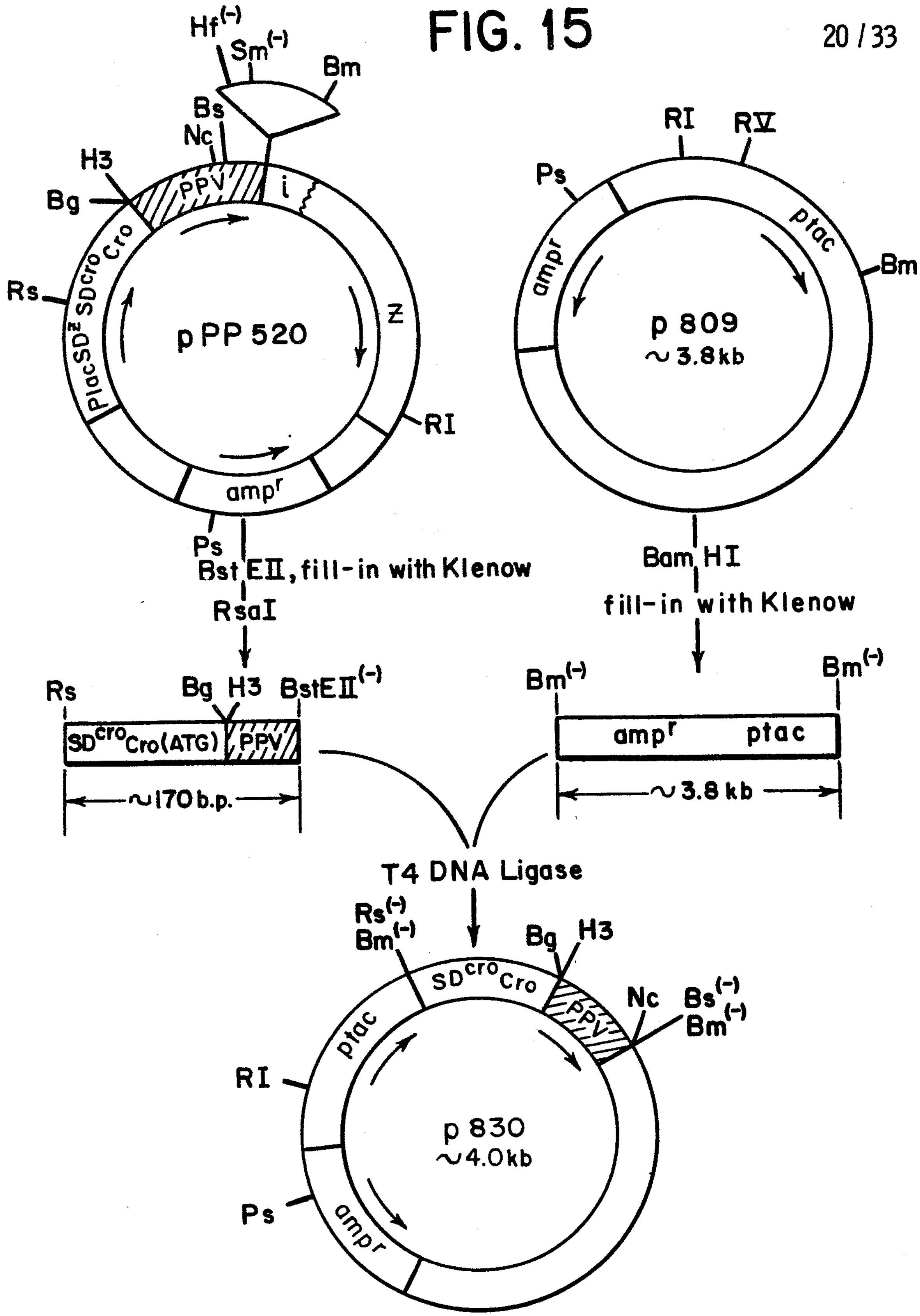

Hf(-)
Sm(-)
Bm
Bs
Nc
H3
Bg
PPV
i
Rs
PlacSDz SDcro Cro
pPP 520
Z
RI
ampr
Ps
Bst EII, fill-in with Klenow
RsaI
RI
RV
Ps
ptac
Bm
ampr
p 809
~3.8kb
Bam HI
fill-in with Klenow
Rs
Bg H3 BstEII(-)
SDcroCro(ATG) PPV
~170 b.p.
Bm(-)
Bm(-)
ampr ptac
~3.8 kb
T4 DNA Ligase
Rs(-)
Bm(-)
Bg H3
SDcroCro
PPV
Nc
Bs(-)
Bm(-)
ptac
RI
p 830
~4.0kb
Ps
ampr

FIG. 16



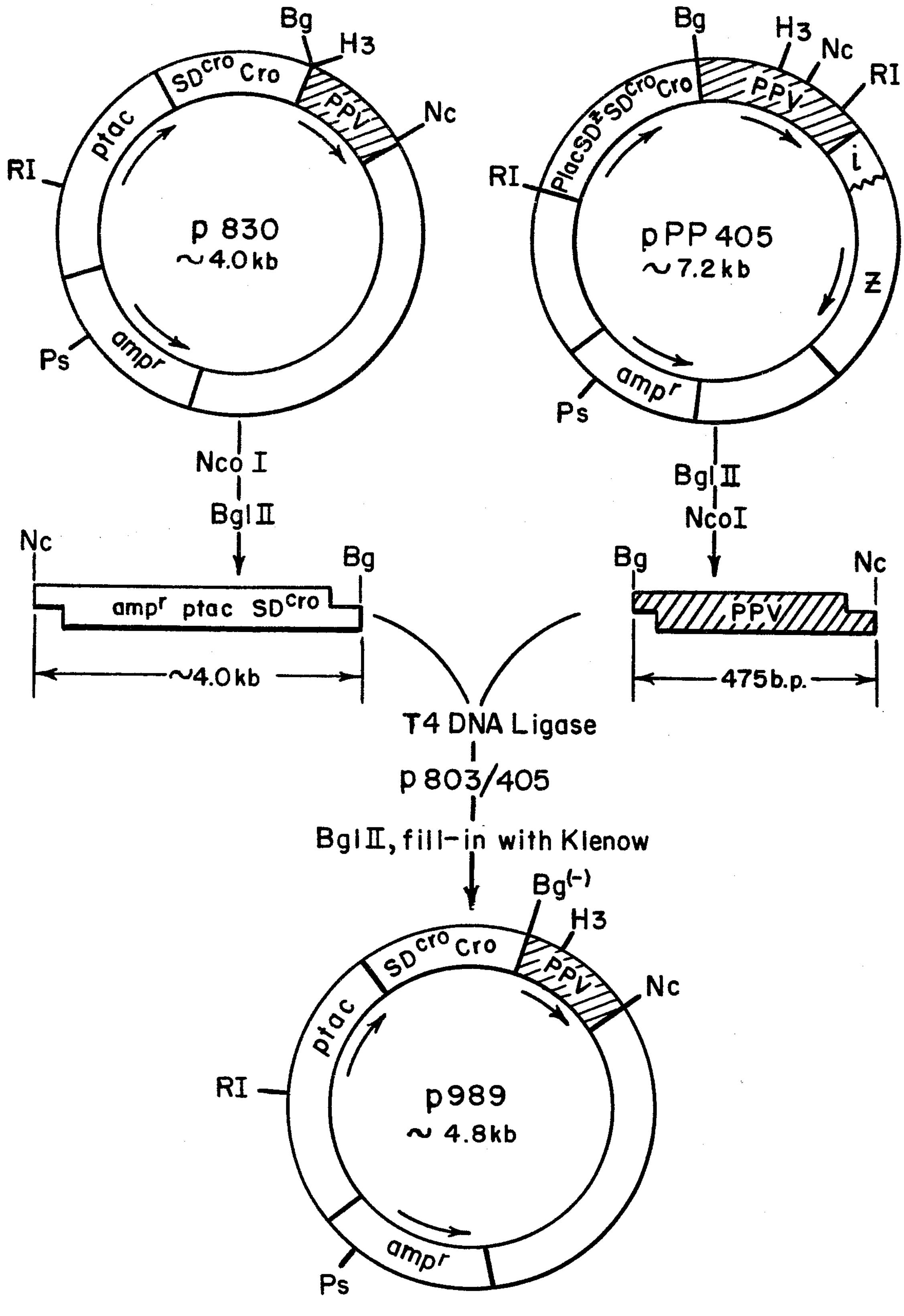

Bg
H3
SDcro Cro
PPV
Nc
ptac
RI
p 830
~4.0kb
ampr
Ps
Bg
H3
Nc
RI
PlacSDZ SDcro Cro
PPV
i
RI
pPP405
~7.2kb
Z
ampr
Ps
Nco I
Bgl II
Bgl II
NcoI
Nc
Bg
ampr ptac SDcro
~4.0kb
Bg
Nc
PPV
475b.p.
T4 DNA Ligase
p 803/405
Bgl II, fill-in with Klenow
Bg(-)
H3
SDcro Cro
PPV
Nc
ptac
RI
p989
~ 4.8kb
ampr
Ps

# FIG. 17



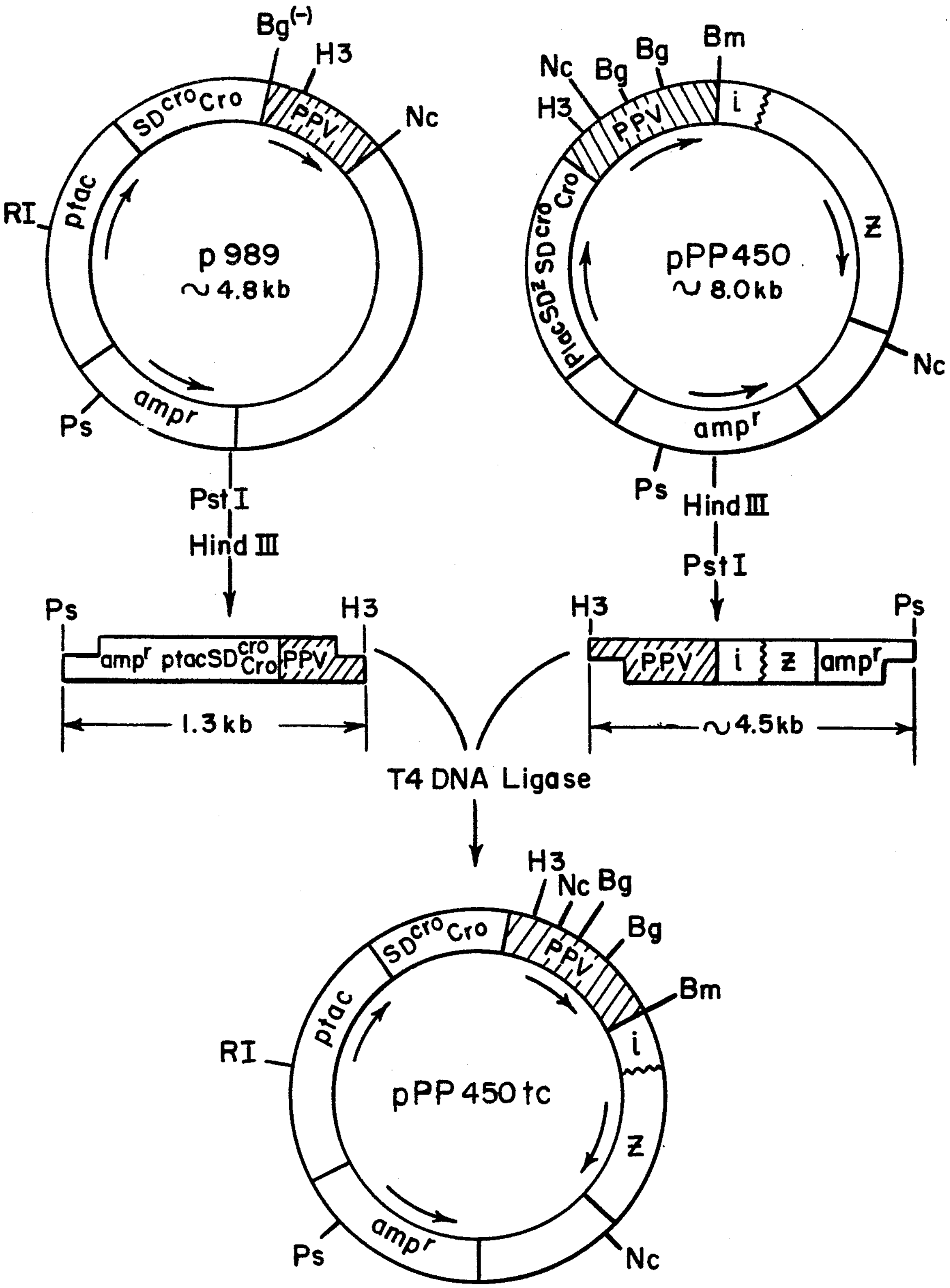
Bg(-)
H3
SDcro Cro
PPV
Nc
RI
ptac
p 989
~4.8 kb
Ps
ampr
Nc
Bg
Bg
Bm
H3
PPV
i
Z
PlacSDz SDcro Cro
pPP450
~8.0 kb
Nc
ampr
Ps
Pst I
Hind III
Hind III
Pst I
Ps
H3
ampr ptacSDcro Cro PPV
1.3 kb
H3
Ps
PPV i Z ampr
~4.5 kb
T4 DNA Ligase
H3
Nc
Bg
Bg
Bm
SDcro Cro
PPV
ptac
RI
i
pPP450tc
Z
Ps
ampr
Nc

FIG. 18



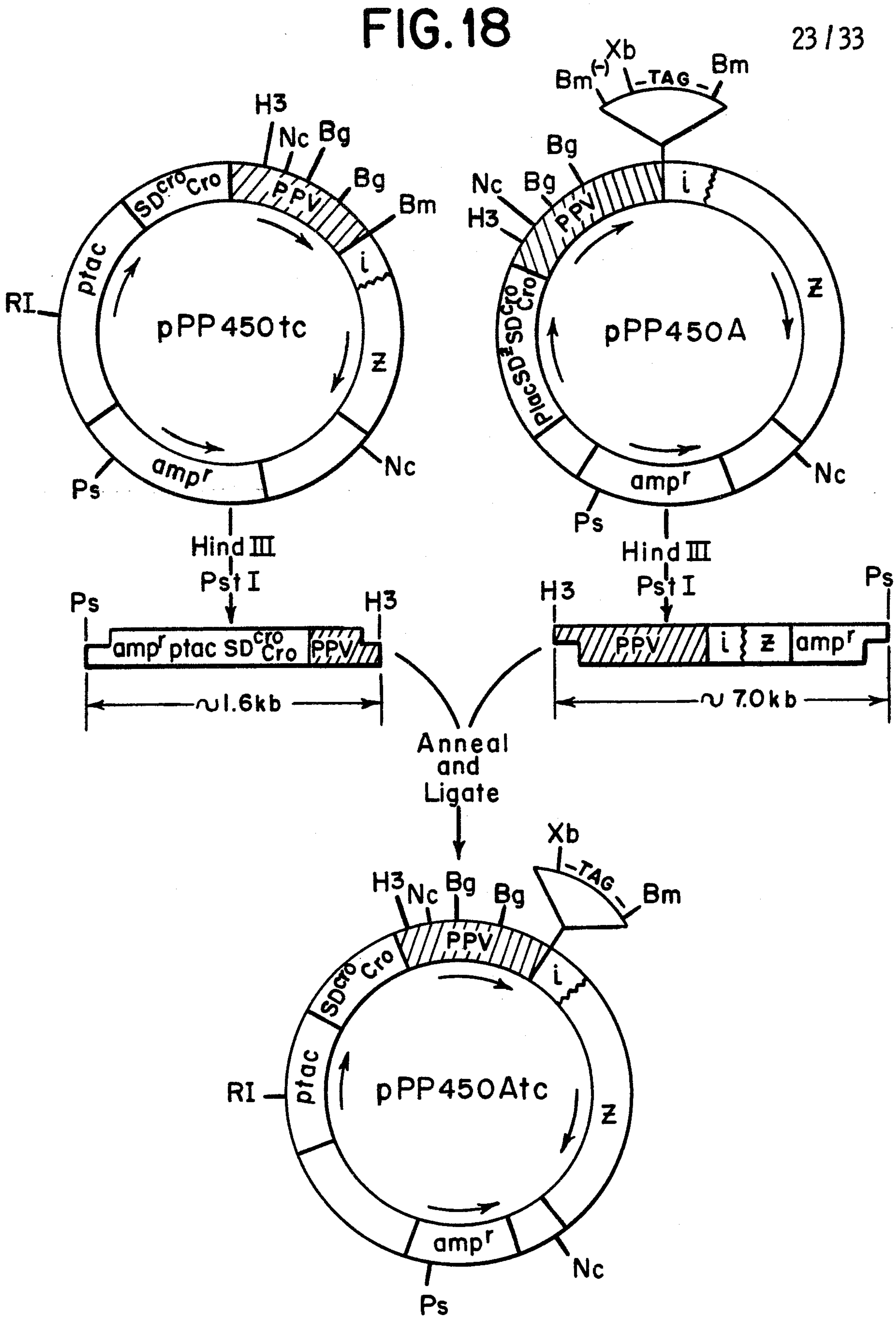

pPP450tc
H3
Nc
Bg
Bg
Bm
SDcro Cro
PPV
i
ptac
RI
Z
ampr
Ps
Nc
pPP450A
Bm(-)
Xb
-TAG-
Bm
Bg
Nc
Bg
H3
PPV
i
Plac SDz SDcro Cro
Z
ampr
Ps
Nc
Hind III
Pst I
Ps
H3
ampr ptac SDcro Cro PPV
~1.6kb
H3
Ps
PPV
i
Z
ampr
~7.0kb
Anneal
and
Ligate
pPP450Atc
Xb
-TAG-
Bm
H3
Nc
Bg
Bg
PPV
SDcro Cro
i
ptac
RI
Z
ampr
Ps
Nc

FIG. 19



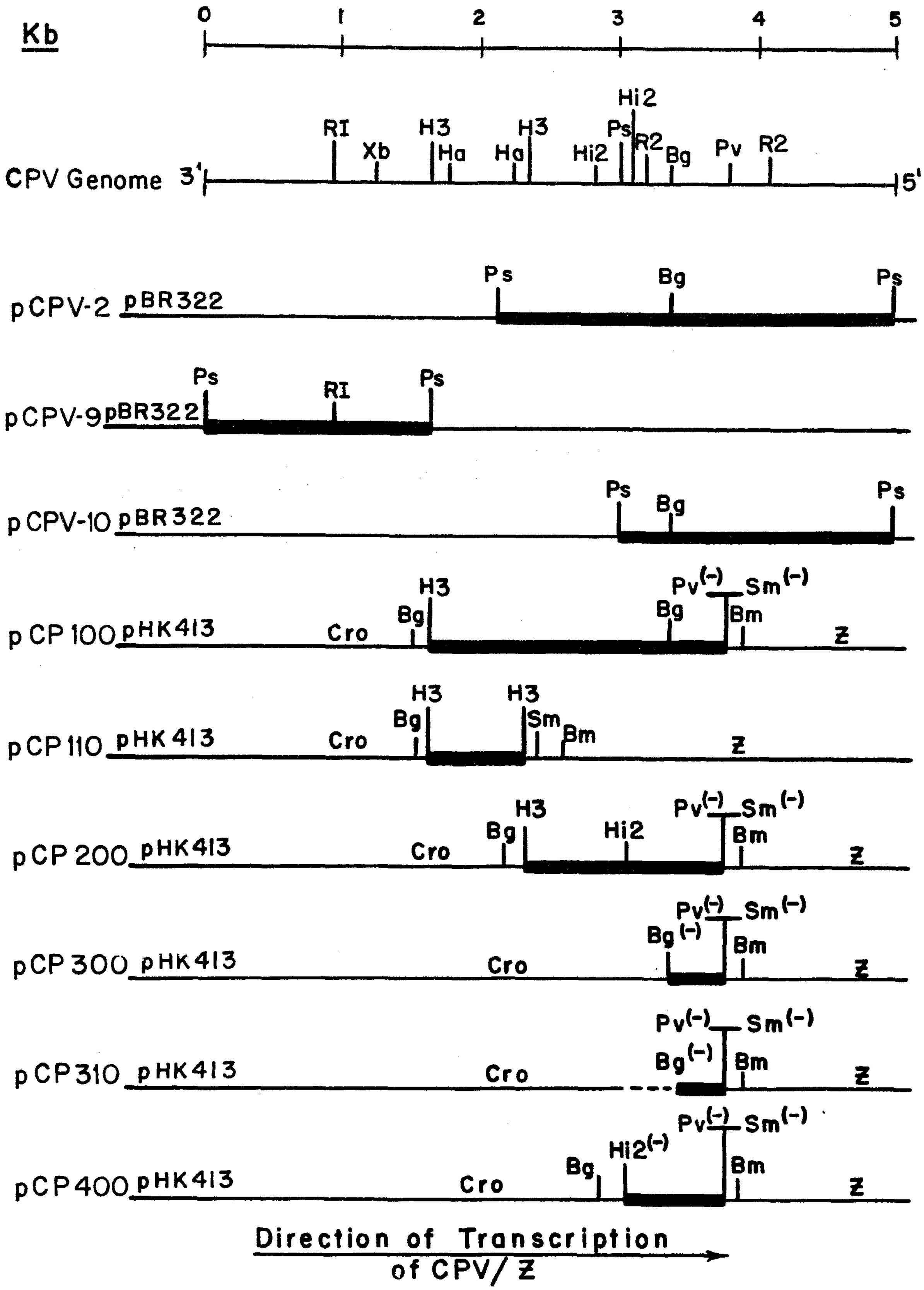
Kb
0
1
2
3
4
5
CPV Genome 3'
5'
RI
Xb
H3
Ha
Ha
H3
Hi2
Ps
Hi2
R2
Bg
Pv
R2
pCPV-2 pBR322
Ps
Bg
Ps
pCPV-9 pBR322
Ps
RI
Ps
pCPV-10 pBR322
Ps
Bg
Ps
pCP100 pHK413
Cro
Bg
H3
Bg
Pv(-) Sm(-)
Bm
Z
pCP110 pHK413
Cro
Bg
H3
H3
Sm
Bm
Z
pCP200 pHK413
Cro
Bg
H3
Hi2
Pv(-) Sm(-)
Bm
Z
pCP300 pHK413
Cro
Bg(-)
Pv(-) Sm(-)
Bm
Z
pCP310 pHK413
Cro
Pv(-) Sm(-)
Bg(-)
Bm
Z
pCP400 pHK413
Cro
Bg
Hi2(-)
Pv(-) Sm(-)
Bm
Z
Direction of Transcription
of CPV/Z

# FIG. 20



AC GGA AAC ATG GCT TTA 17
1 GLY ASN MET ALA LEU

GAT GAT ATT CAT GCA CAA ATT GTA ACA CCT TGG TCA TTG GTT GAT GCA AAT GCT TGG GGA 77
6 ASP ASP ILE HIS ALA GLN ILE VAL THR PRO TRP SER LEU VAL ASP ALA ASN ALA TRP GLY

GTT TGG TTT AAT CCA GGA GAT TGG CAA CTA ATT GTT AAT ACT ATG AGT GAG TTG CAT TTA 137
26 VAL TRP PHE ASN PRO GLY ASP TRP GLN LEU ILE VAL ASN THR MET SER GLU LEU HIS LEU

GTT AGT TTT GAA CAA GAA ATT TTT AAT GTT GTT TTA AAG ACT GTT TCA GAA TCT GCT ACT 197
46 VAL SER PHE GLU GLN GLU ILE PHE ASN VAL VAL LEU LYS THR VAL SER GLU SER ALA THR

CAG CCA CCA ACT AAA GTT TAT AAT AAT GAT TTA ACT GCA TCA TTG ATG GTT GCA TTA GAT 257
66 GLN PRO PRO THR LYS VAL TYR ASN ASN ASP LEU THR ALA SER LEU MET VAL ALA LEU ASP

Bgl II
AGT AAT AAT ACT ATG CCA TTT ACT CCA GCA GCT ATG AGA TCT GAG ACA TTG GGT TTT TAT 317
86 SER ASN ASN THR MET PRO PHE THR PRO ALA ALA MET ARG SER GLU THR LEU GLY PHE TYR

CCA TGG AAA CCA ACC ATA CCA ACT CCA TGG AGA TAT TAT TTT CAA TGG GAT AGA ACA TTA 377
106 PRO TRP LYS PRO THR ILE PRO THR PRO TRP ARG TYR TYR PHE GLN TRP ASP ARG THR LEU

ATA CCA TCT CAT ACT GGA ACT AGT GGC ACA CCA ACA AAT ATA TAC CAT GGT ACA GAT CCA 437
126 ILE PRO SER HIS THR GLY THR SER GLY THR PRO THR ASN ILE TYR HIS GLY THR ASP PRO

GAT GAT GTT CAA TTT TAT ACT ATT GAA AAT TCT GTG CCA GTA CAC TTA CTA AGA ACA GGT 497
146 ASP ASP VAL GLN PHE TYR THR ILE GLU ASN SER VAL PRO VAL HIS LEU LEU ARG THR GLY

GAT GAA TTT GCT ACA GGA ACA TTT TTT TTT GAT TGT AAA CCA TGT AGA CTA ACA CAT ACA 557
166 ASP GLU PHE ALA THR GLY THR PHE PHE PHE ASP CYS LYS PRO CYS ARG LEU THR HIS THR

TGG CAA ACA AAT AGA GCA TTG GGC TTA CCA CCA TTT CTA AAT TCT TTG CCT CAA TCT GAA 617
186 TRP GLN THR ASN ARG ALA LEU GLY LEU PRO PRO PHE LEU ASN SER LEU PRO GLN SER GLU

GGA GCT ACT AAC TTT GGT GAT ATA GGA GTT CAA CAA GAT AAA AGA CGT GGT GTA ACT CAA 677
206 GLY ALA THR ASN PHE GLY ASP ILE GLY VAL GLN GLN ASP LYS ARG ARG GLY VAL THR GLN

PvuII(-) SmaI(-)
ATG GGA AAT ACA AAC TAT ATT ACT GAA GCT ACT ATT ATG AG(T) (A)CA GGG G 717
226 MET GLY ASN THR ASN TYR ILE THR GLU ALA THR ILE MET (A) (T) GLY

FIG. 21

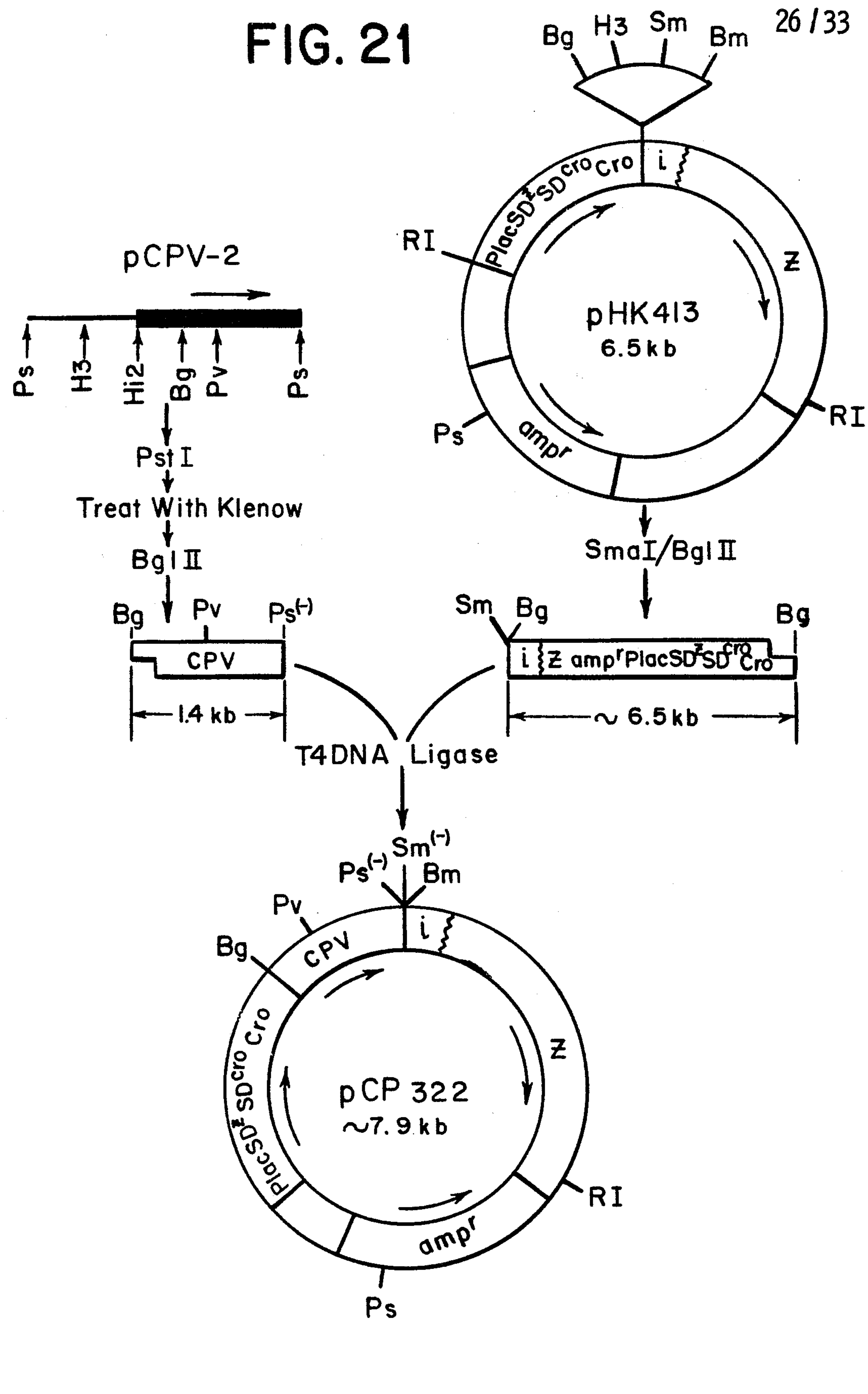
Bg
H3
Sm
Bm
Cro
i
PlacSD$^z$SD$^{cro}$
RI
Z
pHK413
6.5 kb
Ps
amp$^r$
RI
pCPV-2
Ps
H3
Hi2
Bg
Pv
Ps
Pst I
Treat With Klenow
Bgl II
SmaI/Bgl II
Bg
Pv
Ps$^{(-)}$
CPV
1.4 kb
Sm
Bg
Bg
i
Z amp$^r$PlacSD$^z$SD$^{cro}$Cro
~ 6.5 kb
T4DNA Ligase
Sm$^{(-)}$
Ps$^{(-)}$
Bm
Pv
Bg
CPV
i
Cro
PlacSD$^z$SD$^{cro}$
Z
pCP 322
~7.9 kb
RI
amp$^r$
Ps

FIG. 22



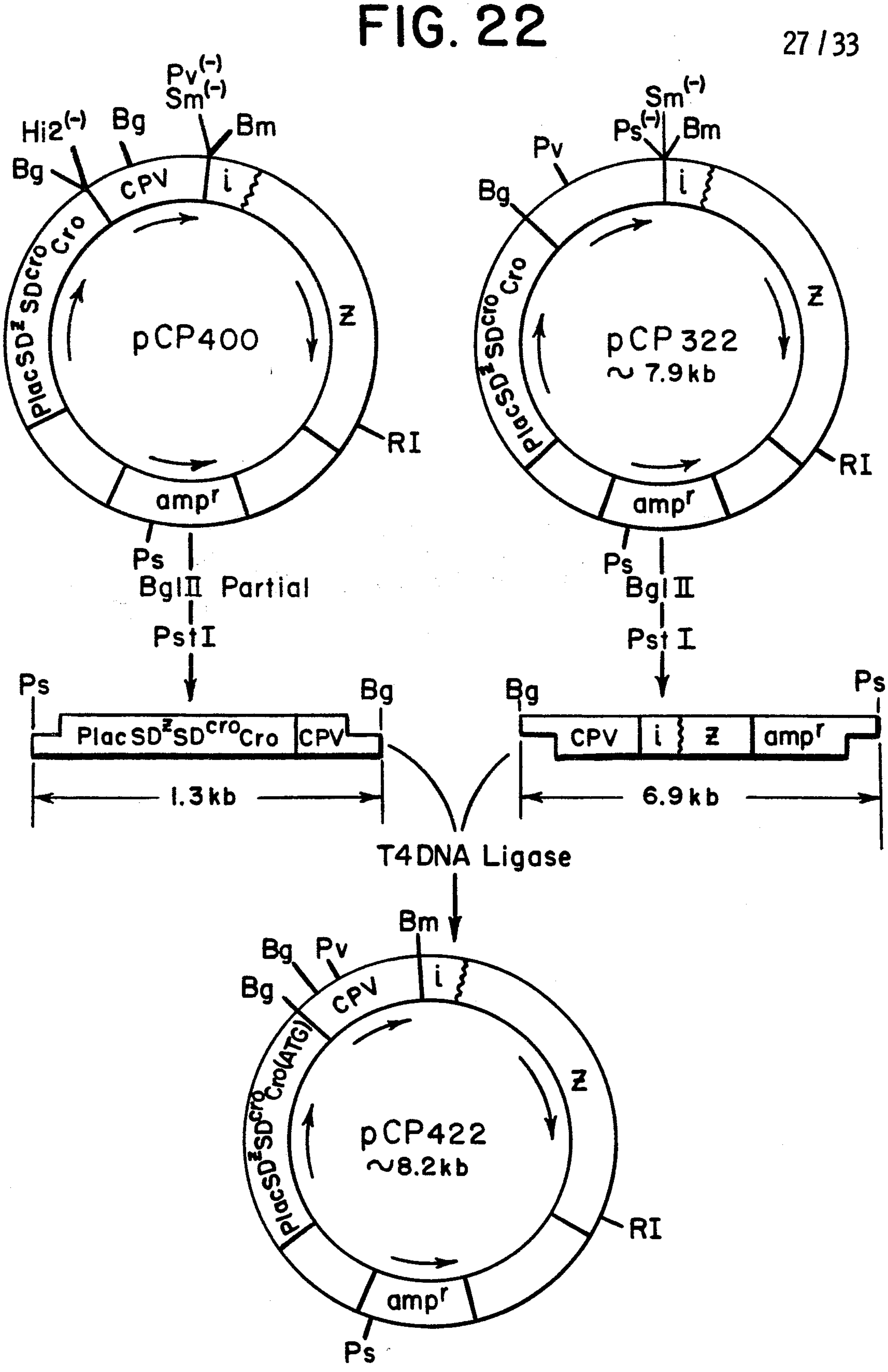
Pv(-)
Sm(-)
Hi2(-)
Bg
Bm
Bg
CPV
i
PlacSD^z SD^cro Cro
pCP400
Z
RI
amp^r
Ps
Sm(-)
Ps(-)
Bm
Pv
Bg
i
PlacSD^z SD^cro Cro
pCP322
~7.9kb
Z
RI
amp^r
Ps
BglII Partial
PstI
BglII
PstI
Ps
Bg
PlacSD^z SD^cro Cro
CPV
1.3kb
Bg
Ps
CPV
i
Z
amp^r
6.9kb
T4DNA Ligase
Bm
Bg
Pv
Bg
CPV
i
PlacSD^z SD^cro Cro(ATG)
pCP422
~8.2kb
Z
RI
amp^r
Ps

FIG.23

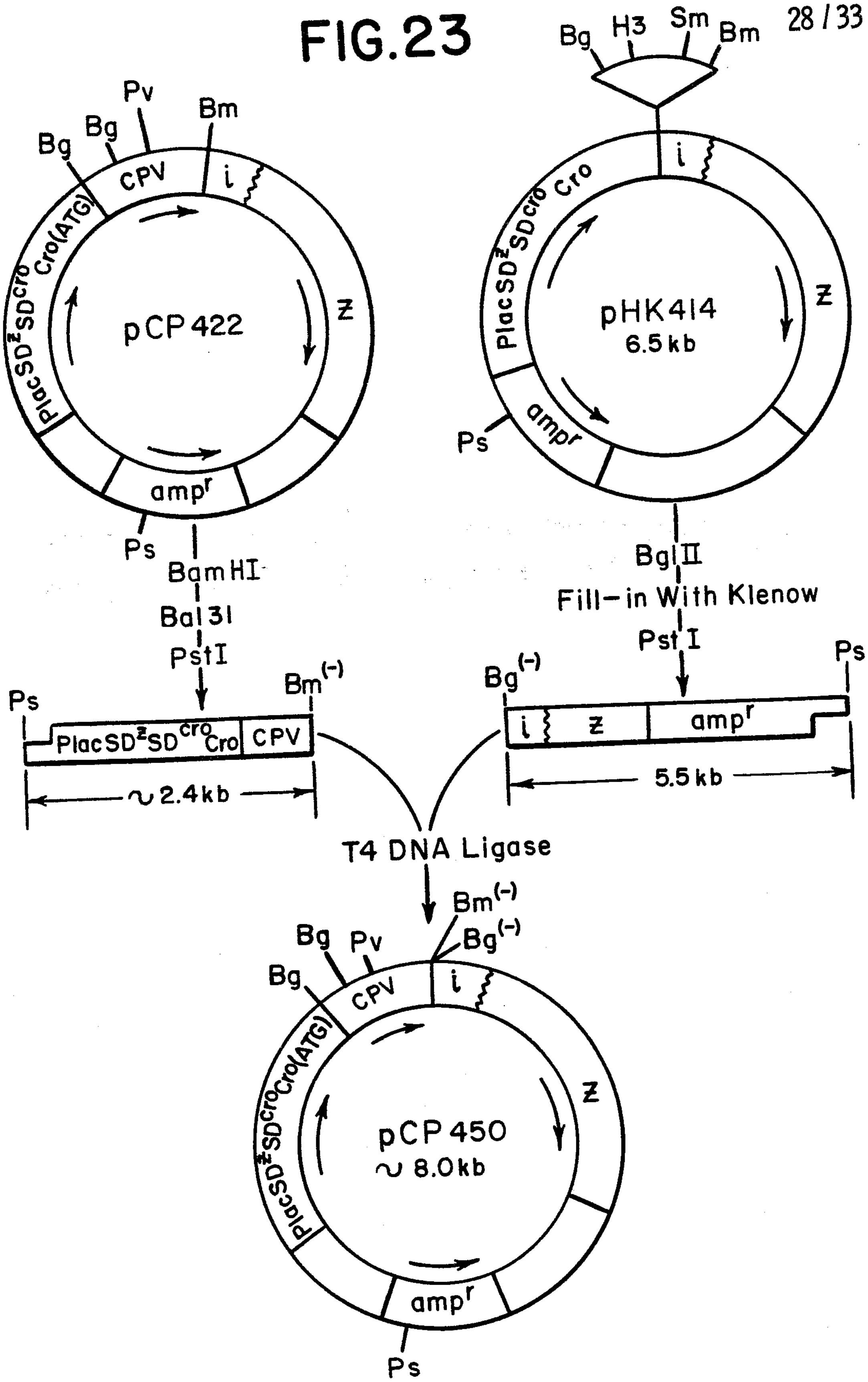

Bg
Bg
Pv
Bm
CPV
i
Cro(ATG)
PlacSD$^z$SD$^{cro}$
pCP422
z
amp$^r$
Ps
Bg
H3
Sm
Bm
i
PlacSD$^z$SD$^{cro}$ Cro
pHK414
6.5 kb
z
amp$^r$
Ps
BamHI
Bal31
PstI
BglII
Fill-in With Klenow
PstI
Ps
PlacSD$^z$SD$^{cro}$Cro
CPV
Bm$^{(-)}$
~2.4 kb
Bg$^{(-)}$
i
z
amp$^r$
Ps
5.5 kb
T4 DNA Ligase
Bm$^{(-)}$
Bg$^{(-)}$
Bg
Pv
Bg
CPV
i
PlacSD$^z$SD$^{cro}$Cro(ATG)
pCP450
~8.0 kb
z
amp$^r$
Ps

FIG. 24

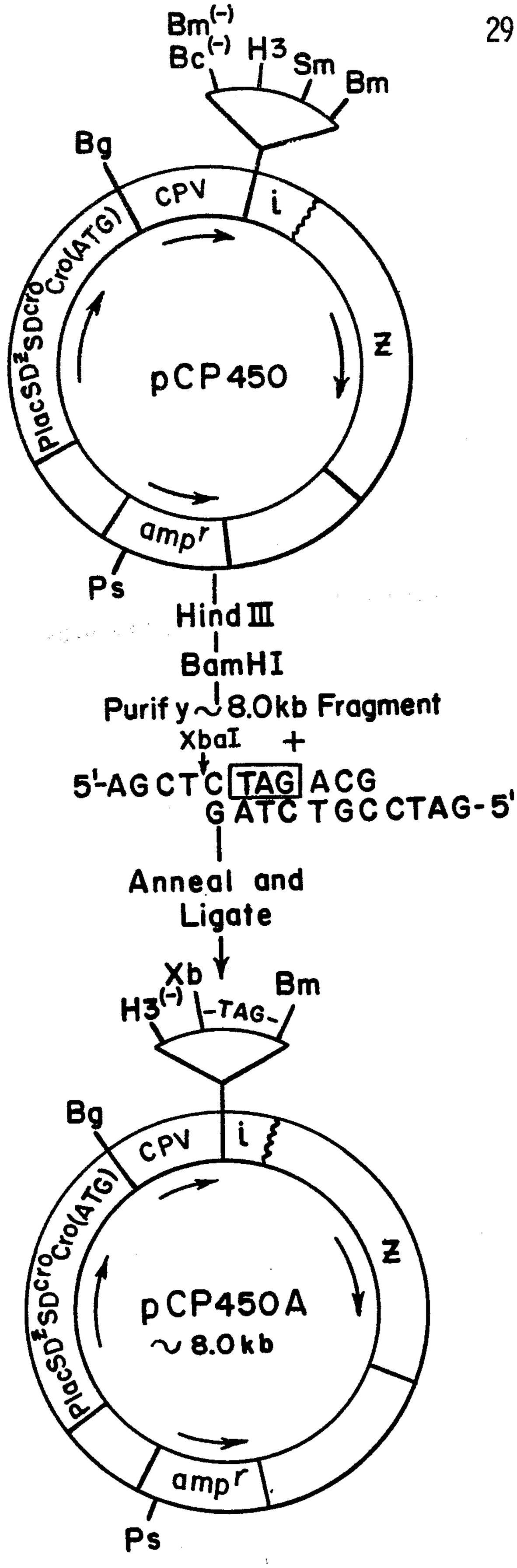
Bm(-)
Bc(-)
H3
Sm
Bm
Bg
CPV
i
PlacSD$^{\Sigma}$SD$^{cro}$Cro(ATG)
z
pCP450
amp$^r$
Ps
Hind III
BamHI
Purify ~ 8.0kb Fragment
XbaI
+
5'-AGCTC TAG ACG
G ATC TGC CTAG-5'
Anneal and
Ligate
Xb
H3(-)
-TAG-
Bm
Bg
CPV
i
PlacSD$^{\Sigma}$SD$^{cro}$Cro(ATG)
z
pCP450A
~ 8.0kb
amp$^r$
Ps

0117767

FIG. 25

30 / 33

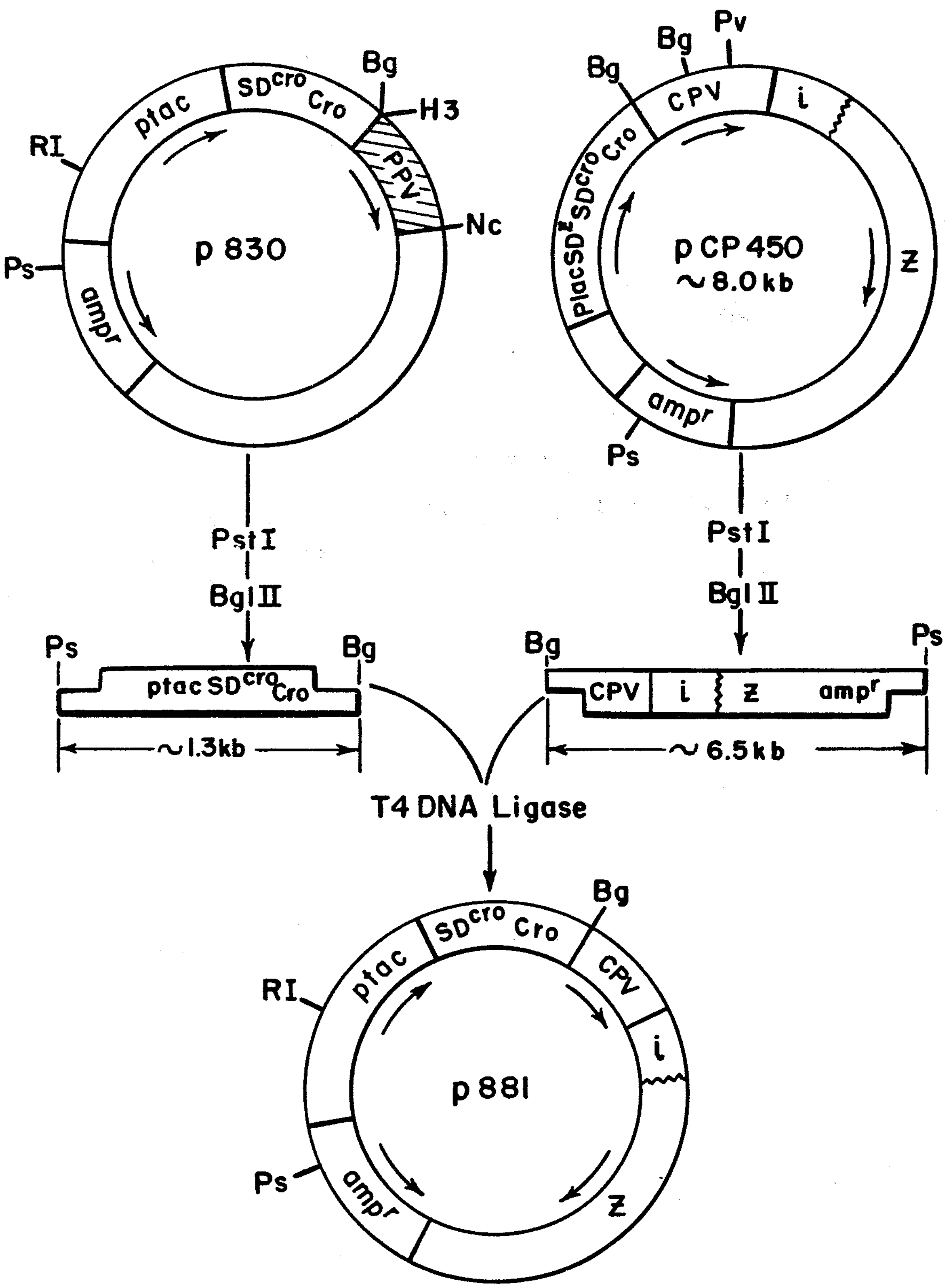

FIG. 26



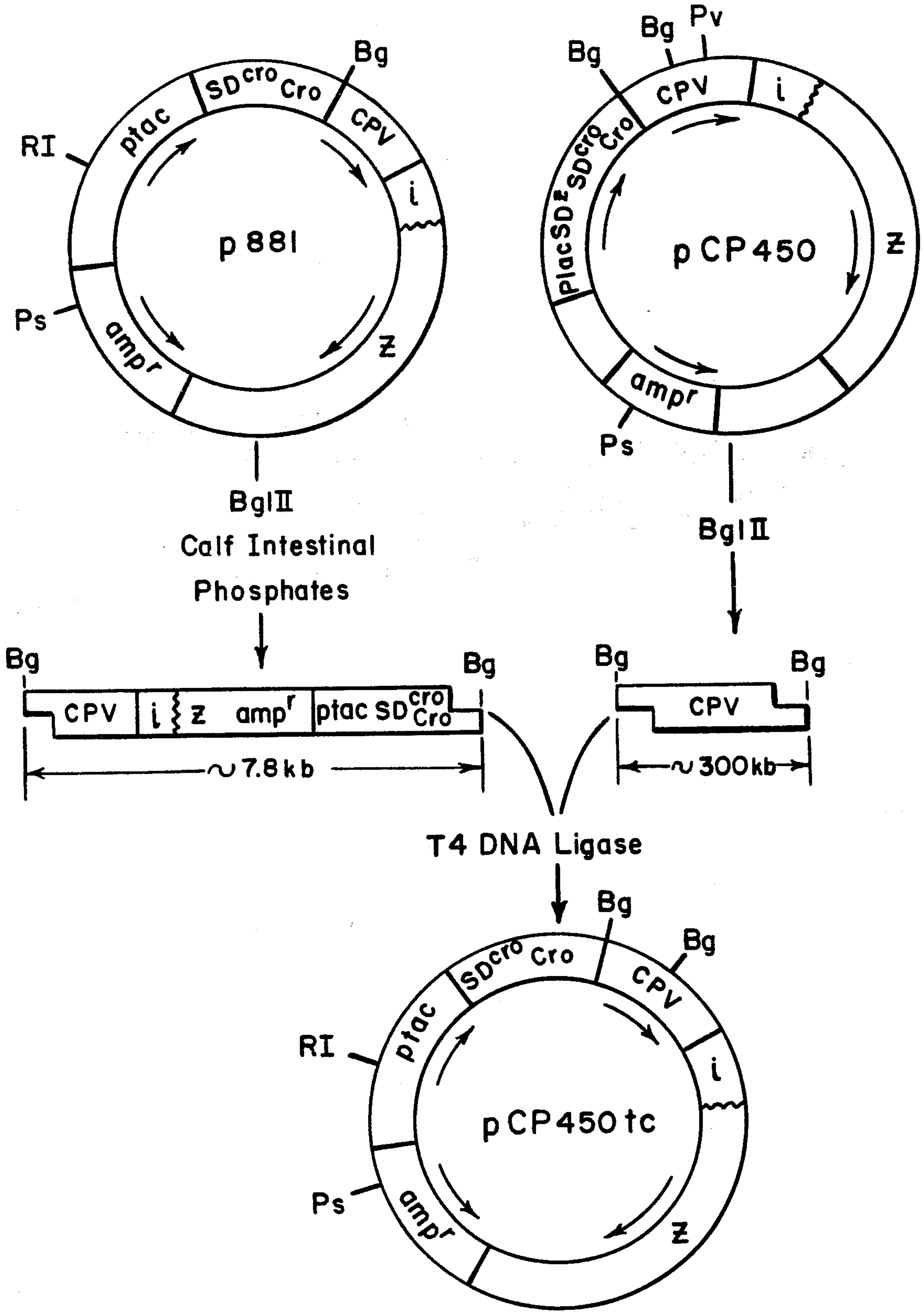

p 881
RI
ptac
SDcro Cro
Bg
CPV
i
Z
ampr
Ps
pCP450
Bg
Pv
Bg
CPV
i
Z
PlacSD# SDcro Cro
ampr
Ps
BglII
Calf Intestinal
Phosphates
BglII
Bg
CPV
i
Z
ampr
ptac SDcro Cro
Bg
~7.8 kb
Bg
CPV
Bg
~300kb
T4 DNA Ligase
pCP450 tc
Bg
Bg
SDcro Cro
CPV
RI
ptac
i
Z
Ps
ampr

# FIG. 27



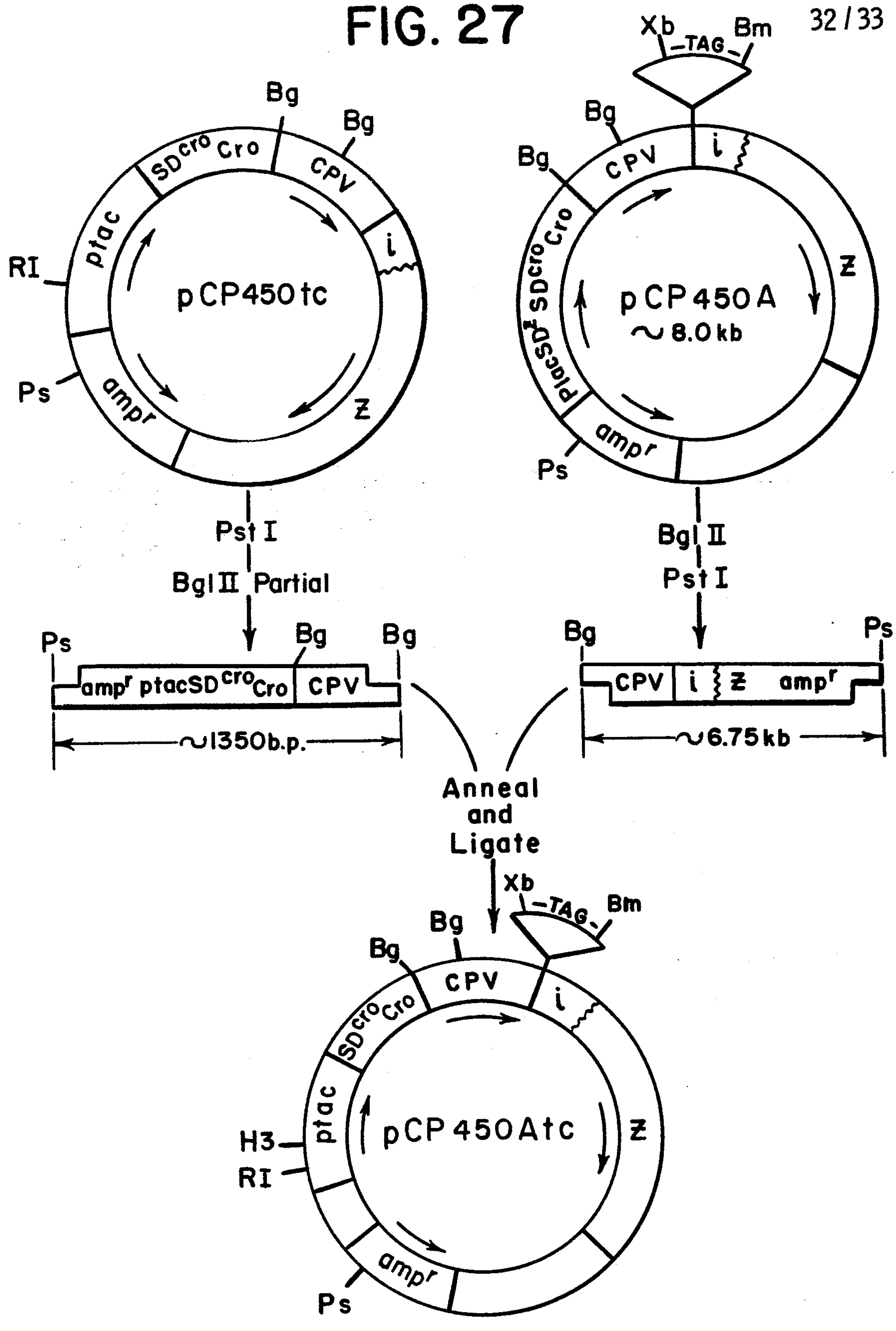

Xb
-TAG-
Bm
Bg
Bg
SD cro Cro
CPV
i
ptac
RI
pCP450tc
Ps
ampr
Z
Bg
Bg
CPV
i
PlacSDZ SDcro Cro
pCP450A
~8.0kb
Z
ampr
Ps
Pst I
BglII Partial
BglII
PstI
Ps
Bg
Bg
ampr ptacSD cro Cro
CPV
~1350b.p.
Bg
Ps
CPV
i
Z
ampr
~6.75kb
Anneal
and
Ligate
Xb
-TAG-
Bm
Bg
Bg
CPV
SD cro Cro
i
ptac
H3
RI
pCP450Atc
Z
ampr
Ps

# FIG. 28



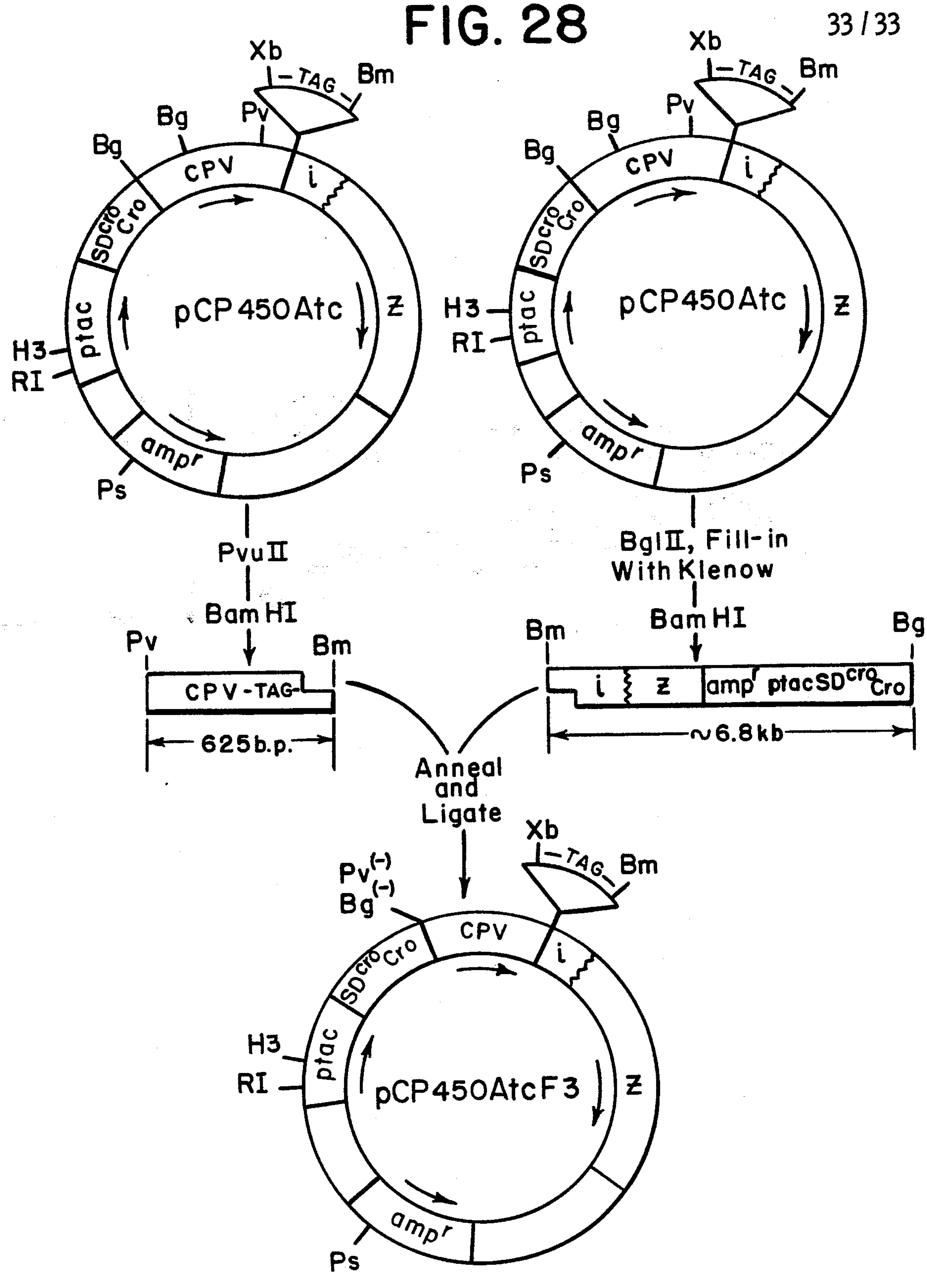
Xb
TAG
Bm
Bg
Pv
Bg
CPV
i
SDcro Cro
pCP450Atc
z
H3
ptac
RI
ampr
Ps
Xb
TAG
Bm
Pv
Bg
Bg
CPV
i
SDcro Cro
H3
pCP450Atc
z
RI
ptac
ampr
Ps
PvuII
BglII, Fill-in
With Klenow
BamHI
BamHI
Pv
Bm
Bm
Bg
CPV-TAG
i
z
ampr ptacSDcro Cro
625b.p.
~6.8kb
Anneal
and
Ligate
Xb
TAG
Bm
Pv(-)
Bg(-)
CPV
SDcro Cro
i
H3
ptac
RI
pCP450AtcF3
z
ampr
Ps

0117767

European Patent Office

# EUROPEAN SEARCH REPORT

Application number

EP 84400024.0

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. ³) |
| A,D, P | JOURNAL OF VIROLOGY, vol. 45, no. 2, February 1983, Washington, USA<br>T.W. MOLITOR et al.: "Parcine Parvovirus: Virus Purification and Structural and Antigenic Properties of Virion Polypeptides" pages 842-854<br>* Page 842 *<br>-- | 1,2,7, 12,99, 100 | C 12 N 15/00<br>C 12 P 21/00<br>C 12 P 21/04<br>C 12 N 7/00<br>A 61 K 39/23//<br>C 12 R 1/19 |
| A,D | AMERICAN JOURNAL OF VETERINARY RESEARCH OF THE AMERICAN VETERINARY MEDICAL ASSOCIATION, vol. 41, no. 12, December 1980, Chicago, Ill. (USA)<br>P.S. PAUL et al.: "Evaluation of a Modified Live-Virus Vaccine for the Prevention of Porcine Parovirus -Induced Reproductive Disease in Swine" pages 2007-2011<br>* Summary *<br>-- | 1-3,7, 12,99, 100 | TECHNICAL FIELDS SEARCHED (Int. Cl. ³)<br>C 12 N<br>C 12 P<br>A 61 K |
| A,D | THE CORNELL VETERINARIAN, vol. 72, January 1982, Ithaka, N.Y., USA<br>R.V.H. POLLOCK et al.: "Dog Response to Inactivated Canine Parvovirus and Feline Panleukopenia Virus Vaccines" pages 16-35<br>* Abstract *<br>-- | 1,2,8, 13,99, 100 | |

The present search report has been drawn up for all claims

| Place of search | Date of completion of the search | Examiner |
|---|---|---|
| VIENNA | 06-04-1984 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

# EUROPEAN SEARCH REPORT

EP 84400024.0

| Category | Citation of document with indication, where appropriate, of relevant passages | Relevant to claim | CLASSIFICATION OF THE APPLICATION (Int. Cl.) |
|---|---|---|---|
| A | CELL, vol. 17, no. 3, July 1979, Cambridge, Massachusetts and London, England<br>C.R. ASTELL et al..: "Structure of 3'Hairpin Termini of Four Rodent Parvovirus Genomes: Nucleotide Sequence Homology at Origins of DNA Replication"<br>pages 691-703<br>* Totality *<br>-- | 1 | |
| A,P | WO - A1 - 83/02 393 (ANIMAL VACCINE RESEARCH CORPORATION)<br>* Abstract *<br>-- | 1 | TECHNICAL FIELDS SEARCHED (Int. Cl.) |
| A,D | PROCEEDINGS OF THE NATIONAL ACADEMY OF SCIENCES OF THE UNITED STATES OF AMERICA, vol. 78, no. 8, August 1981, Baltimor, Md.(USA)<br>V.R. RACANIELLO et al.: "Molecular cloning of poliovirus cDNA and determination of the complete nucleotide sequence of the viral genome"<br>pages 4887-4891<br>* Abstract *<br>---- | 1 | |